(19) Europäisches Patentamt
European Patent Office
Office européen des brevets

(11) EP 4 332 101 A1

(12) **EUROPEAN PATENT APPLICATION**
published in accordance with Art. 153(4) EPC

(43) Date of publication:
**06.03.2024 Bulletin 2024/10**

(21) Application number: **22794985.6**

(22) Date of filing: **28.04.2022**

(51) International Patent Classification (IPC):
***C07D 471/04*** *(2006.01)* ***A61K 31/519*** *(2006.01)*
***A61P 35/00*** *(2006.01)*

(52) Cooperative Patent Classification (CPC):
**A61K 31/519; A61P 35/00; C07D 471/04**

(86) International application number:
**PCT/CN2022/089900**

(87) International publication number:
**WO 2022/228515 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2021 CN 202110480531**
**29.09.2021 CN 202111149653**
**19.01.2022 CN 202210059263**

(71) Applicant: **Scinnohub Pharmaceutical Co., Ltd**
**High-tech Development Zone**
**Chengdu, Sichuan 610041 (CN)**

(72) Inventors:
- **YANG, Anle**
**Chengdu, Sichuan 610041 (CN)**
- **ZHANG, Dewei**
**Chengdu, Sichuan 610041 (CN)**
- **DONG, Lijin**
**Chengdu, Sichuan 610041 (CN)**
- **HE, Quanhong**
**Chengdu, Sichuan 610041 (CN)**
- **YI, Tao**
**Chengdu, Sichuan 610041 (CN)**
- **MENG, Jiang**
**Chengdu, Sichuan 610041 (CN)**
- **TIAN, Lin**
**Chengdu, Sichuan 610041 (CN)**
- **LIANG, Jie**
**Chengdu, Sichuan 610041 (CN)**
- **FENG, Ze**
**Chengdu, Sichuan 610041 (CN)**
- **HU, Kai**
**Chengdu, Sichuan 610041 (CN)**
- **ZHANG, Xiaodong**
**Chengdu, Sichuan 610041 (CN)**
- **ZHANG, Yi**
**Chengdu, Sichuan 610041 (CN)**
- **HU, Xi**
**Chengdu, Sichuan 610041 (CN)**
- **HOU, Yanan**
**Chengdu, Sichuan 610041 (CN)**
- **TANG, Jun**
**Chengdu, Sichuan 610041 (CN)**

(74) Representative: **Thoma, Michael**
**Lorenz Seidler Gossel**
**Rechtsanwälte Patentanwälte**
**Partnerschaft mbB**
**Widenmayerstraße 23**
**80538 München (DE)**

(54) **METHIONINE ADENOSYLTRANSFERASE INHIBITOR, PREPARATION METHOD THEREFOR AND APPLICATION THEREOF**

(57) Disclosed are a methionine adenosyltransferase inhibitor represented by formula (I), a preparation method thereof and an application thereof in the pharmaceutical field, wherein $R_1$, $R_2$, $R_3$ and A are as defined in the description and claims.

EP 4 332 101 A1

## Description

### FIELD OF THE INVENTION

The present invention relates to the field of medicinal chemistry, and particularly relates to a methionine adenosyltransferase inhibitor, a preparation method thereof, and use thereof in the pharmaceutical field.

### BACKGROUND

Loss-of-function mutations in tumor suppressor genes are very common, but there are few therapies achieving selective targeting based on deletion mutations in tumor suppressor genes, which is easy to be understood, that is, the deleted protein is difficult to be directly inhibited to achieve efficacy. Targeted treatment of tumor suppressor genes inactivated by homozygous deletion is particularly difficult to achieve, because the lack of residual proteins renders the treatment strategy of directly activating, stabilizing, or repairing tumor suppressor genes ineffective.

Methionine adenosyltransferase (MAT), also known as S-adenosylmethionine synthetase, is the cellular enzyme catalyzing the synthesis of S-adenosylmethionine (SAM or AdoMet) from methionine and ATP, which is considered as the rate-limiting step in the methionine cycle. SAM is a propylamine donor in polyamine biosynthesis and a main methyl donor used for DNA methylation, and is involved in gene transcription, cell proliferation, as well as generation of secondary metabolites. MAT gene, which can be divided into MAT1A gene and MAT2a gene, encodes MAT, the only enzyme that can catalyze the synthesis of SAM. There are three types of isozymes, i.e., MAT I, MAT III, and MAT II, with the first two being products encoded by MAT1a gene, and the last one being the product encoded by MAT2a gene. MAT1a gene is mainly expressed in the adult liver, whereas MAT2a gene is widely expressed in human tissues other than the liver. More and more studies have found that MAT2a protein is also highly expressed in other tissues or cells of cancer, such as breast cancer, intestinal cancer, leukemia and lymphoma, etc., and the silencing of MAT2a gene leads to the death of corresponding cancer cells, indicating that MAT2a protein has the potential of being used as a treatment target.

Methylthioadenosine phosphorylase (MTAP) is an enzyme expressed in all normal tissues, and it catalyzes the conversion of methylthioadenosine (MTA) to adenine and 5-methylthioglycoside-1-phosphate. Many malignant tumor cell lines lack MTAP activity. Meanwhile, the loss of MTAP activity has been detected in a large number of primary lesions, such as glioma, melanoma, pancreatic cancer, non-small cell lung cancer, bladder cancer, astrocytoma, osteosarcoma, head and neck cancer, myxoid chondrosarcoma, ovarian cancer, endometrial cancer, breast cancer, soft tissue sarcoma, and non-Hodgkin's lymphoma. When MTAP is absent, the accumulation of MTA in cell will reach approximately 100 $\mu$M, and the cell will begin to excrete MTA. The abnormal accumulation of MTA leads to the vulnerability of Protein Arginine Methyltransferase 5 (PRMT5). Since PRMT5 uses SAM as a methyl donor substrate, the inhibition of MAT2a activity reduces intracellular concentration of SAM, thereby selectively reducing PRMT5 methylation activity in MTAP-deficient cells to a level below the threshold required for growth. Therefore, the inhibition of MAT2a activity can generate a combined killing effect in MTAP-deficient cells by inhibiting PRMT5 activity, which may provide therapeutic benefit for a variety of cancers.

### SUMMARY OF THE INVENTION

One of the purposes of the present invention is to provide a compound with MAT2a inhibitory activity.

Specifically, the present invention provides a compound represented by the structure of Formula I below, the pharmaceutically acceptable salt, hydrate, isomer, prodrug and mixture thereof:

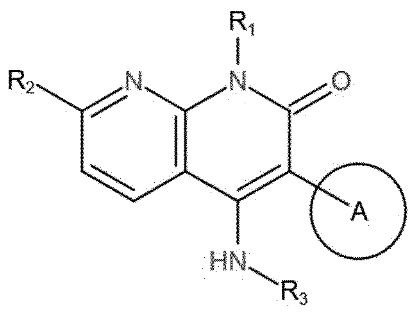

Formula I

wherein, $R_1$ is selected from 5-10 membered aryl or aromatic heterocyclic group; $R_2$ is selected from $-CF_3$ or cyclopropyl;

$R_3$ is selected from hydrogen, alkyl, aryl, aromatic heterocyclic group, cycloalkyl, aliphatic heterocyclic group, bridged cyclic group and spirocyclic group;

A is aryl or aromatic heterocyclic group,

with the proviso that the compound excludes compounds of the following formulae:

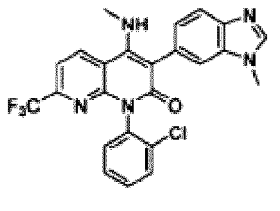

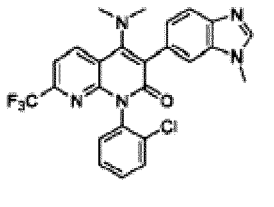

,

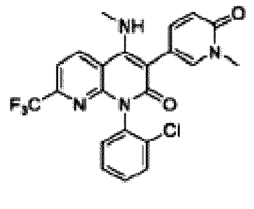

,

and

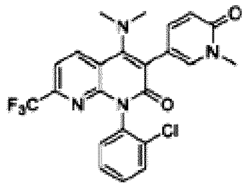

.

Further, $R_1$ of the present invention is selected from imidazolyl, thiazolyl, pyrazolyl, phenyl, pyridinyl, pyrimidinyl, pyridazinyl, and pyrazinyl.

In some particular embodiments, $R_1$ of the present invention can be further substituted with 0-2 $R_a$ groups; each of $R_a$ groups can be independently selected from alkyl, halogen, haloalkyl, alkoxy, haloalkoxy, hydroxy, amino, amine, carboxy, amide, cycloalkyl, and deuterium.

In some particular embodiments, $R_1$ can be further substituted with 0-2 $R_a$ groups; each of $R_a$ groups can be independently selected from $C_1$-$C_3$ alkyl, fluoro, chloro, bromo, iodo, $C_1$-$C_3$ haloalkyl, $C_1$-$C_3$ alkoxy, $C_1$-$C_3$ haloalkoxy, hydroxy, amino, amine, carboxy, acyl, $C_3$-$C_6$ cycloalkyl, and deuterium.

In some particular embodiments, $R_1$ is phenyl, and the phenyl can be further substituted with 0-2 $R_a$ groups; each of $R_a$ groups can be independently selected from $C_1$-$C_3$ alkyl, fluoro, chloro, bromo, iodo, $C_1$-$C_3$ haloalkyl, $C_1$-$C_3$ alkoxy, $C_1$-$C_3$ haloalkoxy, hydroxy, amino, amine, carboxy, acyl, $C_3$-$C_6$cycloalkyl, and deuterium.

In some particular embodiments, $R_1$ is phenyl, and the phenyl can be further substituted with 0-2 $R_a$ groups; each of $R_a$ groups can be independently selected from $C_1$-$C_3$ alkyl, fluoro, chloro, bromo, and iodo.

In some particular embodiments, $R_1$ is selected from phenyl, 4-chlorophenyl, 4-bromophenyl, and 4-methylphenyl, and the phenyl can be further substituted with fluorine.

In some particular embodiments, $R_3$ of the present invention is selected from hydrogen, $C_1$-$C_3$ alkyl, 6-10 membered aryl, 5-10 membered aromatic heterocyclic group, $C_3$-$C_6$ cycloalkyl, 3-6 membered aliphatic heterocyclic group, 4-10 membered bridged cyclic group, and spirocyclic group;

In some particular embodiments, $R_3$ is selected from hydrogen, methyl, ethyl, propyl, isopropyl, cyclopropyl, cyclobutyl, cyclopentyl, oxetanyl, tetrahydrofuranyl, tetrahydropyranyl, thiocyclohexyl, piperidinyl, pyrrolidinyl, phenyl, pyridinyl, pyrimidinyl, imidazolyl, pyrazolyl, thiazolyl, oxazolyl, isoxazolyl, 1,2,4-oxadiazolyl, 4-10 membered bridged cyclic group, and spirocyclic group;

Further, when $R_3$ of the present invention is not hydrogen, $R_3$ can be optionally substituted with one or more groups selected from halogen, alkyl, alkoxy, cyano, hydroxy, amino, deuterium, sulfone, sulfonyl, haloalkyl, cycloalkyl, and aliphatic heterocyclic group;

In some particular embodiments, when $R_3$ of the present invention is not hydrogen, $R_3$ can be optionally substituted with one or more groups selected from halogen, $C_1$-$C_3$ alkyl, $C_1$-$C_3$ alkoxy, cyano, hydroxyl, amino, deuterium, sulfone, sulfonyl, $C_1$-$C_3$ haloalkyl, $C_3$-$C_6$ cycloalkyl, 3-6 membered aliphatic heterocyclic group;

In some particular embodiments, when $R_3$ of the present invention is not hydrogen, $R_3$ can be optionally substituted with one or more groups selected from fluoro, chloro, bromo, iodo, methyl, ethyl, propyl, isopropyl, cyclopropyl, cyclobutyl, cyclopentyl, oxetanyl, tetrahydrofuryl, tetrahydropyranyl, thiocyclohexyl, piperidinyl, pyrrolidinyl, trifluoromethyl, hydroxyl, amino, cyano, deuterium, sulfone, and sulfonyl.

In some particular embodiments, $R_3$ is selected from hydrogen and $C_1$-$C_3$ alkyl.

Further, ring A of the present invention is selected from 6-10 membered aromatic cyclic group and 5-10 membered aromatic heterocyclic group.

In some particular embodiments, ring A of the present invention is selected from phenyl, naphthyl, imidazolyl, pyrazolyl, triazolyl, thiazolyl, furanyl, pyrrolyl, thienyl, oxazolyl, isoxazolyl, oxadiazolyl, pyridinyl, pyrimidinyl, pyridazinyl, pyrazinyl, benzoimidazolyl, benzopyrazolyl, benzothiazolyl, benzoxazolyl, benzobisoxazole, imidazopyridinyl, benzisoxazolyl, naphthyridinyl, quinolinyl, isoquinolinyl, quinoxalinyl, pyrazolopyridinyl, triazolopyridinyl, pyridonyl, quinazolinyl, cinnolinyl, pyridopyrazine, benzotriazolyl, and benzoxadiazolyl.

In some particular embodiments, ring A of the present invention is selected from phenyl, naphthyl, imidazolyl, pyrazolyl, triazolyl, thiazolyl, furanyl, pyrrolyl, thienyl, oxazolyl, isoxazolyl, oxadiazolyl, pyridinyl, pyrimidinyl, benzoimidazolyl, benzopyrazolyl, benzothiazolyl, benzoxazolyl, imidazopyridinyl, quinolinyl, quinoxalinyl, pyrazolopyridinyl, triazolopyridinyl, pyridonyl, quinazolinyl, cinnolinyl, pyridopyrazine, benzotriazolyl, and benzoxadiazolyl.

In some particular embodiments, ring A of the present invention can be further substituted with one or more groups selected from alkyl, cycloalkyl, aliphatic heterocyclic group, halogen, alkoxy, amino, amine, hydroxy, cyano, haloalkyl, haloalkoxy, $-(CH_2)_nOCH_3$, $-(CH_2)_nSO_2CH_3$, and $-(CH_2)_nN(CH_3)_2$, wherein n = 1, 2, or 3.

In some particular embodiments, ring A of the present invention can be further substituted with one or more groups selected from $C_1$-$C_3$ alkyl, $C_1$-$C_3$ alkoxy, halogen, $C_1$-$C_3$ haloalkyl, amino, cyano, $-(CH_2)_nOCH_3$, $-(CH_2)_nSO_2CH_3$, and $-(CH_2)_nN(CH_3)_2$, wherein n = 1, 2, or 3.

In some particular embodiments, ring A of the present invention can be further substituted with one or more groups selected from methyl, methoxy, $-CF_3$, $-CH_2CF_3$, $-NH_2$, F, cyano, $-(CH_2)_2OCH_3$, $-(CH_2)_2SO_2CH_3$, $-(CH_2)_2N(CH_3)_2$.

In some particular embodiments, the substituent(s) on ring A can further form a ring, and form a fused ring with ring A, such as

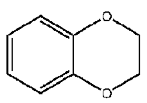, 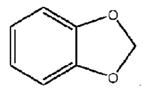 and 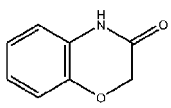.

In some particular embodiments, ring A of the present invention is selected from the following groups:

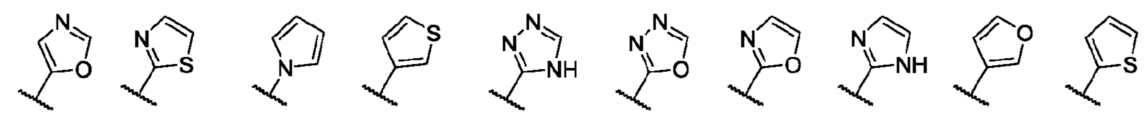

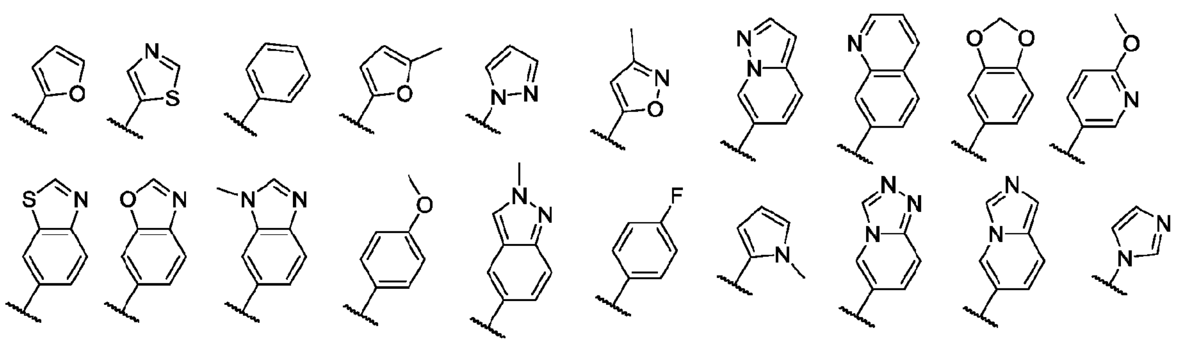

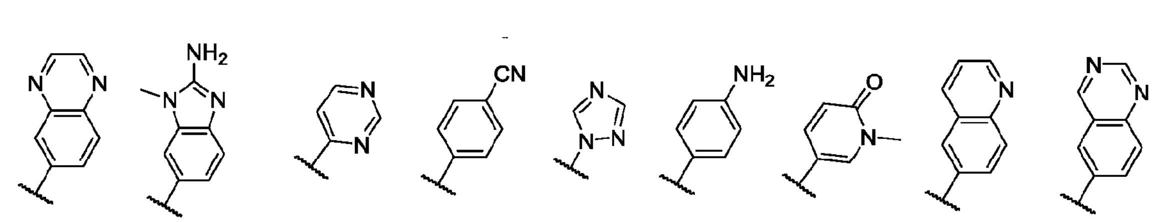

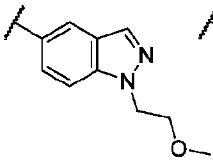 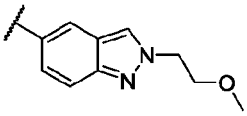 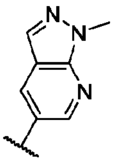 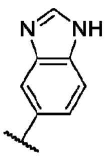 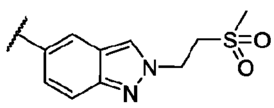

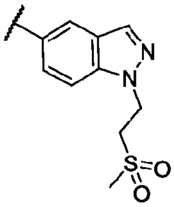 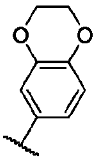 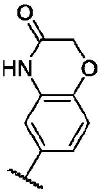 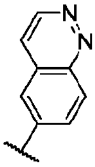 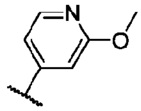 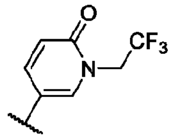 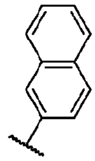

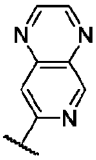 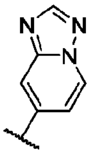 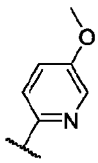 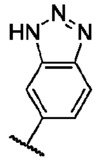 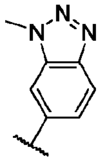 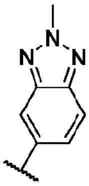 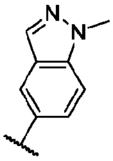 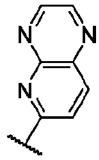

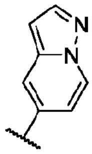 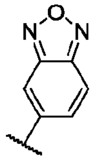 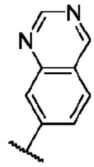 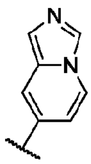 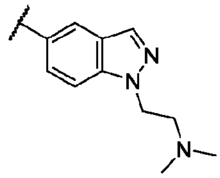 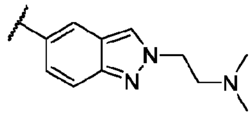 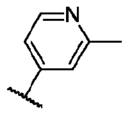

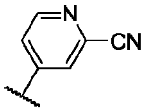 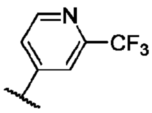

Another purpose of the present invention is to provide a compound of the structure represented by Formula II or Formula III below, the pharmaceutically acceptable salt, hydrate, isomer, prodrug and mixture thereof:

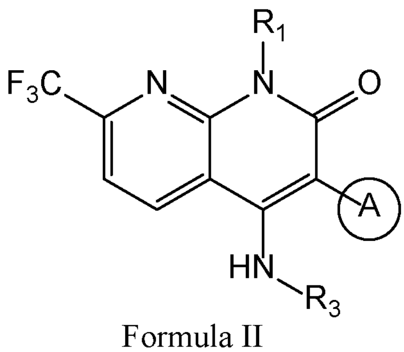

Formula II

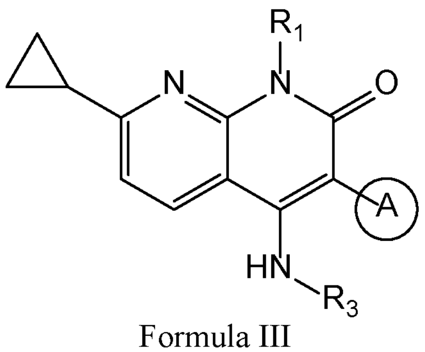

Formula III

wherein $R_1$, $R_3$, and A are as defined above.

In some particular embodiments, the compound of Formula I in the present invention has the following structure:

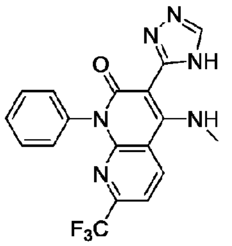
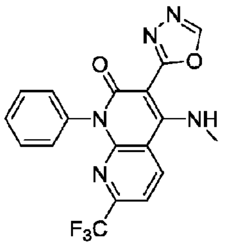
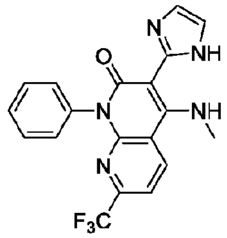
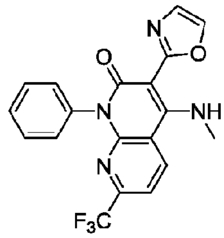

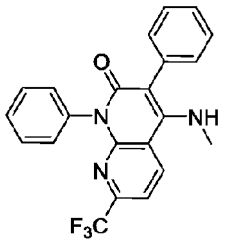
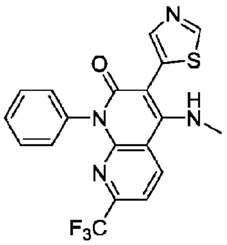
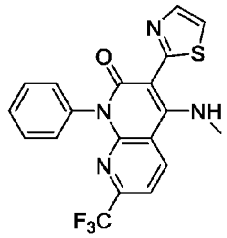
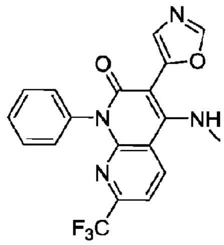

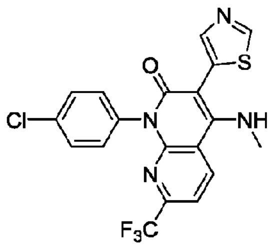
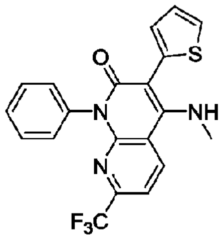
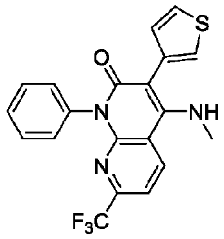
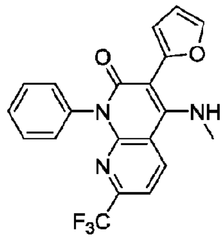

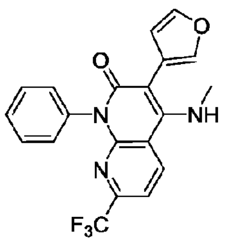
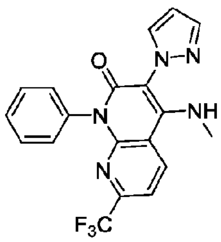
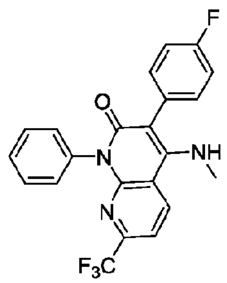
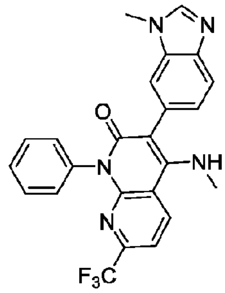

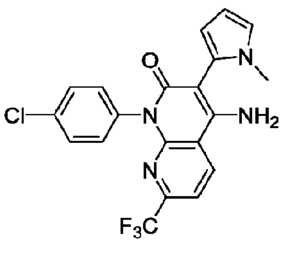
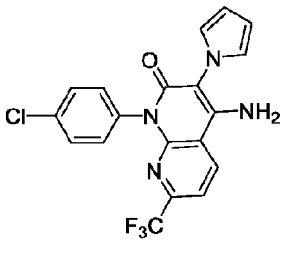
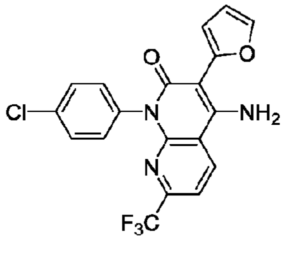

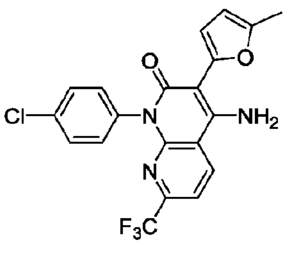
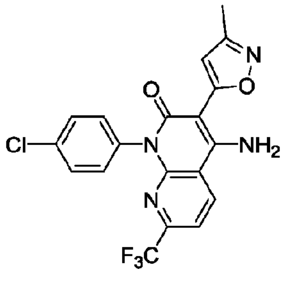
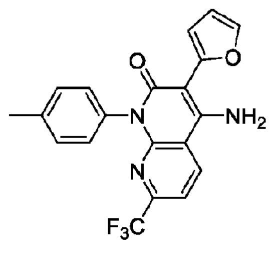

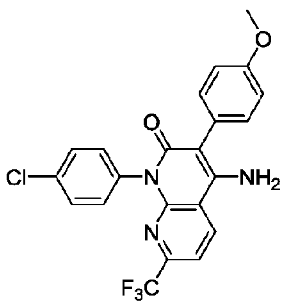
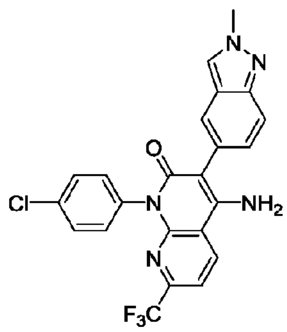
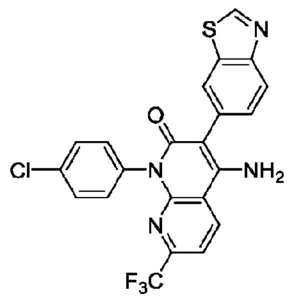

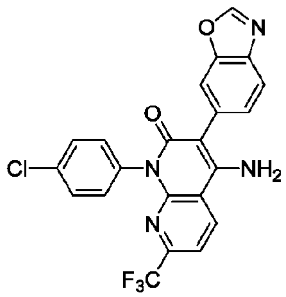
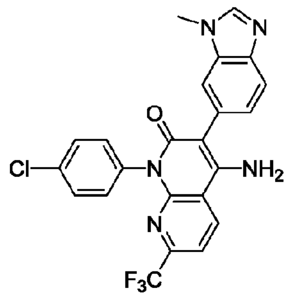
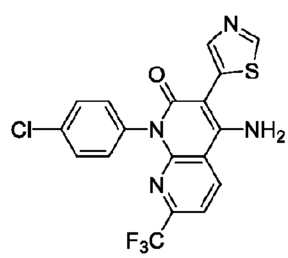

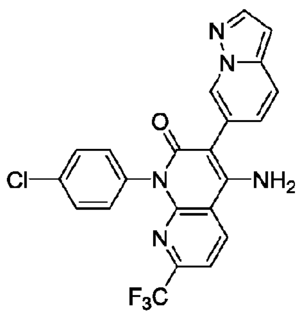
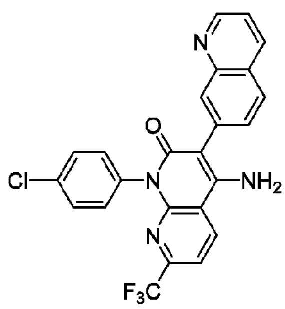
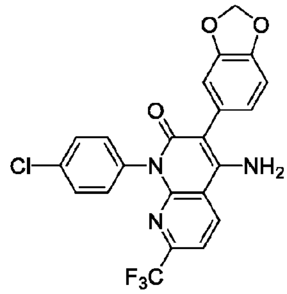

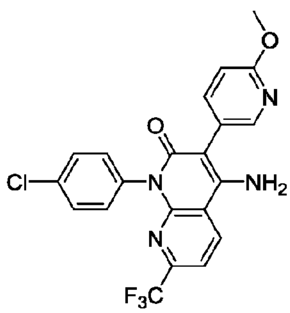
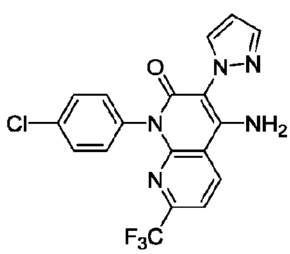
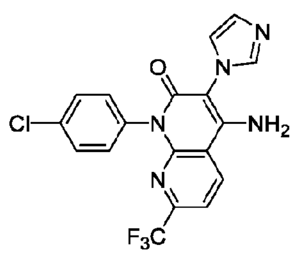

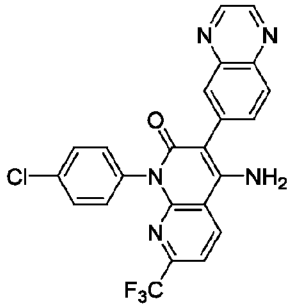
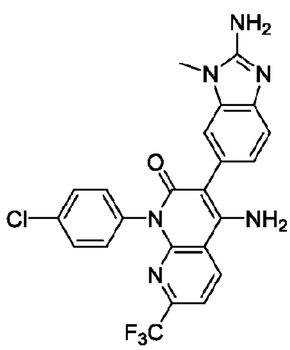
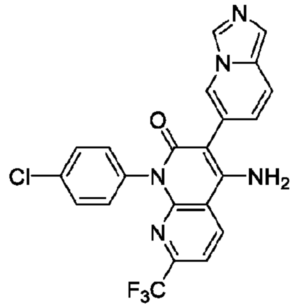

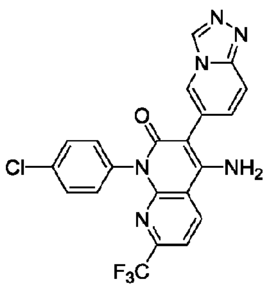

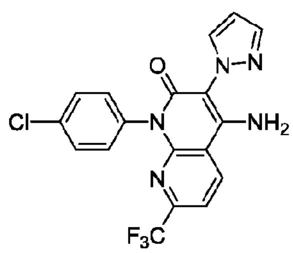

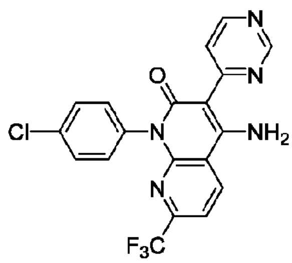

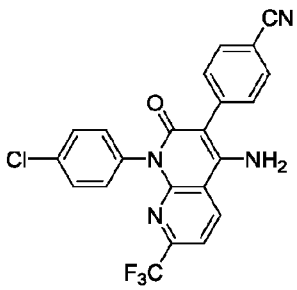

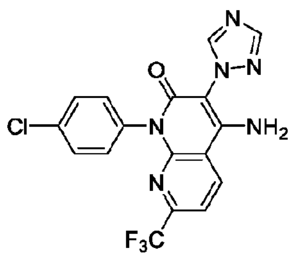

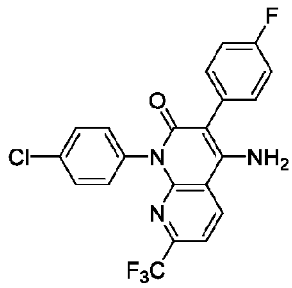

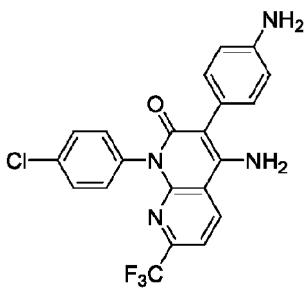

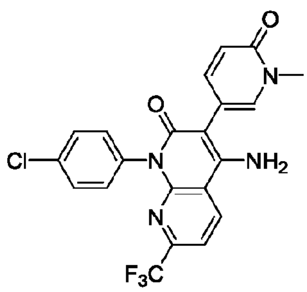

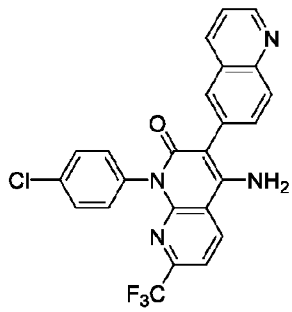

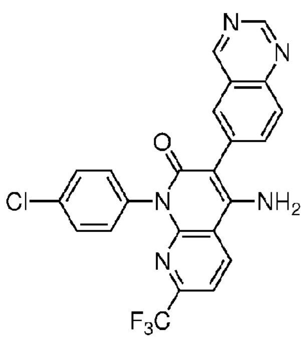

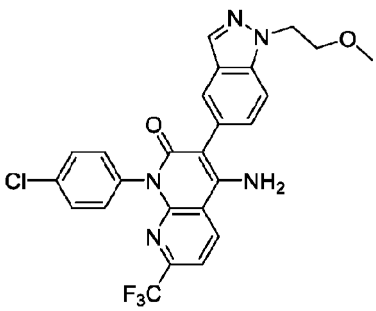

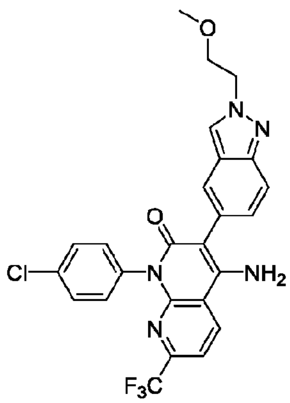

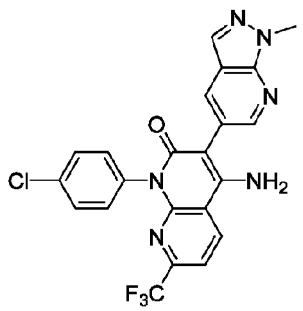

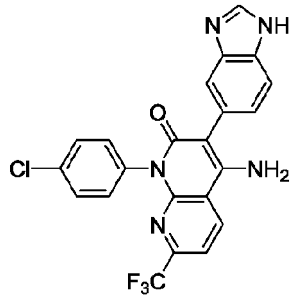

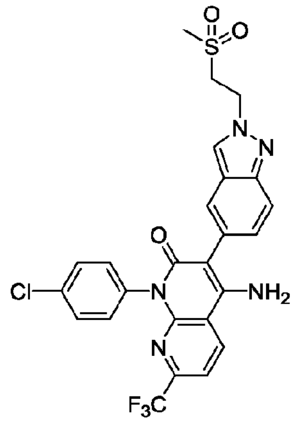

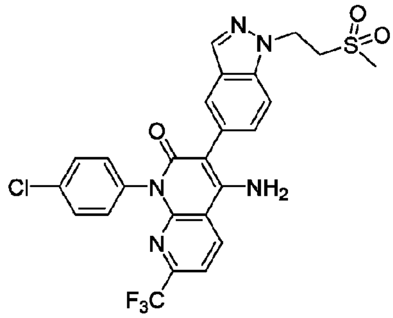

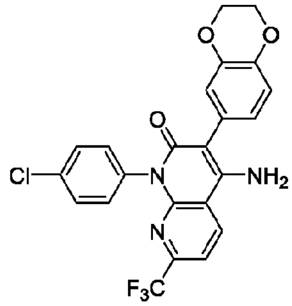

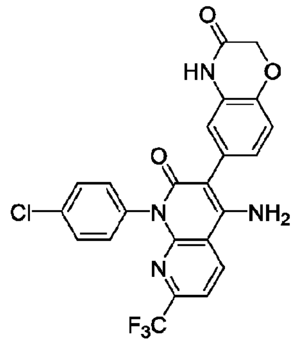

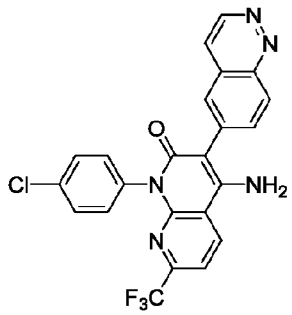

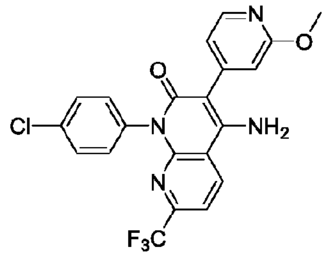

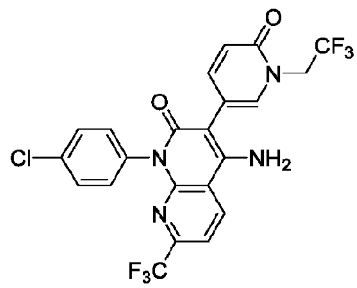

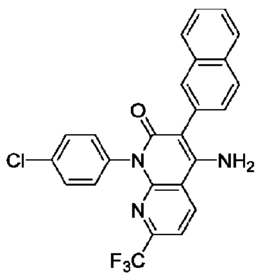

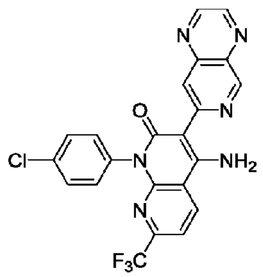

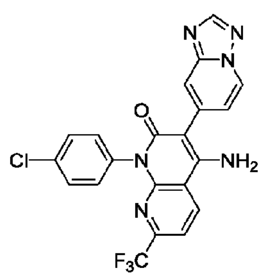

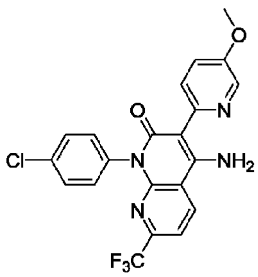

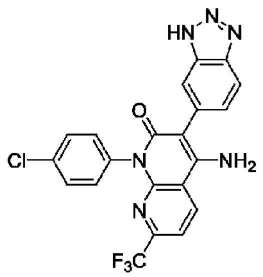

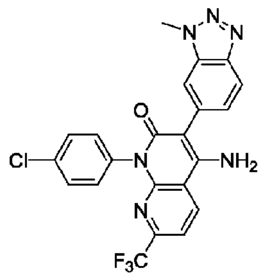

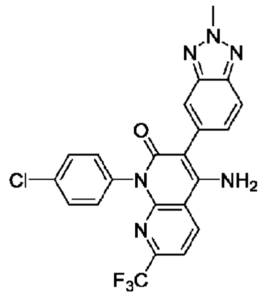

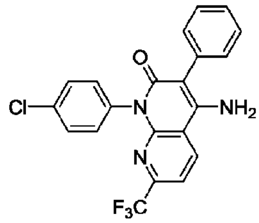

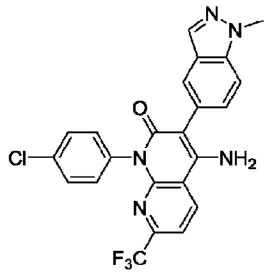

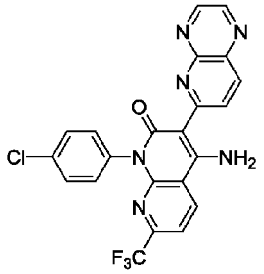

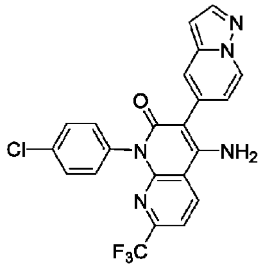

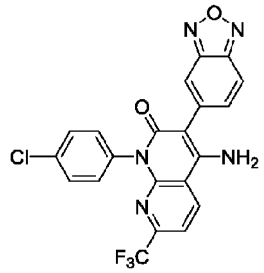

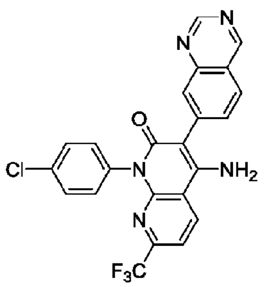

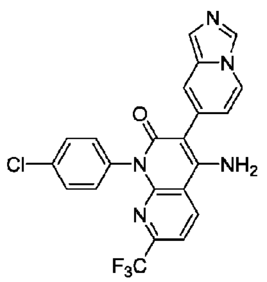

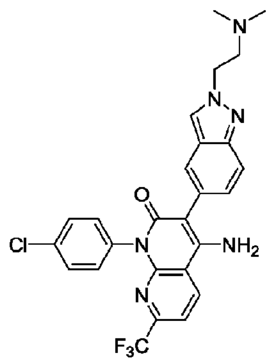

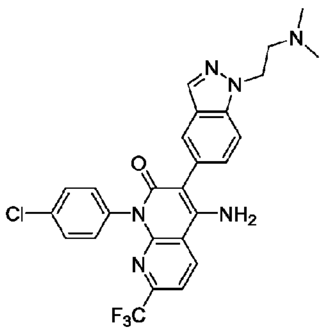

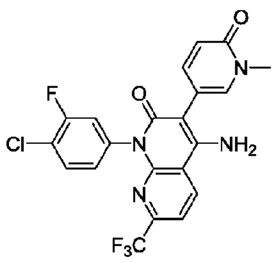

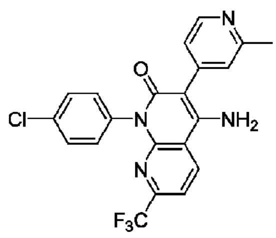

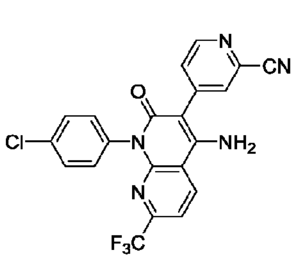

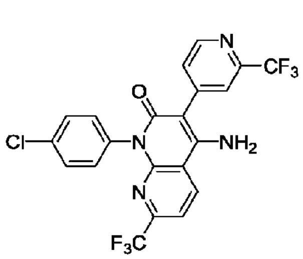

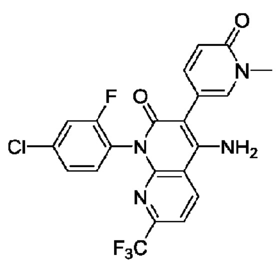

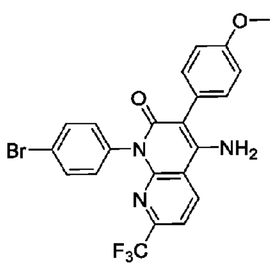
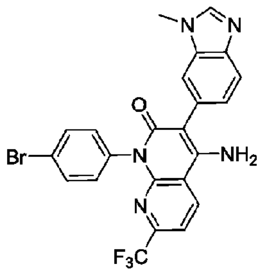
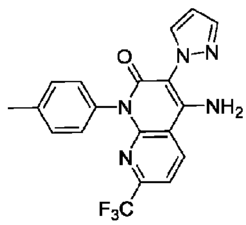

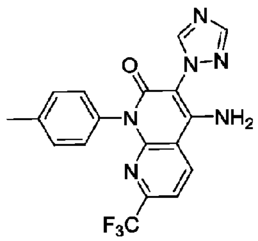
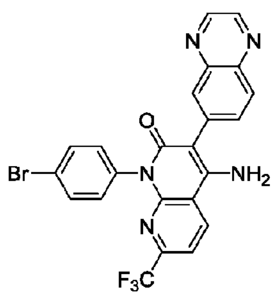
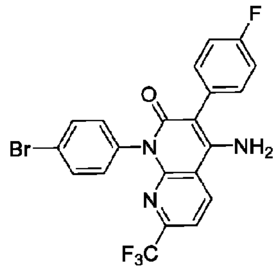

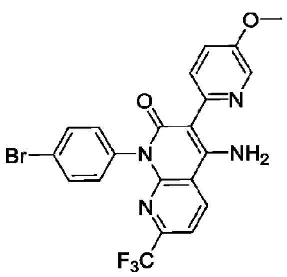
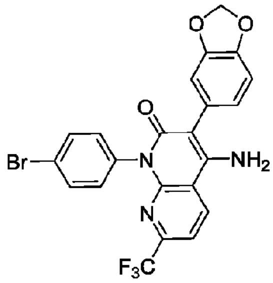
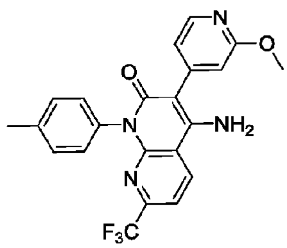

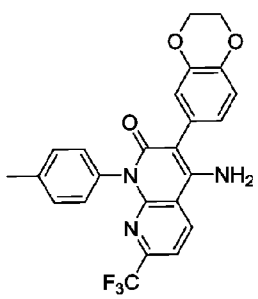
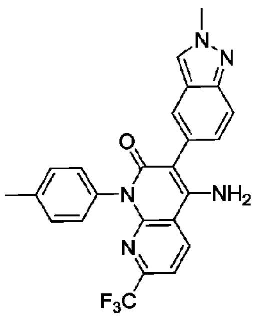
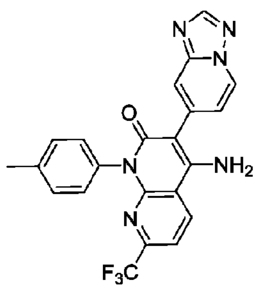

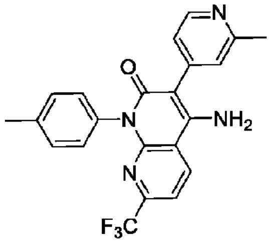
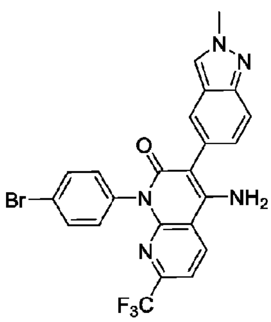
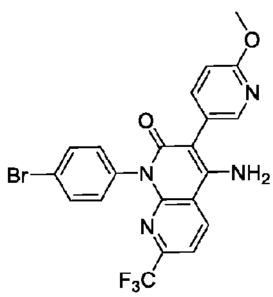

Another purpose of the present invention is to provide use of a compound of Formula I or Formula II or Formula III, the pharmaceutically acceptable salt, hydrate, isomer, prodrug, or mixture thereof in the preparation of a medicament

for the treatment of a MAT2a-related disease.

Another purpose of the present invention is to provide a pharmaceutical composition comprising a therapeutically effective dose of a compound of Formula I or Formula II or Formula III, or comprising a pharmaceutically acceptable salt, hydrate, isomer, prodrug, or mixture of a compound of Formula I or Formula II or Formula III, and a pharmaceutically acceptable carrier.

The present invention further provides use of the above-mentioned pharmaceutical compositions in the preparation of a medicament for the treatment of a MAT2a-related disease.

The MAT2a-related disease in the present invention is cancer or tumor. Further, the cancer or tumor comprises neuroblastoma, intestinal cancer such as rectal cancer, colon cancer, familial adenomatous polyposis cancer and hereditary nonpolyposis colorectal cancer, esophageal cancer, lip cancer, laryngeal cancer, hypopharyngeal cancer, tongue cancer, salivary gland cancer, gastric cancer, adenocarcinoma, medullary thyroid carcinoma, papillary thyroid carcinoma, renal carcinoma, renal parenchymal carcinoma, ovarian cancer, cervical cancer, uterine body cancer, endometrial cancer, choriocarcinoma, pancreatic cancer, prostate cancer, testicular cancer, breast cancer, urinary system cancer, melanoma, brain tumor such as glioblastoma, astrocytoma, meningioma, medulloblastoma and peripheral neuroectodermal tumor, Hodgkin's lymphoma, non-Hodgkin's lymphoma, Burkitt's lymphoma, acute lymphoblastic leukemia (ALL), chronic lymphoblastic leukemia (CLL), acute myeloid leukemia (AML), chronic myeloid leukemia (CML), adult T-cell leukemia, hepatocellular carcinoma, gallbladder cancer, bronchogenic carcinoma, small cell lung cancer, non-small cell lung cancer, multiple myeloma, basal cell tumor, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma, and plasmacytoma. In one embodiment, the cancer is lung cancer, non-small cell lung cancer (NSLC), bronchioloalveolar carcinoma, bone cancer, pancreatic cancer, skin cancer, head and neck cancer, skin or intraocular melanoma, uterine cancer, ovarian cancer, rectal cancer, anal cancer, gastric cancer, stomach cancer, colon cancer, breast cancer, uterine cancer, fallopian tube cancer, endometrial cancer, cervical cancer, vaginal cancer, vulva cancer, Hodgkin's disease, esophageal cancer, small intestine cancer, endocrine system cancer, thyroid cancer, parathyroid cancer, adrenal cancer, soft tissue sarcoma, urethral cancer, penile cancer, prostate cancer, bladder cancer, renal or ureteral carcinoma, renal cell carcinoma, renal pelvic carcinoma, mesothelioma, hepatocellular carcinoma, biliary tract cancer, chronic or acute leukemia, lymphoblastic lymphoma, central nervous system (CNS) tumor, spinal axis tumor, brain stem glioma, glioblastoma multiforme, astrocytoma, schwannoma, ependymoma, medulloblastoma, meningioma, squamous cell carcinoma, pituitary adenoma, including a refractory form of any of the above-mentioned cancers, or a combination of one or more of the above-mentioned cancers.

Another purpose of the present invention is to provide a method of treating a cancer or tumor disease comprising administering to a patient in need thereof one or more of the above-mentioned pharmaceutical composition, or the compound of Formula I or the pharmaceutically acceptable salt, hydrate, isomer, prodrug or mixture thereof.

Definition

Unless otherwise indicated, the following terms and phrases used herein are intended to have the meanings set forth below. A particular term or phrase shall not be considered uncertain or unclear in the absence of a specific definition, but should be understood according to its ordinary meaning. When a trade name is used herein, it refers to the corresponding product or the active ingredient thereof.

As used herein, the term "pharmaceutically acceptable" refers to those suitable for use in contact with the tissues of human beings and animals without excessive toxicity, irritation, allergic response, or other problem or complication, commensurate with a reasonable benefit/risk ratio.

The term "pharmaceutically acceptable salt" refers to a salt of a compound of the present invention, prepared from a compound with a specific substituent found in the present invention and a relatively non-toxic acid or base. When the compound of the present invention comprises a relatively acidic functional group, a base addition salt can be obtained by contacting the neutral form of such compounds with a sufficient amount of base in a pure solution or in a suitable inert solvent. When the compound of the present invention comprises a relatively basic functional group, an acid addition salt can be obtained by contacting the neutral form of such compounds with a sufficient amount of acid in a pure solution or a suitable inert solvent.

The compound of the present invention can exist in a specific form of geometric isomer or stereoisomer or atropisomer. All such compounds, including cis- and trans-isomers, (-)- and (+)-enantiomers, (R)- and (S)-enantiomers, diastereomers, (*D*)-isomers, (L)-isomers, and the racemic and other mixtures thereof, are contemplated in the present invention. All these isomers and mixtures thereof fall within the scope of the "isomers" described in the present invention.

"Alkyl" refers to a straight or branched saturated aliphatic hydrocarbon group. For example, $C_1$-$C_3$ alkyl refers to a saturated aliphatic hydrocarbon group containing 1 to 3 carbon atoms, including but not limited to methyl, ethyl, propyl, isopropyl and the like, and various isomers thereof.

"Cycloalkyl" refers to a saturated or partially unsaturated monocyclic or polycyclic hydrocarbon substituent. For

example, "$C_3$-$C_6$ cycloalkyl" refers to a cycloalkyl comprising 3 to 6 carbon atoms, and the typical $C_3$-$C_6$ cycloalkyls include, but are not limited to, cyclopropyl, cyclobutyl, cyclopentyl, cyclopentenyl, cyclohexyl, cyclohexenyl and the like.

"Aliphatic heterocyclic group" refers to a saturated monocyclic hydrocarbon substituent, wherein one or more of the ring atoms are substituted with a heteroatom selected from N, O, or S, and the other ring atoms are carbon. For example, "3-6 membered aliphatic heterocyclic group" refers to a saturated cyclic hydrocarbon substituent comprising 3-6 ring atoms, wherein one or more of the ring atoms are substituted with a heteroatom selected from N, O, S, and the other ring atoms are carbon. Specific examples include, but are not limited to, oxetanyl, pyrrolidinyl, tetrahydrofuranyl, morpholino and the like.

"Bridged cyclic group" refers to a cyclic structure group formed by two or more ring structures sharing two non-adjacent ring atoms with each other.

"Spirocyclic group" refers to a cyclic structure group formed by two rings sharing one carbon atom.

"Aryl" refers to an aromatic cyclic group, and examples of the aryl moiety include phenyl, naphthyl and the like.

"Aromatic heterocyclic group" refers to an aromatic cyclic substituent, wherein one or more of the ring atoms are substituted with a heteroatom selected from N, O, or S, and the other ring atoms are carbon. For example, "5-10 membered aromatic heterocyclic group" refers to an aromatic heterocyclic group comprising 5 to 10 ring atoms, wherein one or more of the ring atoms are substituted with a heteroatom selected from N, O, S, and the other ring atoms are carbon. Specific examples of "5-10 membered aromatic heterocyclic group" include, but are not limited to, imidazolyl, pyrazolyl, triazolyl, thiazolyl, furanyl, pyrrolyl, thienyl, oxazolyl, isoxazolyl, oxadiazolyl, pyridinyl, pyrimidinyl, pyridazinyl, pyrazinyl, benzoimidazolyl, benzopyrazolyl, benzothiazolyl, benzoxazolyl, benzobisoxazole, imidazopyridinyl, benzisoxazolyl, naphthyridinyl, quinolinyl, isoquinolinyl, quinoxalinyl, pyrazolopyridinyl, triazolopyridinyl, pyridonyl, quinazolinyl, cinnolinyl, pyridopyrazine, benzotriazolyl, benzoxadiazolyl and the like.

"Sulfone" refers to the group $-S(=O)_2-$;

"Sulfonyl" refers to $-S(=O)_2-NR_bR_c$, wherein $R_b$ and $R_c$ are any substituents, respectively, such as hydrogen, alkyl, cycloalkyl, aryl, heterocyclic group and the like.

"Amino" refers to $-NH_2$;

"Amine" refers to $-NH-R_x$, wherein $R_x$ is any substituent, such as alkyl, cycloalkyl, aryl, heterocyclic group, and the like.

"Acyl" refers to $-C(=O)-R_d$, wherein $R_d$ is any substituent, such as alkyl, cycloalkyl, amino, aryl, heterocyclic group, haloalkyl, and the like.

"Optionally" means that the subsequently described event or situation may, but not necessarily, occur.

When any variable appears more than once in a composition or structure of a compound, the definition thereof is independent in each case.

The abbreviations in the present invention are all known to those skilled in the art, and represent meanings commonly known in the art, unless otherwise indicated. For example, DMF refers to *N,N*-dimethylformamide; THF refers to tetrahydrofuran; Me refers to methyl.

It has been experimentally demonstrated that the compound of the present invention possesses excellent MAT2a enzyme inhibitory activity, excellent inhibitory effect on the growth of cancer cells, and good safety, along with good druggability. Therefore, the compound of the present invention shall have an excellent prospect of use in MAT2a-related cancer or tumor disease.

## EMBODIMENTS OF THE INVENTION

The methods of synthesizing the compounds and intermediates of the present invention are described below by way of example. The following examples are only intended to serve as examples of the present invention, and should not be taken as a limitation to the scope of the present invention. Unless otherwise indicated, the raw materials and reagents involved in the present invention are all available commercially, and the specific source does not affect the implementation of the technical solution of the present invention.

### Preparation Example 1: Preparation of 3-bromo-4-(methylamino)-1-phenyl-7-(trifluoromethyl)-1,8-naphthyridin-2(1*H*)-on e

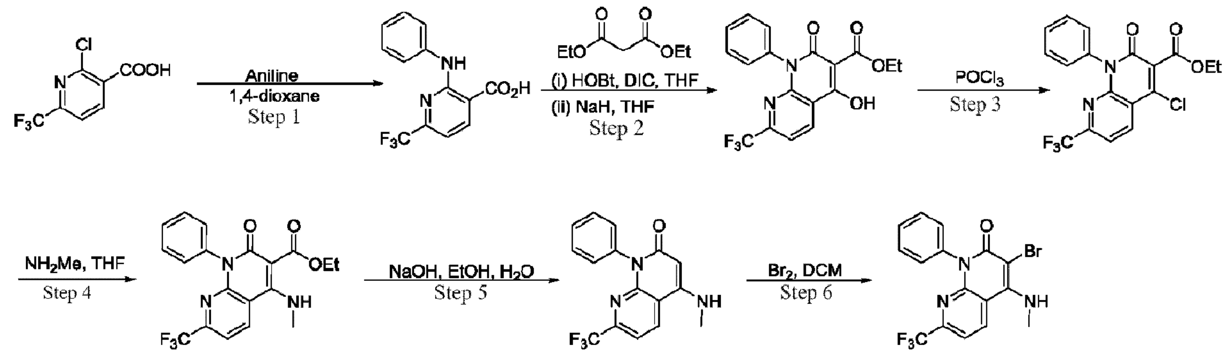


Step 1: Preparation of 2-(phenylamino)-6-(trifluoromethyl)nicotinic acid

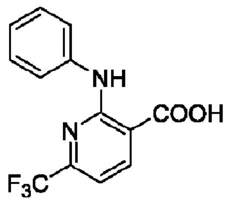

2-Chloro-6-(trifluoromethyl)nicotinic acid (1.0 g) was dissolved in 1 ,4-dioxane (10 mL), into which aniline (2.0 g) was added. The reaction was initiated by microwave at 120 °C for 5 h, and LCMS showed that most of the raw materials were completely reacted. Then, the system was concentrated under reduced pressure, and the residue was added to petroleum ether with stirring, and filtered. The filtrate was concentrated under reduced pressure, and the resulting crude product was purified by column chromatography to give 1.0 g of the title compound.

MS (ESI) m/z $(M+H)^+$ = 283.0.

$^1$H NMR (400 MHz, DMSO-$d_6$) $\delta$ 14.14 (brs, 1H), 10.59 (s, 1H), 8.47 (d, *J* = 7.8 Hz, 1H), 7.82-7.63 (m, 2H), 7.37 (dd, *J* = 8.5, 7.3 Hz, 2H), 7.29 (d, *J* = 7.9 Hz, 1H), 7.07 (td, *J* = 7.3, 1.1 Hz, 1H).

Step 2: Preparation of ethyl 4-hydroxy-2-oxo-1-phenyl-7-(trifluoromethyl)-1,2-dihydro-1,8-naphthyridine-3-c arboxylate

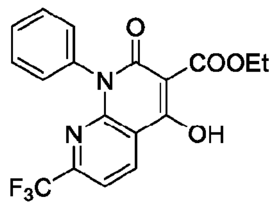

2-(Phenylamino)-6-(trifluoromethyl)nicotinic acid (500 mg) was dissolved in tetrahydrofuran, into which N,N-diisopropylcarbodiimide (670 mg) and 1-hydroxybenzotriazole (718 mg) were added, followed by reacting at room temperature for one hour. Additionally, diethyl malonate (567 mg) was dissolved in tetrahydrofuran (10 mL), and then sodium hydride (355 mg) was added in portions in ice bath, followed by reacting at room temperature for 1 h. Then, the 2-(phenylamino)-6-(trifluoromethyl)nicotinic acid system was added dropwise to the diethyl malonate system, and the reaction was continued at room temperature for 2 h after the addition. LC-MS showed that the reaction was completed. The system was quenched by adding saturated ammonium chloride aqueous solution, and extracted with ethyl acetate. The organic phases were combined, dried over anhydrous sodium sulfate, filtered, and concentrated. The residue was purified by column chromatography to give 250 mg of the target compound.

MS (ESI) m/z $(M+H)^+$=379.1.

Step 3: Preparation of ethyl 4-chloro-2-oxo-1-phenyl-7-(trifluoromethyl)-1,2-dihydro-1,8-naphthyridine-3-ca rboxylate

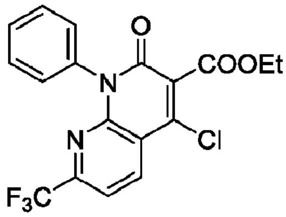

Ethyl 4-hydroxy-2-oxo-1-phenyl-7-(trifluoromethyl)-1 ,2-dihydro-1 ,8-naphthyridine-3-carboxylate (250 mg) was dissolved in phosphorus oxychloride (1 mL), which was heated and reacted at 90 °C for 2 h. LC-MS showed that the reaction was completed. The system was added dropwise to an appropriate amount of ice water, the pH of which was adjusted with saturated sodium carbonate solution to weak basicity, and it was extracted with ethyl acetate. The organic phases were combined, dried over anhydrous sodium sulfate, filtrated, and concentrated, and the residue was purified by column chromatography to give 180 mg of the target compound.
MS (ESI) m/z $(M+H)^+$=397.0.

Step 4: Preparation of ethyl 4-(methylamino)-2-oxo-1-phenyl-7-(trifluoromethyl)-1 ,2-dihydro-1 ,8-naphthyridi ne-3-carboxylate

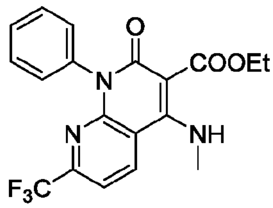

Ethyl 4-chloro-2-oxo-1-phenyl-7-(trifluoromethyl)-1,2-dihydro-1,8 -naphthyridine-3-carboxylate (160 mg) was dissolved in methylamine (2 M in THF, 1 mL), and the reaction was carried out at room temperature for 1 h. LC-MS showed that the reaction was completed. The system was concentrated under reduced pressure and used directly in the next step without further purification.
MS (ESI) m/z $(M+H)^+$=392.1.
$^1H$ NMR (400 MHz, DMSO-$d_6$) δ 8.77 (d, *J* = 8.3 Hz, 1H), 7.79 (d, *J* = 8.2 Hz, 1H), 7.71 (d, *J* = 5.4 Hz, 1H), 7.49 (t, *J* = 7.3 Hz, 2H), 7.45-7.39 (m, 1H), 7.28-7.19 (m, 2H), 4.23 (q, *J* = 7.1 Hz, 2H), 2.92 (d, *J*= 4.8 Hz, 3H), 1.27 (t, *J* = 7.1 Hz, 3H).

Step 5: Preparation of 4-(methylamino)-1-phenyl-7-(trifluoromethyl)-1 ,8-naphthyridin-2(1 A/l-one

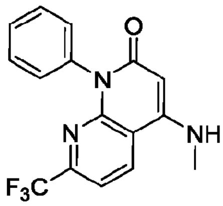

Ethyl

4-(methylamino)-2-oxo-1-phenyl-7-(trifluoromethyl)-1,2-dihydro-1,8-naphthyridi ne-3-carboxylate (100 mg) was dissolved in ethanol/water (8 mL, 4:2), into which sodium hydroxide solution (1.3 mL, 1 M) was added, and it was

heated and reacted at 60°C overnight. TLC showed that the reaction was completed, and the system was concentrated to remove ethanol under reduced pressure, into which an appropriate amount of water was added, and it was extracted with ethyl acetate. The organic phases were combined, backwashed three times with saturated sodium chloride solution, dried over anhydrous sodium sulfate, filtered, and concentrated, and the crude product was purified by column chromatography to give 70 mg of the title compound.

Step 6: Preparation of 3-bromo-4-(methylamino)-1-phenyl-7-(trifluoromethyl)-1,8-naphthyridin-2(1H)-o ne

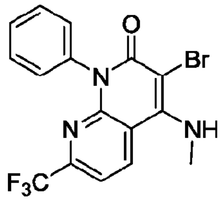

4-(Methylamino)-1-phenyl-7-(trifluoromethyl)-1,8-naphthyridin-2(1*H*)-one (370.0 mg) was dissolved in dichloromethane (4 mL), and the system was cooled down to 0 °C, into which liquid bromine (186 mg) was added. The system was naturally restored to room temperature and reacted for 1 h. TLC showed that the reaction was completed. The system was poured into water (20 mL), the pH of which was adjusted with saturated sodium bicarbonate solution to about 8, and it was extracted with ethyl acetate. The organic phases were combined, backwashed with saturated sodium chloride solution once, dried over anhydrous sodium sulfate, filtrated, and concentrated. The resulting crude product was separated on a chromatographic column to give 270.0 mg of the title compound.
MS (ESI) m/z $(M+H)^+$ = 398.0, 400.0.

**Preparation Example 2: Preparation of 3-bromo-1-(4-chlorophenyl)-4-(methylamino)-7-(trifluoromethyl)-1,8-naphthyridin -2(1*H*)-one**

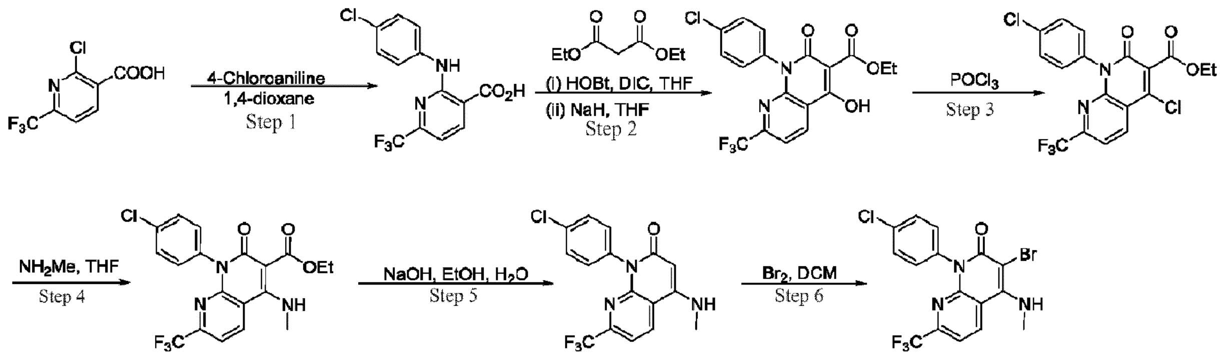


The compound of this Preparation Example was obtained by preparation with reference to the similar method in the above Preparation Example 1.
MS (ESI) m/z $(M+H)^+$=432.0, 434.0.

**Preparation Example 3: Preparation of 4-amino-3-bromo-1-(4-chlorophenyl)-7-(trifluoromethyl)-1,8-naphthyridin-2(1*H*)-one**

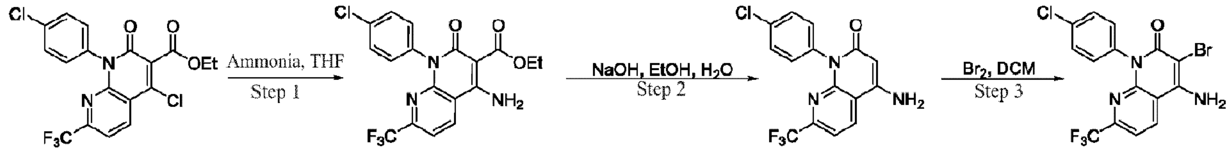


The compound of this Preparation Example was obtained by preparation with reference to the similar method in the above Preparation Example 2.

MS (ESI) m/z $(M+H)^+$ = 417.9, 419.9.

$^1H$ NMR (400 MHz, DMSO-$d_6$) δ 8.88 (d, *J* = 8.2 Hz, 1H), 7.80 (d, *J* = 8.2 Hz, 1H), 7.59 - 7.53 (m, 2H), 7.37 (s, 2H), 7.34 - 7.29 (m, 2H).

**Preparation Example** 4: **Preparation of 2-(furan-2-yl)-N-(p-tolyl)acetamide**

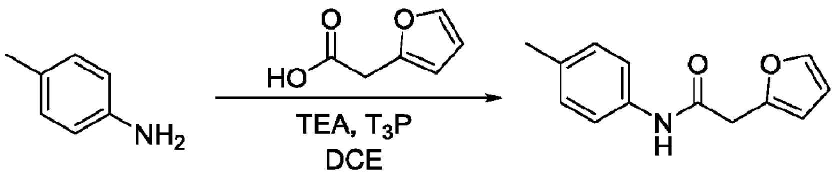


p-Toluidine (500 mg), 2-(furan-2-yl)acetic acid (700 mg), triethylamine (1.9 mL) and 1-propylphosphonic anhydride (5.89 mL, 50% in EA) were dissolved in 1,2-dichloroethane (15 mL), and reacted at 65°C for 2 h. LCMS showed that the reaction of the raw materials was completed. The reaction solution was cooled down to room temperature, into which saturated sodium bicarbonate solution was added, and it was extracted with ethyl acetate. The organic phases were combined, dried over anhydrous sodium sulfate, filtered, and concentrated, and the crude product was purified by column chromatography to give 800 mg of the title compound.

MS (ESI) m/z $(M+H)^+$= 216.0.

**Preparation Example 5: Preparation of 6-(4,4,5,5-tetramethyl-1,3,2-dioxaborolan-2-yl)-1H-benzo[d]imidazol-2-amine**

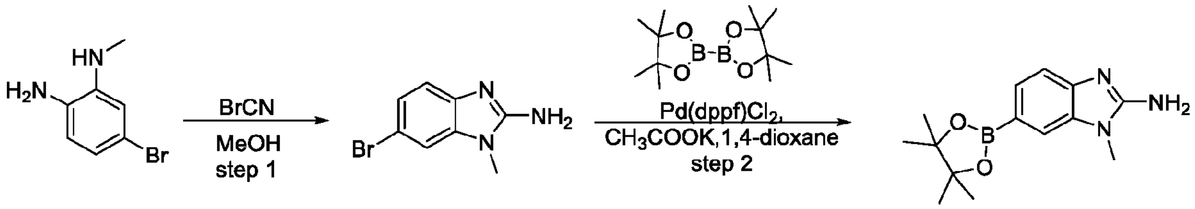


Step 1: Preparation of 6-bromo-1-methyl-1H-benzo[d]imidazol-2-amine

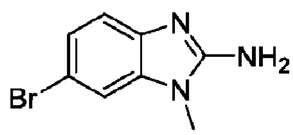

4-Bromo-2-methylaminoaniline (600 mg) was dissolved in methanol (30 mL), into which cyanogen bromide (630 mg) was added, and the reaction was carried out at room temperature for 2 h. After the disappearance of the raw materials was monitored by LCMS, the reaction solution was concentrated under reduced pressure, and the residue was purified by column chromatography to yield 486 mg of the target compound.

MS (ESI) m/z $(M+H)^+$= 225.9.

Step 2: Preparation of 6-(4,4,5,5-tetramethyl-1,3,2-dioxaborolan-2-yl)-1H-benzo[d]imidazol-2-amine

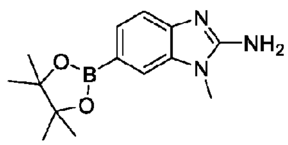

In a nitrogen atmosphere, 6-bromo-1-methyl-1H-benzo[d]imidazol-2-amine (100 mg) was dissolved in 1,4-dioxane (15 mL), into which 11'-bis(diphenylphosphino)ferrocene palladium chloride (65 mg), bis(pinacolato)diboron (223 mg), and potassium acetate (122 mg) were added, and it was heated to react at 90 °C for 4 h. After the disappearance of the raw materials was monitored by LCMS, the reaction solution was concentrated under reduced pressure, and the residue was purified by normal phase column chromatography to give 100 mg of the target compound.

MS (ESI) m/z $(M+H)^+$= 274.1.

**Preparation Example** 6: **Preparation** of **ethyl 2-(1-methyl-1H-benzo[d]imidazol-6-yl)acetate**

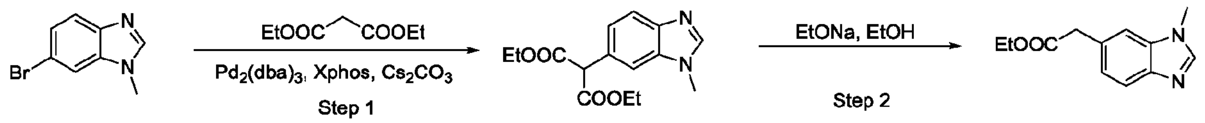


Step 1 : Preparation of diethyl 2-(1-methyl-1H-benzo[d]imidazol-6-yl)malonate

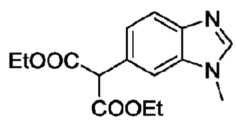

6-Bromo-1-methyl-1H-benzo[d]imidazole (300 mg), diethyl malonate (450 mg), tris[dibenzylideneacetone]dipalladium (65 mg), 2-dicyclohexylphosphino-2',4',6'-triisopropylbiphenyl (35 mg), and cesium carbonate (700 mg) were added into toluene, and it was heated up to 100 °C and reacted with stirring for 6 h under the protection of nitrogen. LCMS showed that the reaction was completed, it was concentrated under reduced pressure, and the residue was purified by column chromatography to give 300 mg of the target compound.

MS (ESI) m/z $(M+H)^+$=291.1.

Step 2: Preparation of ethyl 2-(1-methyl-1H-benzo[d]imidazol-6-yl)acetate

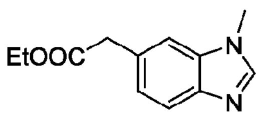

Diethyl 2-(1-methyl-1H-benzo[d]imidazol-6-yl)malonate (300 mg) was dissolved in anhydrous ethanol (10 mL), into which sodium ethoxide (680 mg) was added, and the reaction was performed with heating reflux and stirring under the protection of nitrogen for 4 h. LCMS showed that the reaction was completed, and the reaction was terminated with acetic acid, it was concentrated under reduced pressure, and the residue was purified by column chromatography to give 200 mg of the target compound.

MS (ESI) m/z $(M+H)^+$=219.1.

**Preparation Example** 7: **preparation of 1-(tetrahydro-2H-pyran-2-yl)-5-(4,4,5,5-tetramethyl-1,3,2-dioxaborolan-2-yl)-1H -benzo[d][1,2,3]triazole and**

**1-(tetrahydro-2H-pyran-2-yl)-6-(4,4,5,5-tetramethyl-1,3,2-dioxaborolan-2-yl)-1H -benzo[d][1,2,3] triazole**

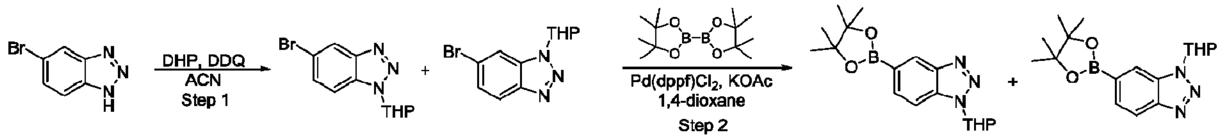


Step 1: Preparation of 5-bromo-1-(tetrahydro-2H-pyran-2-yl)-1H-benzo[d][1,2,3]triazole and 6-bromo-1-(tetrahydro-2H-pyran-2-yl)-1H-benzo[d][1 ,2,3]triazole 5-Bromo-1H-benzotriazole (300 mg) was dissolved in acetonitrile (10 mL), into which 3,4-dihydro-2H-pyran (150 mg) and 2,3-dichloro-5,6-dicyanobenzoquinone (35 mg) were added, and the reaction was performed with stirring under the protection of nitrogen at room temperature for 6 h. LCMS showed that the reaction was completed, and it was concentrated under reduced pressure, and the residue was purified by column chromatography to give 350 mg of the target compound.

MS (ESI) m/z $(M-84+H)^+$= 198.1.

Step 2: Preparation of 1-(tetrahydro-2H-pyran-2-yl)-5-(4,4,5,5-tetramethyl-1,3,2-dioxaborolan-2-yl)-1 H-benzo[d][1,2,3] triazole and 1-(tetrahydro-2H-pyran-2-yl)-6-(4,4,5,5-tetramethyl-1,3,2-dioxaborolan-2-yl)-1 H-benzo[d][1,2,3]triazole

The target compound (350 mg) obtained in the previous step was dissolved in 1,4-dioxane (10 mL), into which 1,1'-bis(diphenylphosphino)ferrocene palladium chloride (45 mg), bis(pinacolato)diboron (630 mg), and potassium acetate (245 mg) were added, and it was heated to react at 90 °C for 4 h under the protection of nitrogen. After the disappearance of the raw materials was monitored by LCMS, the reaction solution was concentrated under reduced pressure. The residue was purified by normal phase column chromatography to yield 270 mg of the target compound.
MS (ESI) m/z $(M-84+H)^+$= 246.1.

**Preparation Example 8: Preparation of 4-amino-3-bromo-1-(4-chloro-3-fluorophenyl)-7-(trifluoromethyl)-1,8-naphthyridi n-2(1H)-one**

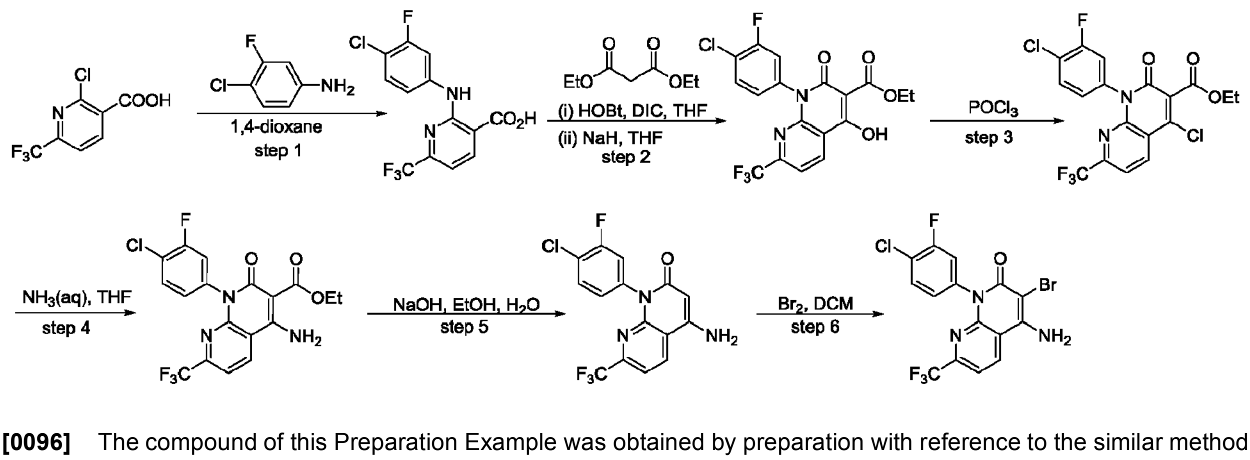


The compound of this Preparation Example was obtained by preparation with reference to the similar method in the above Preparation Examples 2 & 3.
MS (ESI) m/z $(M+H)^+$=435.9, 437.9.

**Preparation Example 9: Preparation of 4-amino-3-bromo-1-(4-chloro-2-fluorophenyl)-7-(trifluoromethyl)-1,8-naphthyridi n-2(1H)-one**

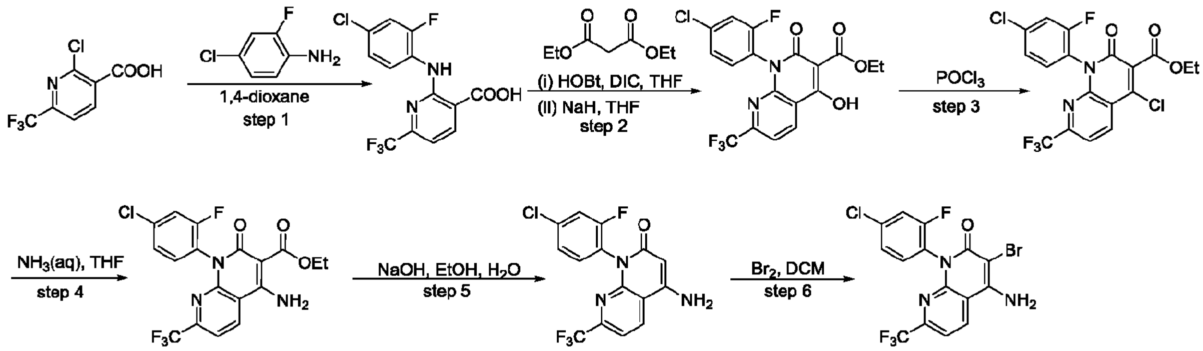


The compound of this Preparation Example was obtained by preparation with reference to the similar method in the above Preparation Examples 2 & 3.
MS (ESI) m/z $(M+H)^+$= 435.9, 437.9.

**Preparation Example 10: Preparation of 4-amino-3-bromo-1-(4-methylphenyl)-7-(trifluoromethyl)-1,8-naphthyridin-2(1H)-one**

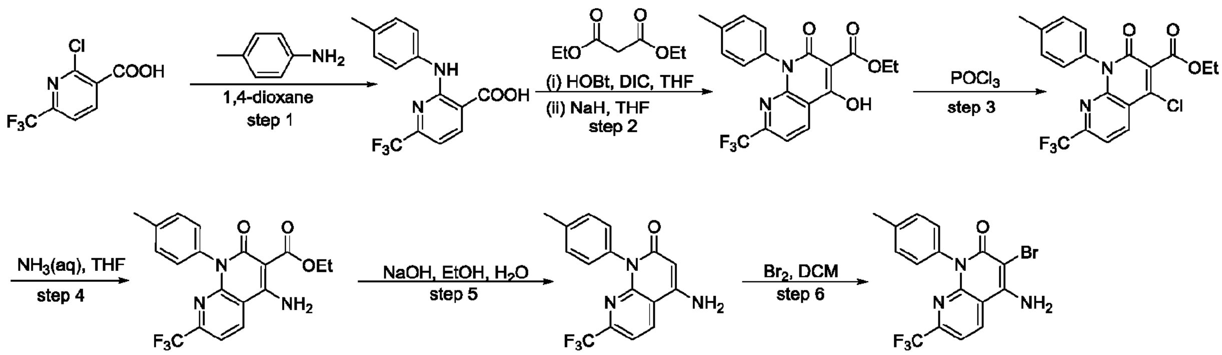


The compound of this Preparation Example was obtained by preparation with reference to the similar method in the above Preparation Examples 2 & 3.
MS (ESI) m/z $(M+H)^+$= 398.0, 400.0.

**Preparation Example 11: Preparation of 1H-benzo[d][1,2,3]triazol-1-yl 2-((4-bromophenyl)amino)-6-(trifluoromethyl)nicotinate**

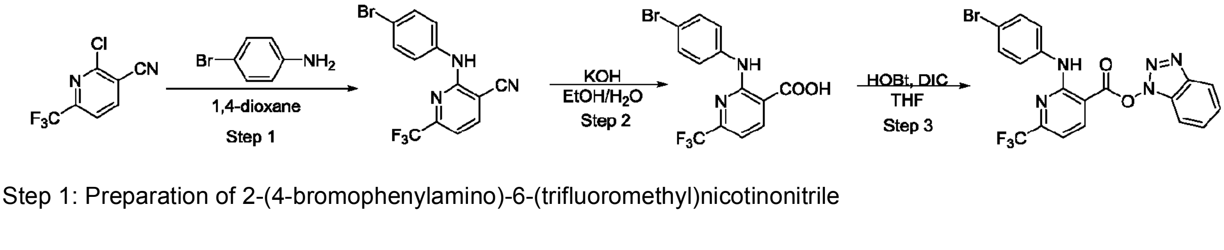


Step 1: Preparation of 2-(4-bromophenylamino)-6-(trifluoromethyl)nicotinonitrile

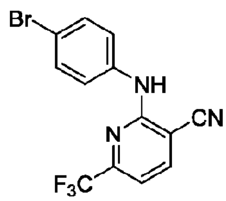

2-Chloro-6-(trifluoromethyl)nicotinonitrile (1.0 g) was dissolved in 1,4-dioxane (5 mL), into which 4-bromoaniline (1.1 g) was added, and the reaction was initiated by microwave at 120°C for 5 h. LCMS showed that most of the raw materials were reacted completely. The system was concentrated under reduced pressure, and the residue was purified by column chromatography to give 1.5 g of the title compound.

MS (ESI) m/z $(M+H)^+$ = 342.0, 344.0.

Step 2: Preparation of 2-(4-bromophenylamino)-6-(trifluoromethyl)nicotinic acid

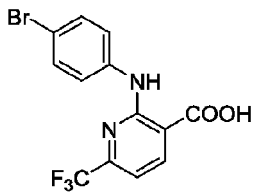

2-(4-Bromophenylamino)-6-(trifluoromethyl)nicotinonitrile (1.5 g) was dissolved in ethanol/water, into which potassium hydroxide (1.2 g) was added in one portion, and the system was reacted under reflux for 6 h. LCMS showed that the raw materials were depleted, the pH of which was adjusted with 2 N hydrochloric acid to about 5, and it was extracted with ethyl acetate. The organic phases were combined, dried over anhydrous sodium sulfate, filtrated, and concentrated under reduced pressure, and the resulting crude product was purified by column chromatography to give 1.4 g of the product.

MS (ESI) m/z $(M+H)^+$= 361.0, 363.0.

Step 3: Preparation of 1H-benzo[d][1,2,3]triazol-1-yl 2-((4-bromophenyl)amino)-6-(trifluoromethyl)nicotinate

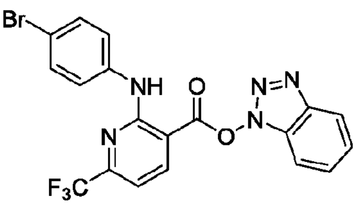

2-(4-Bromophenylamino)-6-(trifluoromethyl)nicotinic acid (1.4 g) was dissolved in tetrahydrofuran, into which N,N-diisopropylcarbodiimide (730 mg) and 1-hydroxybenzotriazole (790 mg) were added, and the reaction was carried out at room temperature for 2 h. TLC showed that the reaction was completed, and it was concentrated under reduced pressure, and the residue was purified by column chromatography to give 1.6 g of the target compound.

**Preparation Example 12: Preparation of 1H-benzo[d][1,2,3]triazol-1-yl 2-((4-tolyl)amino)-6-(trifluoromethyl)nicotinate**

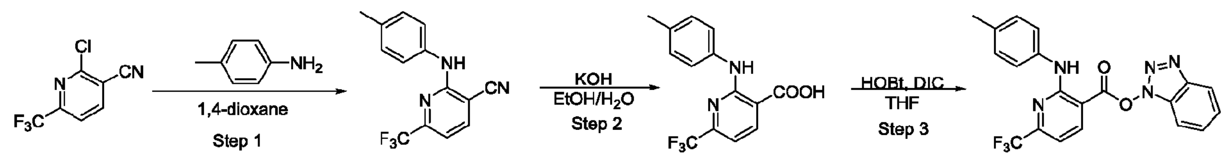


The compound of this Preparation Example was obtained by preparation with reference to the similar method in the above Preparation Example 11 .

**Example 1: Preparation of 4-(methylamino)-3-(oxazol-5-yl)-1-phenyl-7-(trifluoromethyl)-1,8-naphthyridin-2( 1*H*)-one**

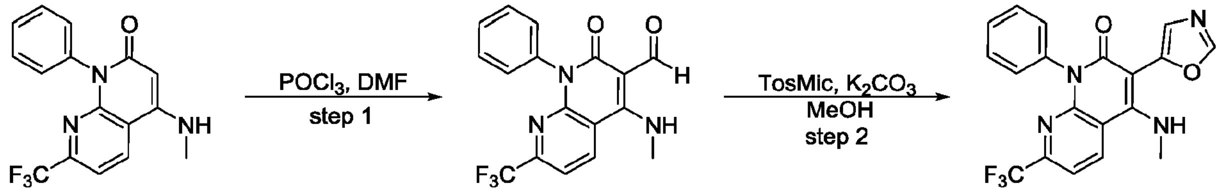


Step 1: Preparation of 4-(methylamino)-2-oxo-1-phenyl-7-(trifluoromethyl)-1 ,2-dihydro-1 ,8-naphthyridi ne-3-carbaldehyde

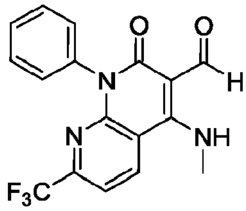

4-(Methylamino)-1-phenyl-7-(trifluoromethyl)-1,8-naphthyridin-2(1*H*)-one (70 mg) was dissolved in *N,N*-dimethylformamide (1 mL), into which phosphorus oxychloride (321 μL) was added slowly at 0 °C. The system was restored to room temperature and reacted for 3 h. TLC showed that the raw materials were depleted, and the system was quenched by adding water (100 mL), the pH of which was adjusted to about 8 with saturated sodium bicarbonate solution, and it was extracted with ethyl acetate. The organic phases were combined, backwashed three times with saturated sodium chloride solution, dried over anhydrous sodium sulfate, filtered and concentrated. The resulting crude product was purified by preparative TLC to obtain 50 mg of the target.

Step 2: Preparation of 4-(methylamino)-3-(oxazol-5-yl)-1 -phenyl-7-(trifluoromethyl)-1,8-naphthyridin-2 (1*H*)-one

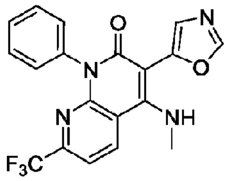

4-(Methylamino)-2-oxo-1-phenyl-7-(trifluoromethyl)-1,2-dihydro-1,8-naphthyridi ne-3-carbaldehyde (30 mg) was dissolved in methanol (2 mL), into which tosylmethyl isocyanide (50 mg) and potassium carbonate (36 mg) were added, and the reaction was carried out at room temperature for 30 min. TLC showed that the raw materials were reacted completely. The system was purified by preparative HPLC and freeze-dried to yield 0.81 mg of the title compound.

MS (ESI) m/z $(M+H)^+$ = 387.0.

$^1H$ NMR (400 MHz, DMSO-$d_6$) δ 8.85 (d, *J* = 8.3 Hz, 1H), 8.42 (s, 1H), 7.72 (dd, *J* = 8.3, 1.7 Hz, 1H), 7.54 (t, *J* = 7.5 Hz, 2H), 7.46 (dd, *J* = 8.2, 6.3 Hz, 1H), 7.30 - 7.24 (m, 2H), 6.77 (d, *J* = 2.9 Hz, 1H), 4.03 (s, 3H).

**Example 2: Preparation of 4-(methylamino)-1-phenyl-3-(thiazol-2-yl)-7-(trifluoromethyl)-1,8-naphthyridin-2( 1*H*)-one**

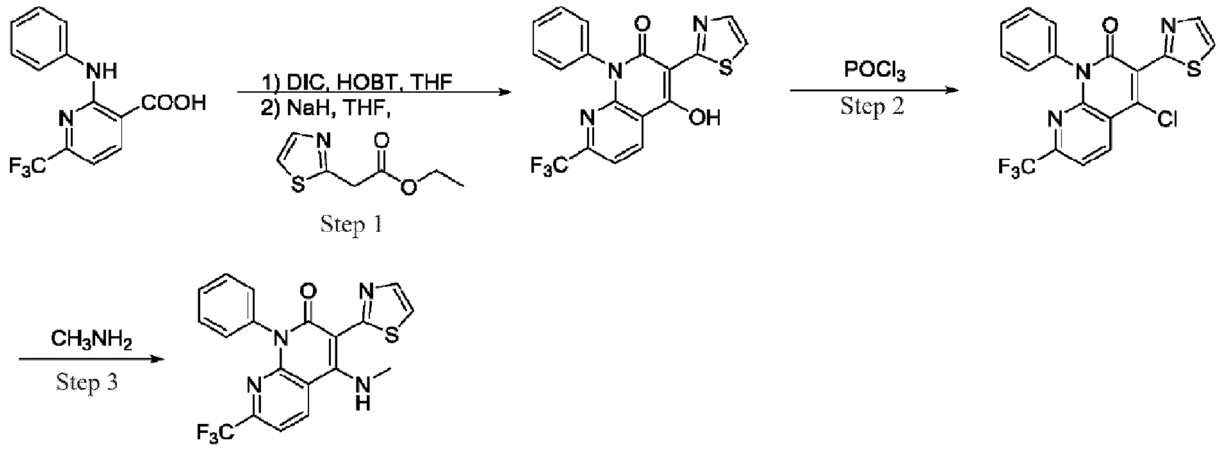


Step 1: Preparation of 4-hydroxy-1-phenyl-3-(thiazol-2-yl)-7-(trifluoromethyl)-1,8-naphthyridin-2(1*H*)-o ne

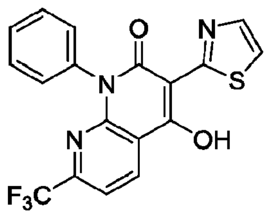

2-(Phenylamino)-6-(trifluoromethyl)nicotinic acid (220.0 mg) was dissolved in tetrahydrofuran (10 mL), into which 1-hydroxybenzotriazole (105.0 mg) and *N,N'*-diisopropylcarbodiimide (120.0 mg) were added sequentially. The reaction was carried out at room temperature for 1 h, and the reaction solution was reserved for use. Sodium hydride (204.0 mg) was placed into a 100 mL eggplant bottle, and it was replaced with argon for three times. At 0 °C, a solution (2 mL) of 2-ethyl acetate-thiazole (290.0 mg) in tetrahydrofuran was added thereto slowly, and the system was naturally restored to room temperature and reacted for 1 h. The above-mentioned reserved reaction solution was then added slowly into the system, and the reaction was continued for 2 h at room temperature. TLC showed that the raw materials were reacted completely. The system was poured into water (50 mL), extracted with ethyl acetate for 3 times. The organic phases were combined, backwashed with saturated sodium chloride solution once, dried over anhydrous sodium sulfate, filtered, and concentrated, and the resulting crude product was purified by column chromatography to obtain 250.0 mg of the title compound.

MS (ESI) m/z $(M+H)^+$= 390.0.

Step 2: Preparation of 4-chloro-1-phenyl-3-(thiazol-2-yl)-7-(trifluoromethyl)-1,8-naphthyridin-2(1*H*)-on e

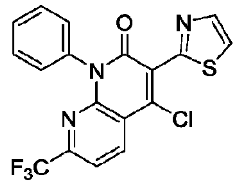

4-Hydroxy-1-phenyl-3-(thiazol-2-yl)-7-(trifluoromethyl)-1,8-naphthyridin-2(1*H*)-one (260.0 mg) was dissolved in phosphorus oxychloride (5.0 mL) and heated at 90°C to react for 1 h. TLC showed that the raw materials were reacted completely. The system was cooled down to room temperature, and the reaction solution was slowly dripped into water, the pH of which was adjusted with saturated sodium bicarbonate solution to about 9, and it was extracted with ethyl acetate for 3 times. The organic phases were combined, backwashed with saturated sodium chloride solution once, dried over anhydrous sodium sulfate, filtrated, and concentrated. The resulting crude product was purified by column chromatography to yield 230.0 mg of the title compound.

MS (ESI) m/z $(M+H)^+$= 408.0.

Step 3: Preparation of 4-(methylamino)-1 -phenyl-3-(thiazol-2-yl)-7-(trifluoromethyl)-1,8-naphthyridin-2 (1*H*)-one

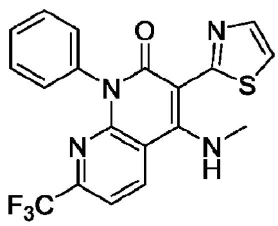

4-Chloro-1-phenyl-3-(thiazol-2-yl)-7-(trifluoromethyl)-1,8-naphthyridin-2(1*H*)-on e (50.0 mg) was dissolved in *N*-methylpyrrolidone (2 mL), into which methylamine (0.3 mL, 2.0 mol/L in THF) was added. The reaction of the system was initiated by microwave at 150 °C for 1 h. TLC showed that the raw materials were depleted. Water (10 mL) was added thereto, and it was extracted with ethyl acetate. The organic phases were combined, backwashed with saturated sodium chloride solution once, dried over anhydrous sodium sulfate, filtered and concentrated. The resulting crude product was purified by preparative HPLC and freeze-dried to yield 12.6 mg of the title compound.

MS (ESI) m/z $(M+H)^+$ = 403.0.

$^1H$ NMR (400 MHz, DMSO-$d_6$) $\delta$ 12.07 (s, 1H), 9.07 (d, J = 8.4 Hz, 1H), 7.98 (d, J = 3.4 Hz, 1H), 7.74 - 7.66 (m, 2H), 7.57 - 7.46 (m, 3H), 7.34 - 7.28 (m, 2H), 3.56 (d, J = 5.5 Hz, 3H).

**Example 3: Preparation of 4-amino-1-(4-chlorophenyl)-3-(1*H*-pyrrol-1-yl)-7-(trifluoromethyl)-1,8-naphthyridi n-2(1*H*)-one**

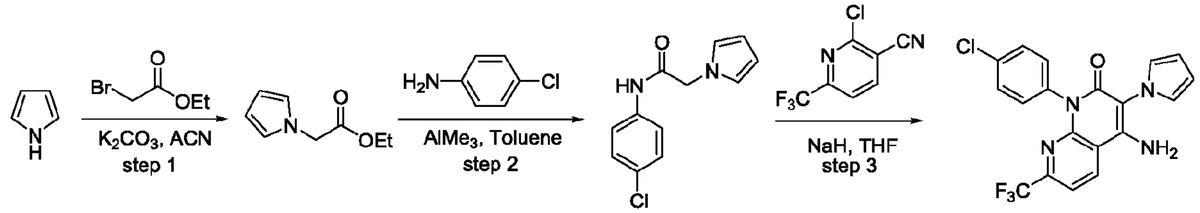


Step 1: Preparation of ethyl 2-(1*H*-pyrrol-1-yl)acetate

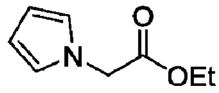

Pyrrole (1.03 mL), ethyl 2-bromoacetate (1.6 mL) and potassium carbonate (4 g) were dissolved in acetonitrile and reacted at 80 °C for 3 h. After the disappearance of the raw materials was monitored by LCMS, the reaction was terminated by addition of saturated aqueous ammonium chloride solution (5 mL), and extracted with ethyl acetate (10 mL*3) for three times. The organic phases were combined, dried over anhydrous sodium sulfate, filtered, and concentrated, and the residue was purified by column chromatography to obtain 600 mg of the target compound.

Step 2: Preparation of *N*-(4-chlorophenyl)-2-(1*H*-pyrrol-1-yl)acetamide

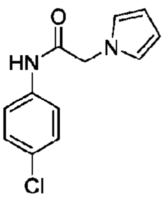

Ethyl 2-(1*H*-pyrrol-1-yl)acetate (5 g), p-chloroaniline (14.2 g) and trimethylaluminum (2 M, 16 mL) were dissolved in toluene, and reacted at 100 °C for 1 h. After the disappearance of the raw materials was monitored by LCMS, it was cooled down to room temperature, into which methanol: dichloromethane = 1:1 (50 mL) were added. It was heated under reflux for 15 min, filtered and concentrated. The residue was purified by column chromatography to give 1.5 g of the target compound.

Step 3: Preparation of 4-amino-1-(4-chlorophenyl)-3-(1*H*-pyrrol-1-yl)-7-(trifluoromethyl)-1,8-naphthyri din-2(1*H*)-one

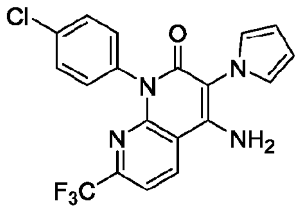

*N*-(4-chlorophenyl)-2-(1*H*-pyrrol-1-yl)acetamide (200 mg) was dissolved in tetrahydrofuran, into which sodium hydride (68 mg) was added under the condition of ice-water bath at 0 °C, which was reacted for 30 min. 2-Chloro-6-(trifluoromethyl)nicotinonitrile (352 mg) was added and reacted for 1 h. The disappearance of the raw materials was monitored by LCMS. The reaction was terminated by the addition of water (5 mL), and it was extracted with ethyl acetate for three times (10 mL*3). The organic phases were combined, dried over anhydrous sodium sulfate, filtered, and concentrated. The residue was purified by reversed-phase preparative HPLC to give 15 mg of the target compound.

MS (ESI) m/z $(M+H)^+$ = 404.9。

$^1$H NMR (400 MHz, DMSO-$d_6$) δ 8.91 (d, *J* = 8.2 Hz, 1H), 7.81 (d, *J* = 8.2 Hz, 1H), 7.60-7.50 (m, 2H), 7.37-7.28 (m, 2H), 6.74 (t, *J* = 2.1 Hz, 2H), 6.70 (s, 2H), 6.23 (t, *J* = 2.1 Hz, 2H).

**Example 4: Preparation of 4-(methylamino)-1-phenyl-3-(thien-3-yl)-7-(trifluoromethyl)-1,8-naphthyridin-2(1*H* )-one**

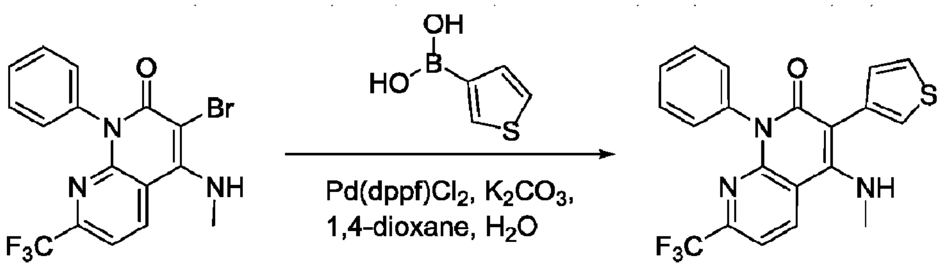


3-Bromo-4-(methylamino)-1-phenyl-7-(trifluoromethyl)-1 ,8-naphthyridin-2(1 H)-o ne (30.0 mg), thien-3-ylboronic acid (50.0 mg), and potassium carbonate (53.0 mg) were dissolved in 1 ,4-dioxane/water (1.0 mL/0.2 mL) under nitrogen atmosphere, into which [1,1'-bis(diphenylphosphino)ferrocene]palladium dichloride (27 mg) was added, and the system was reacted at 100 °C for 1 h. TLC showed that the raw materials were depleted. Water (10 mL) was added thereto, and it was extracted with ethyl acetate. The organic phases were combined, backwashed once with saturated sodium chloride solution, dried over anhydrous sodium sulfate, filtered and concentrated. The resulting crude product was purified by preparative HPLC and freeze-dried to give 13.0 mg of the title compound.

MS (ESI) m/z $(M+H)^+$ = 402.1.

$^1$H NMR (400 MHz, DMSO-d6) δ 8.76 (d, J = 8.2 Hz, 1H), 7.76 (d, J = 8.3 Hz, 1H), 7.53 - 7.36 (m, 5H), 7.27 - 7.14 (m, 3H), 7.00 (q, J = 5.0 Hz, 1H), 2.42 (d, J = 4.9 Hz, 3H).

**Example 5: Preparation of 4-(methylamino)-1-phenyl-3-(4*H*-1,2,4-triazol-3-yl)-7-(trifluoromethyl)-1,8-napht hyridin-2(1*H*)-one**

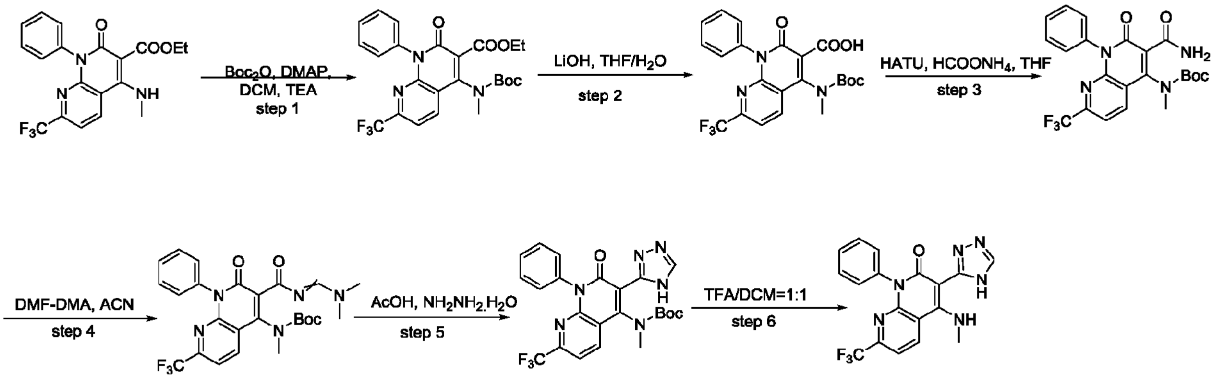


Step 1: Preparation of ethyl 4-((tert-butoxycarbonyl)(methyl)amino)-2-oxo-1-phenyl-7-(trifluoromethyl)-1,2-di hydro-1,8-naphthyridine-3-carboxylate

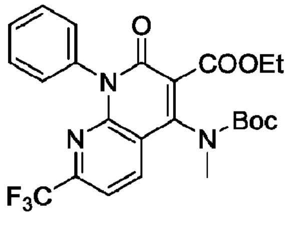

Ethyl

4-(methylamino)-2-oxo-1-phenyl-7-(trifluoromethyl)-1,2-dihydro-1,8-naphthyridi ne-3-carboxylate (770 mg) was dissolved in dichloromethane (30 mL), into which triethylamine (398 mg), di-tert-butyl dicarbonate (859 mg), and 4-dimethylaminopyridine (120 mg) were added, and it was reacted for one hour at room temperature, and the system

changed from turbidity to clarification. LC-MS showed that the reaction was completed. The system was concentrated under reduced pressure, and the residue was purified by column chromatography to give 900 mg of the target compound.

MS (ESI) m/z $(M+H)^+$= 492.1.

Step 2: Preparation of 4-((tert-butoxycarbonyl)(methyl)amino)-2-oxo-1-phenyl-7-(trifluoromethyl)-1,2-di hydro-1,8-naphthyridine-3-carboxylic acid

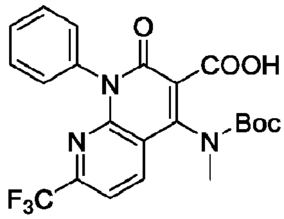

Ethyl

4-((tert-butoxycarbonyl)(methyl)amino)-2-oxo-1-phenyl-7-(trifluoromethyl)-1,2-di hydro-1,8-naphthyridine-3-carboxylate (300 mg) was dissolved in a mixed solution of tetrahydrofuran/water (8 mL/2 mL), into which lithium hydroxide (88 mg) was added, and it was heated to 55 ° C for 4 h. LC-MS showed that the reaction was completed. The pH of the system was adjusted to weak acidity with oxalic acid, extracted with ethyl acetate. The organic phases were combined, dried over anhydrous sodium sulfate, filtered, and concentrated. The residue was purified by column chromatography to yield 210 mg of the target compound.

MS (ESI) m/z $(M+H)^+$= 464.1.

Step 3: Preparation of tert-butyl (3-carbamoyl-2-oxo-1-phenyl-7-(trifluoromethyl)-1,2-dihydro-1,8-naphthyridin-4 -yl)(methyl)carbamate

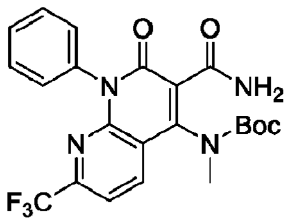

4-((tert-butoxycarbonyl)(methyl)amino)-2-oxo-1-phenyl-7-(trifluoromethyl)-1,2-di hydro-1,8-naphthyridine-3-carboxylic acid (210 mg) was dissolved in tetrahydrofuran (20 mL), into which 2-(7-azabenzotriazol-1-yl)-N,N,N',N'-tetramethyluronium hexafluorophosphate (862 mg) and ammonium formate (171 mg) were added. The reaction was carried out overnight at room temperature. LC-MS showed that the reaction was completed. The system was added with an appropriate amount of water, extracted with ethyl acetate. The organic phases were combined, backwashed once with saturated sodium chloride solution, dried over anhydrous sodium sulfate, filtered, and concentrated, and the resulting crude product could be used directly in the next reaction without further purification.

MS (ESI) m/z $(M+H)^+$= 463.1.

Step 4: Preparation of tert-butyl (3-(((dimethylamino)methylene)carbamoyl)-2-oxo-1-phenyl-7-(trifluoromethyl)-1, 2-dihydro-1,8-naphthyridin-4-yl)(methyl)carbamate

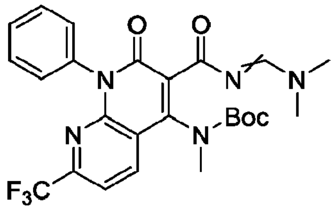

The crude tert-butyl (3-carbamoyl-2-oxo-1-phenyl-7-(trifluoromethyl)-1,2-dihydro-1 ,8-naphthyridin-4 -yl)(methyl)carbamate (200 mg) was dissolved in acetonitrile (10 mL), into which N,N-dimethylformamide dimethyl acetal (103 mg) was added, and the reaction was carried out at room temperature for 1 h. LC-MS showed that the reaction was completed. The system was concentrated under reduced pressure, and the resulting crude product was used directly in the next reaction without further purification.

MS (ESI) m/z $(M+H)^+$= 518.2.

Step 5: Preparation of tert-butyl (2-oxo-1-phenyl-3-(4*H*-1,2,4-triazol-3-yl)-7-(trifluoromethyl)-1,2-dihydro-1,8-n aphthyridin-4-yl)(methyl)carbamate

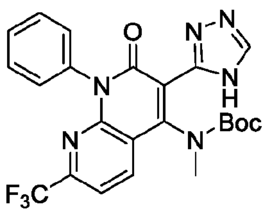

The crude tert-butyl (3-(((dimethylamino)methylene)carbamoyl)-2-oxo-1-phenyl-7-(trifluoromethyl)-1 , 2-dihydro-1,8-naphthyridin-4-yl)(methyl)carbamate (210 mg) was dissolved in acetic acid (6 mL), into which hydrazine hydrate (51 mg) was added, and it was heated up to 90 °C and reacted for 2 h. LC-MS showed that the reaction was completed. The system was concentrated under reduced pressure and the resulting crude product was used directly in the next reaction without further purification.

MS (ESI) m/z $(M+H)^+$= 487.2.

Step 6: Preparation of 4-(methylamino)-1-phenyl-3-(4*H*-1,2,4-triazol-3-yl)-7-(trifluoromethyl)-1,8-nap hthyridin-2(1*H*)-one

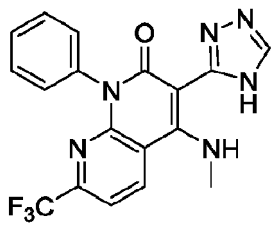

The crude tert-butyl (2-oxo-1-phenyl-3-(4*H*-1,2,4-triazol-3-yl)-7-(trifluoromethyl)-1,2-dihydro-1,8-n aphthyridin-4-yl)(methyl)carbamate (180 mg) was dissolved in a mixed solvent of trifluoroacetic acid/dichloromethane (2 mL/2 mL), which was reacted at room temperature for 5 h. LC-MS showed that the reaction was completed. The system was concentrated under reduced pressure, and the residue was purified by reversed-phase preparative HPLC to give 6 mg of the target compound.

MS (ESI) m/z $(M+H)^+$ = 387.1 .

$^1H$ NMR (400 MHz, DMSO-$d_6$) $\delta$ 13.88 (s, 1H), 10 15 (s, 1H), 8.98 (d, *J* = 8.4 Hz, 1H), 8.12 (s, 1H), 7.74 (d, *J* = 8.3 Hz, 1H), 7.52 (t, *J* = 7.5 Hz, 2II), 7.45 (t, *J* = 7.4 Hz, 111), 7.29 (d, *J* = 7.6 Hz, 2H), 3.22 (s, 3H).

**Example 6: Preparation of 4-(methylamino)-3-(1,3,4-oxadiazol-2-yl)-1-phenyl-7-(trifluoromethyl)-1,8-naphth yridin-2(1*H*)-one**

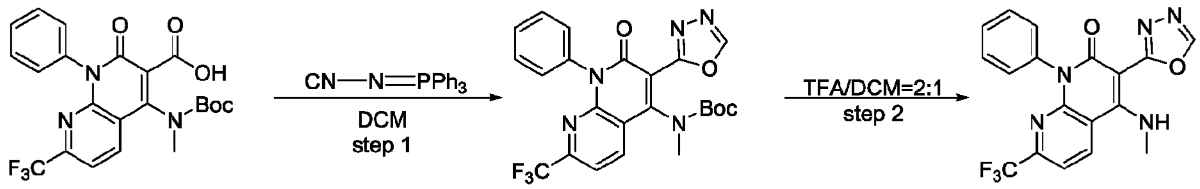


Step 1: Preparation of tert-butyl (3-(1,3,4-oxadiazol-2-yl)-2-oxo-1-phenyl-7-(trifluoromethyl)-1,2-dihydro-1,8-n aphthyridin-4-yl)(methyl)carbamate

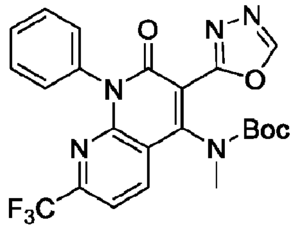

4-((tert-butoxycarbonyl)(methyl)amino)-2-oxo-1-phenyl-7-(trifluoromethyl)-1,2-di hydro-1,8-naphthyridine-3-carboxylic acid (40 mg) was dissolved in dichloromethane (20 mL), into which the solution of (isocyanoimino)triphenylphosphorane (52 mg) in dichloromethane was slowly added, and the reaction was carried out overnight at room temperature. LC-MS showed that the reaction was completed. The system was concentrated under reduced pressure, and the residue was purified by column chromatography to give 20 mg of the target compound.

MS (ESI) m/z $(M-55)^+$= 432.1.

Step 2: Preparation of 4-(methylamino)-3-(1,3,4-oxadiazol-2-yl)-1-phenyl-7-(trifluoromethyl)-1,8-naph thyridin-2(1*H*)-one

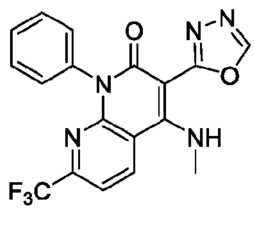

Tert-butyl (3-(1,3,4-oxadiazol-2-yl)-2-oxo-1-phenyl-7-(trifluoromethyl)-1,2-dihydro-1,8-n aphthyridin-4-yl)(methyl)carbamate (20 mg) was dissolved in a mixed solution of trifluoroacetic acid/dichloromethane (1 mL/2 mL), and the reaction was carried out at room temperature for 4 h. LC-MS showed that the reaction was completed. The system was concentrated to remove the dichloromethane, and saturated sodium carbonate aqueous solution was used to adjust the pH to weak basicity. It was extracted with ethyl acetate. The organic phases were combined, dried over anhydrous sodium sulfate, filtered, and concentrated. The residue was purified by reversed-phase preparative HPLC to give 7 mg of the target compound.

MS (ESI) m/z $(M+H)^+$=388.1.

$^1$H NMR (400 MHz, DMSO-$d_6$) δ 9.39 (s, 1H), 8.85 (d, *J* = 8.3 Hz, 1H), 8.12 (d, *J* = 5.4 Hz, 1H), 7.85 (d, *J* = 8.3 Hz, 1H), 7.49 (dd, *J* = 8.2, 6.6 Hz, 2H), 7.45-7.39 (m, 1H), 7.30-7.23 (m, 2H), 2.42 (d, *J* = 4.9 Hz, 3H).

**Example 7: Preparation of 4-(methylamino)-3-(oxazol-2-yl)-1-phenyl-7-(trifluoromethyl)-1,8-naphthyridin-2( 1*H*)-one**

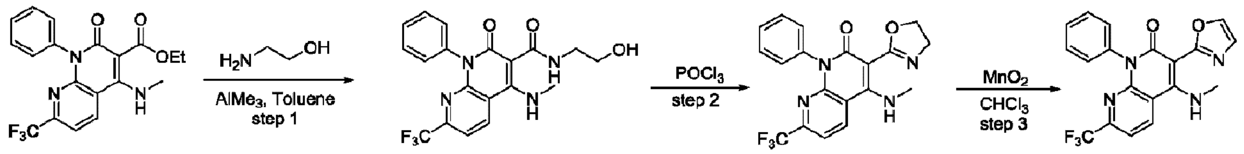


Step 1: Preparation of N-(2-hydroxyethyl)-4-(methylamino)-2-oxo-1-phenyl-7-(trifluoromethyl)-1,2-dihy dro-1,8-naphthyridine-3-carboxamide

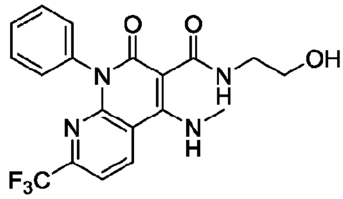

Ethyl

4-(methylamino)-2-oxo-1-phenyl-7-(trifluoromethyl)-1,2-dihydro-1,8-naphthyridi ne-3-carboxylate (220 mg) was dissolved in toluene under nitrogen atmosphere, into which trimethylaluminum (61 mg) and ethanolamine (51 mg) were added at 0 °C, the system was restored to room temperature after addition, and it was continued to be heated for reaction at 80 °C for 2 h. Water was added to quench the reaction, and an appropriate amount of dichloromethane and methanol was added thereto for refluxing 15 min, which was filtrated, and concentrated to give 200 mg of the crude product of the target compound.

Step 2: Preparation of 3-(4,5-dihydrooxazol-2-yl)-4-(methylamino)-1-phenyl-7-(trifluoromethyl)-1,8-na phthyridin-2(1*H*)-one

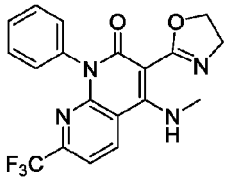

N-(2-hydroxyethyl)-4-(methylamino)-2-oxo-1-phenyl-7-(trifluoromethyl)-1 ,2-dihy dro-1 ,8-naphthyridine-3-carboxamide (20 mg) was dissolved in phosphorus oxychloride, and the reaction was carried out at 80 °C for 1 h. It was monitored by LCMS that the reaction was completed, the phosphorus oxychloride was removed by concentration, the pH of which was adjusted with saturated sodium bicarbonate solution to be approximately alkaline, and it was extracted with ethyl acetate. The organic phases were combined, dried over anhydrous sodium sulfate, filtered and concentrated, and the resulting crude product was purified by preparative HPLC to give 10 mg of the target compound.

MS (ESI) m/z $(M+H)^+$= 389.1 .

$^1$H NMR (400 MHz, DMSO-$d_6$) $\delta$ 8.77 (d, *J* = 8.3 Hz, 1H), 7.78 (d, *J* = 8.1 Hz, 2H), 7.62 - 7.31 (m, 3H), 7.21 (d, *J* = 7.1 Hz, 2H), 4.29 (t, *J* = 9.5 Hz, 2H), 3.89 (t, *J* = 9.5 Hz, 2H), 2.97 (d, *J* = 4.8 Hz, 3H).

Step 3: Preparation of 4-(methylamino)-3-(oxazol-2-yl)-1-phenyl-7-(trifluoromethyl)-1,8-naphthyridin-2 (1H)-one

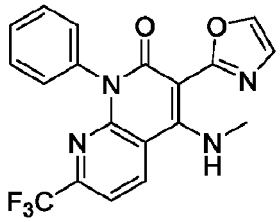

3-(4,5-dihydrooxazol-2-yl)-4-(methylamino)-1-phenyl-7-(trifluoromethyl)-1,8-na phthyridin-2(1H)-one (100 mg) was dissolved in chloroform (3 mL), into which manganese dioxide (224 mg) was added, and the reaction was initiated by microwave under the heating condition of 100 °C for 3 h. The disappearance of the raw materials was monitored by LCMS, the filtration was performed, and the system was concentrated under reduced pressure. The residue was purified by reversed-phase preparative HPLC to give 0.57 mg of the target compound.

MS (ESI) m/z $(M+H)^+$= 387.1.

**Example 8: Preparation of 3-(1H-imidazol-2-yl)-4-(methylamino)-1-phenyl-7-(trifluoromethyl)-1,8-naphthyrid in-2(1H)-one**

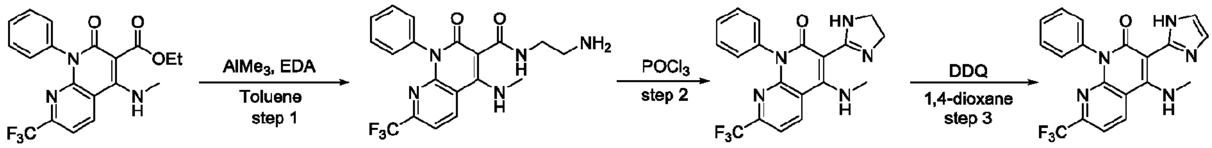


The compounds of this Example were obtained by preparation with reference to the similar method in the above Example 7.

MS (ESI) m/z $(M+H)^+$ = 386.1.

$^1$H NMR (400 MHz, DMSO-$d_6$) δ 14.63 (s, 1H), 9.21 (d, *J* = 8.3 Hz, 1H), 8.73 (d, *J* = 5.2 Hz, 1H), 7.87 (d, *J* = 8.3 Hz, 1H), 7.77 (s, 2H), 7.50 (d, *J* = 7.7 Hz, 2H), 7.45 (t, *J* = 7.3 Hz, 1H), 7.25 (d, *J* = 7.6 Hz, 2H), 2.41 (d, *J* = 4.6 Hz, 3H).

**Example 15: Preparation of 4-(methylamino)-1-phenyl-3-(1*H*-pyrazol-1-yl)-7-(trifluoromethyl)-1,8-naphthyridi n-2(1*H*)-one**

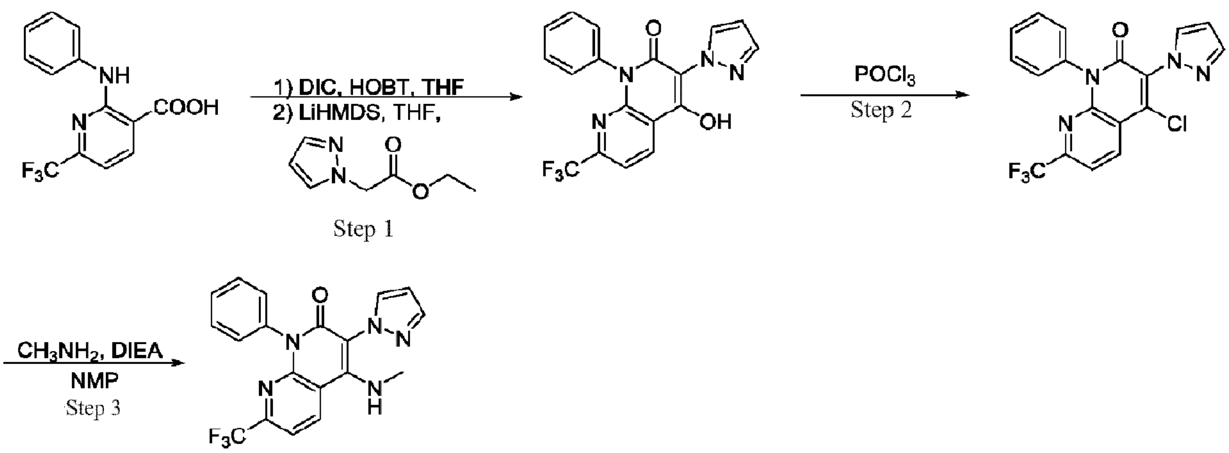


Step 1: Preparation of 4-hydroxy-1-phenyl-3-(1*H*-pyrazol-1-yl)-7-(trifluoromethyl)-1,8-naphthyridin-2(1 *H*)-one

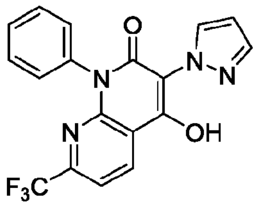

2-(Phenylamino)-6-(trifluoromethyl)nicotinic acid (220.0 mg) was dissolved in tetrahydrofuran (10 mL), and 1-hydroxybenzotriazole (105.0 mg) and *N,N'*-diisopropylcarbodiimide (120.0 mg) were added thereto sequentially. The reaction was carried out at room temperature for 1 h, into which ethyl 2-(1*H*-pyrazol-1-yl)acetate (115 mg) was added. The system was transferred to an ice-water bath, and lithium bis(trimethylsilyl)amide (3 mL, 4.0 mmol) was added thereto under the protection of nitrogen, so as to react for 0.5 h. TLC showed that the raw materials were completely reacted. The reaction was terminated by adding ammonium chloride aqueous solution (5 mL), and it was extracted with ethyl acetate for 3 times. The organic phases were combined, backwashed once with saturated sodium chloride solution, dried over anhydrous sodium sulfate, filtered, and concentrated, and the resulting crude product was purified by column chromatography to yield 250.0 mg of the title compound.

Step 2: Preparation of 4-chloro-1-phenyl-3-(1H-pyrazol-1-yl)-7-(trifluoromethyl)-1,8-naphthyridin-2(1H )-one

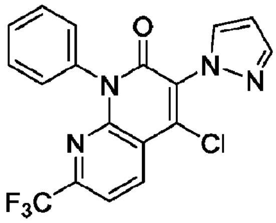

4-Hydroxy-1-phenyl-3-(1H-pyrazol-1-yl)-7-(trifluoromethyl)-1,8-naphthyridin-2(1 H)-one (250 mg) was dissolved in phosphorus oxychloride, and the reaction was carried out at 90 °C for 2 h. After the disappearance of the raw materials was monitored by LCMS, saturated aqueous sodium bicarbonate solution was added to adjust the pH thereof to weak basicity, and it was extracted with ethyl acetate for three times (10 ml*3). The organic phases were combined, dried over anhydrous sodium sulfate, filtered, and concentrated, and the residue was purified by column chromatography to give 78 mg of the target compound.

Step 3: Preparation of 4-(methylamino)-1-phenyl-3-(1H-pyrazol-1-yl)-7-(trifluoromethyl)-1,8-naphthyri din-2(1H)-one

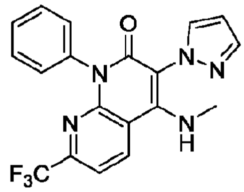

4-Chloro-1-phenyl-3-(1H-pyrazol-1-yl)-7-(trifluoromethyl)-1,8-naphthyridin-2(1H )-one (40 mg) was dissolved in N-methylpyrrolidone (2 mL), methylamine (6.2 mg) and N,N-diisopropylethylamine (0.1 mL) were added thereto dropwise, and the reaction was initiated by microwave under the heating condition of 150 °C for 0.5 h. After the disappearance of the raw materials was monitored by LCMS, the reaction was terminated by adding water (5 mL), and it was extracted with ethyl acetate for three times (10 mL*3). The organic phases were combined, dried over anhydrous sodium sulfate, filtered, and concentrated under reduced pressure, and the residue was purified by reversed-phase preparative HPLC to give 20 mg of the target compound.
MS (ESI) m/z $(M+H)^+$ = 386.0.

$^{1}H$ NMR (400 MHz, DMSO-$d_6$) δ 8.85 (d, *J* = 8.3 Hz, 1H), 7.88-7.80 (m, 2H), 7.63 (d, *J* = 1.8 Hz, 1H), 7.57 (q, *J* = 5.0 Hz, 1H), 7.50 (dd, *J* = 8.3, 6.6 Hz, 2H), 7.46 -7.39 (m, 1H), 7.31-7.20 (m, 2H), 6.42 (t, *J* = 2.1 Hz, 1H), 2.27 (d, *J* = 4.9 Hz, 3H).

**Example 30: Preparation of 4-amino-1-(4-chlorophenyl)-3-(furan-2-yl)-7-(trifluoromethyl)-1,8-naphthyridin-2( 1*H*)-one**

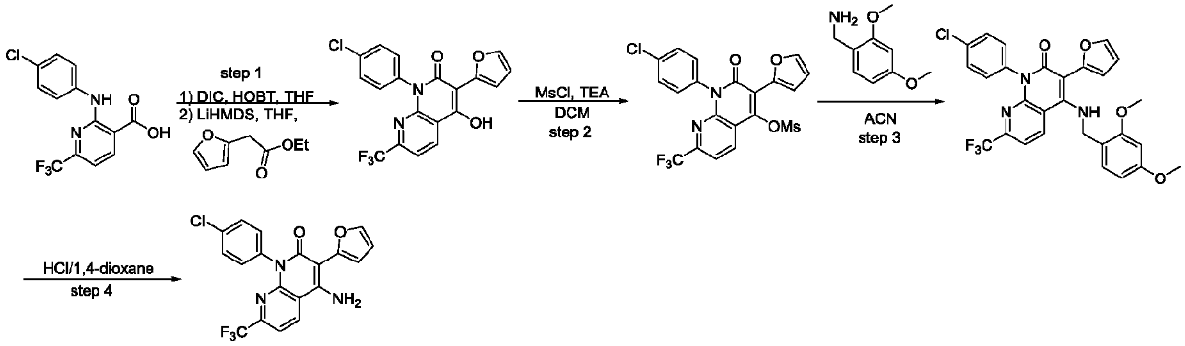


Step 1: Preparation of 1-(4-chlorophenyl)-3-(furan-2-yl)-4-hydroxy-7-(trifluoromethyl)-1,8-naphthyridin -2(1*H*)-one

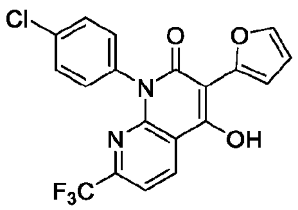

Under argon atmosphere, ethyl 2-furanacetate (225 mg) was dissolved in anhydrous tetrahydrofuran (6 mL), the reaction system was cooled down to -78 °C, and the solution of lithium bis(trimethylsilyl)amide (3.0 mL, 3.0 mmol) in tetrahydrofuran was then added thereto, reacted for 1 h and kept in reserve. 2-((4-chlorophenyl)amino)-6-(trifluoromethyl)nicotinic acid (400 mg) was dissolved in tetrahydrofuran, 1-hydroxybenzotriazole (105.0 mg) and N,N'-diisopropylcarbodiimide (120.0 mg) were added thereto sequentially, and the reaction was carried out at room temperature for 1 h. This reaction solution was added to the above reserved system and moved to room temperature for overnight reaction. LCMS showed that the reaction of the raw materials was completed. The reaction was quenched by adding saturated ammonium chloride solution. The crude product was concentrated and purified by column chromatography to give 120 mg of the title compound.

MS (ESI) m/z $(M+H)^+$= 406.9.

Step 2: Preparation of 1-(4-chlorophenyl)-3-(furan-2-yl)-2-oxo-7-(trifluoromethyl)-1,2-dihydro-1,8-nap hthyridin-4-yl mesylate

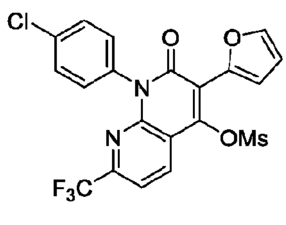

1-(4-Chlorophenyl)-3-(furan-2-yl)-4-hydroxy-7-(trifluoromethyl)-1,8-naphthyridin -2(1*H*)-one (30 mg) was dissolved in dichloromethane (5 mL), triethylamine (0.1 mL) and methanesulfonyl chloride (0.4 mL) were added thereto, and the reaction was carried out for 1 h at room temperature. LCMS showed that the reaction of the raw material was completed. The reaction solution was concentrated to give 35 mg of the title compound.

MS (ESI) m/z $(M+H)^+$= 484.9.

Step 3: Preparation of 1-(4-chlorophenyl)-4-((2,4-dimethoxybenzyl)amino)-3-(furan-2-yl)-7-(trifluorome thyl)-1,8-naphthyridin-2(1*H*)-one

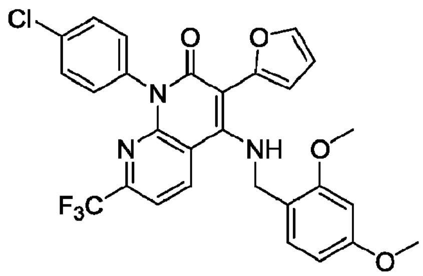

(2,4-Dimethoxyphenyl)methanamine (0.4 mL) was dissolved in acetonitrile (5 mL), the solution of 1-(4-chlorophenyl)-3-(furan-2-yl)-2-oxo-7-(trifluoromethyl)-1,2-dihydro-1,8-nap hthyridin-4-yl mesylate (80 mg) in acetonitrile was added thereto, and the reaction was carried out overnight at room temperature. TLC showed that the raw materials were reacted completely. The reaction solution was concentrated, and the crude product was purified by column chromatography to give 44 mg of the title compound.

MS (ESI) m/z $(M+H)^+$= 555.9.

Step 4: Preparation of 4-amino-1-(4-chlorophenyl)-3-(furan-2-yl)-7-(trifluoromethyl)-1 ,8-naphthyridin-2(1H)-one

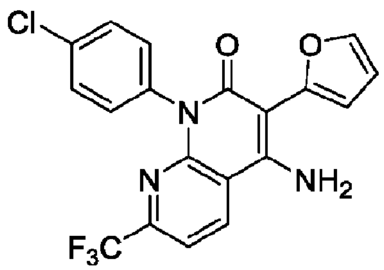

1-(4-Chlorophenyl)-4-((2,4-dimethoxybenzyl)amino)-3-(furan-2-yl)-7-(trifluorom ethyl)-1,8-naphthyridin-2(1*H*)-one (44 mg) was dissolved in 1 ,4-dioxane (5 mL), the solution of hydrogen chloride in 1,4-dioxane (1 mL) was added thereto, and the reaction was carried out at room temperature for 15 min. TLC showed that the raw materials were reacted completely. Saturated sodium bicarbonate solution was added to adjust the pH thereof to weak basicity, and it was extracted with ethyl acetate. The organic phases were combined, dried over anhydrous sodium sulfate, filtrated, and concentrated. The crude product was purified by preparative HPLC to give 2 mg of the title compound.

MS (ESI) m/z $(M+H)^+$ = 405.9.

$^1H$ NMR (400 MHz, DMSO-$d_6$) $\delta$ 8.93 (d, *J* = 8.2 Hz, 1H), 7.83 - 7.75 (m, 2H), 7.60 - 7.54 (m, 2H), 7.46 (s, 2H), 7.39 - 7.29 (m, 2H), 7.03 (d, *J* = 3.4 Hz, 1H), 6.63 (dd, *J* = 3.4, 1.8 Hz, 1H).

**Example 62: Preparation of 4-amino-3-(1H-benzo[d][1,2,3]triazol-6-yl)-1-(4-chlorophenyl)-7-(trifluoromethyl )-1,8-naphthyridin-2(1H)-one**

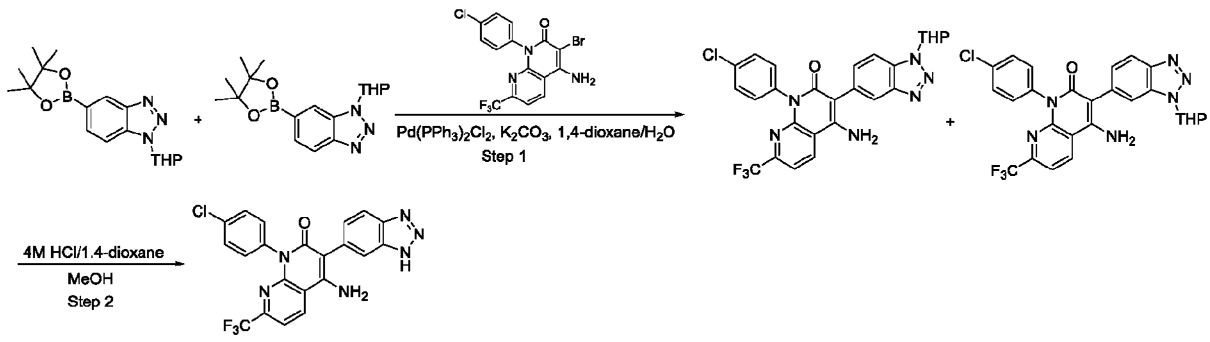


Step 1: Preparation of 4-amino-1-(4-chlorophenyl)-3-(1-(tetrahydro-2H-pyran-2-yl)-1H-benzo[d][1,2, 3]triazol-5-yl)-7-(trifluoromethyl)-1,8-naphthyridin-2(1H)-one and 4-amino-1-(4-chlorophenyl)-3-(1-(tetrahydro-2H-pyran-2-yl)-1H-benzo[d][1,2, 3]triazol-6-yl)-7-(trifluoromethyl)-1,8-naphthyridin-2(1H)-one

In a nitrogen atmosphere, 3-bromo-4-amino-1-(4-chlorophenyl)-7-(trifluoromethyl)-1,8-naphthyridin-2(1*H*)-one (50 mg), the product obtained in Preparation Example 7 (60 mg), and potassium carbonate (33.0 mg) were dissolved in 1,4-dioxane/water (1.0 mL/0.2 mL), [1,1'-bis(diphenylphosphino)ferrocene]palladium dichloride (8 mg) was added thereto, and the system was reacted at 100 °C for 1 h. LCMS showed that the raw materials were depleted. The reaction solution was concentrated under reduced pressure, and the residue was purified by normal phase column chromatography to give 60 mg of the target compound.

MS (ESI) m/z $(M+H)^+$= 541.1.

Step 2: Preparation of 4-amino-3-(1H-benzo[d][1,2,3]triazol-6-yl)-1-(4-chlorophenyl)-7-(trifluorometh yl)-1,8-naphthyridin-2(1H)-one

The product (60 mg) obtained in the previous step was dissolved in methanol (4 mL), hydrochloric acid-1,4-dioxane solution (1 mL, 4 M) was added thereto, and stirred at room temperature for 2 h. After the disappearance of the raw materials was monitored by LCMS, saturated sodium bicarbonate solution was used to adjust the pH thereof to about 8. The mixture was concentrated under reduced pressure, and the residue was purified by reversed-phase preparative HPLC to give 20 mg of the title compound.

MS (ESI) m/z $(M+H)^+$= 457.1.

$^1H$ NMR (400 MHz, DMSO-$d_6$) $\delta$ 15.71 (s, 1H), 8.87 (d, *J* = 8.2 Hz, 1H), 7.96 (s, 1H), 7.82-7.69 (m, 2H), 7.55 (d, *J*= 8.6 Hz, 2H), 7.36-7.33 (m, 3H), 6.65 (s, 2H).

**Example 79: Preparation of 4-amino-1-(4-bromophenyl)-3-(4-methoxyphenyl)-7-(trifluoromethyl)-1,8-naphthy ridin-2(1H)-one**

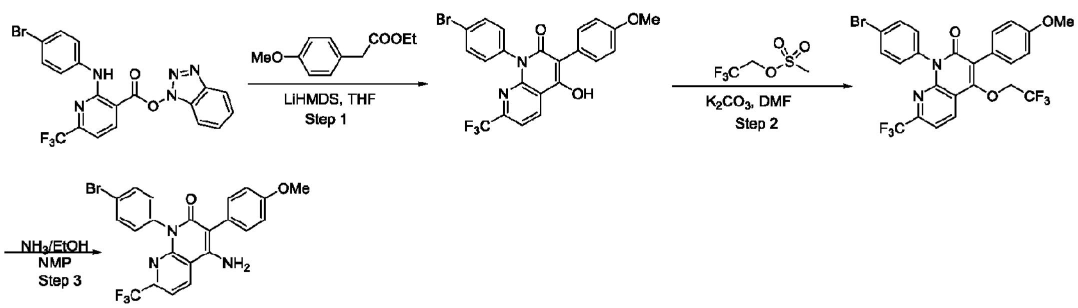


Step 1: Preparation of 1-(4-bromophenyl)-4-hydroxy-3-(4-methoxyphenyl)-7-(trifluoromethyl)-1,8-naph thyridin-2(1H)-one

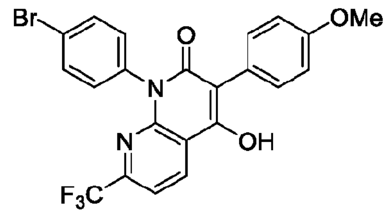

Ethyl 4-methoxyphenylacetate (200 mg) was dissolved in tetrahydrofuran, into which lithium bis(trimethylsilyl)amide (5 mL, 1 M in THF) was added dropwise at -70 °C with stirring for 0.5 h. 1H-benzo[d][1,2,3]triazol-1-yl 2-((4-bromophenyl)amino)-6-(trifluoromethyl)nicotinate (200 mg) was then added thereto, and it was slowly heated to room temperature for 1 h. LC-MS showed that the reaction was completed. The system was quenched by the addition of saturated ammonium chloride aqueous solution, and was extracted with ethyl acetate. The organic phases were combined, dried over anhydrous sodium sulfate, filtered, and concentrated. The residue was purified by column chromatography to give 120 mg of the target compound.

MS (ESI) m/z $(M+H)^+$= 491.1, 493.1.

Step 2: Preparation of 1-(4-bromophenyl)-3-(4-methoxyphenyl)-4-(2,2,2-trifluoroethoxy)-7-(trifluoromet hyl)-1,8-naphthyridin-2(1H)-one

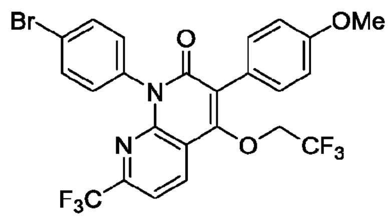

1-(4-Bromophenyl)-4-hydroxy-3-(4-methoxyphenyl)-7-(trifluoromethyl)-1,8-naph thyridin-2(1H)-one (120 mg) was dissolved in N,N-dimethylformamide, potassium carbonate (70 mg) and (2,2,2)-trifluoroethyl methanesulfonate (200 mg) were added thereto at room temperature, and stirred overnight at room temperature. LC-MS showed that the reaction was completed. The system was purified by reversed-phase column chromatography to give 90 mg of the target compound.

MS (ESI) m/z $(M+H)^+$ = 573.1.575.1.

Step 3: Preparation of 4-amino-1-(4-bromophenyl)-3-(4-methoxyphenyl)-7-(trifluoromethyl)-1,8-naphth yridin-2(1H)-one

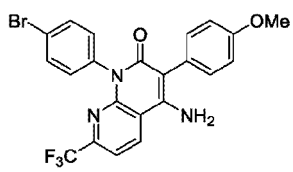

1-(4-Bromophenyl)-3-(4-methoxyphenyl)-4-(2,2,2-trifluoroethoxy)-7-(trifluoromet hyl)-1,8-naphthyridin-2(1H)-one (90 mg) was dissolved in N-methylpyrrolidone, the solution of ammonia in ethanol (1 mL, 2 M in EtOH) was added thereto, and the reaction was initiated by microwave at 150°C for 5 h. LCMS showed that most of the raw materials were reacted completely. The system was purified by reversed-phase preparative HPLC to give 20 mg of the title compound.

MS (ESI) m/z $(M+H)^+$= 490.0, 492.0.

$^1H$ NMR (400 MHz, DMSO-$d_6$) $\delta$ 8.82 (d, *J* =8.2 Hz, 1H), 7.74 (d, *J* =8.2 Hz, 1H), 7.67 (d, *J* =8.6 Hz, 2H), 7.25 (t, *J* =8.1 Hz, 4H), 7.02 (d, *J* = 8.7 Hz, 2H), 6.42 (s, 2H), 3.80 (s, 3H).

The raw materials were prepared according to the similar methods in the above-mentioned Preparation Examples, and the compounds of the Examples in the following table were also obtained according to similar methods in the

preceding Examples:

| Example | Structure | Raw material & Reaction reagent | Reference Examples of the preparation method | MS$(M+H)^+$&$^1$H NMR |
|---|---|---|---|---|
| 9 | 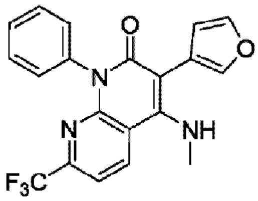 | 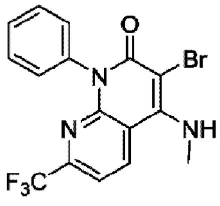 & 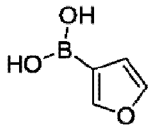 | Example 4 | MS (ESI) m/z $(M+H)^+$ = 386.1. 1H NMR (400 MHz, DMSO-d6) δ 8.76 (d, J = 8.2 Hz, 1H), 7.81 - 7.66 (m, 3H), 7.52 - 7.39 (m, 3H), 7.26 - 7.19 (m, 2H), 6.96 (q, J = 5.1 Hz, 1H), 6.60 (d, J = 1.7 Hz, 1H), 2.64 (d, J = 4.9 Hz, 3H). |
| 10 | 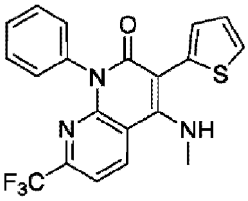 | 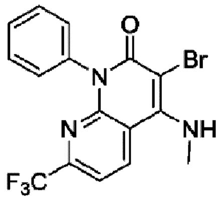 & 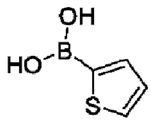 | Exam ple 4 | MS (ESI) m/z $(M+H)^+$ = 402.1. $^1$H NMR (400 MHz, DMSO-d6) δ 8.79 (d, J = 8.3 Hz, 111), 7.78 (d, J = 8.2 Hz, 1H), 7.59 (dd, J = 5.2, 1.2 Hz, 1H), 7.49 (dd, J = 8.3, 6.7 Hz, 2H), 7.45 - 7.38 (m, 1H), 7.28 - 7.17 (m, 3H), 7.08 (dd, J = 5.2, 3.5 Hz, 111), 6.99 (dd, J = 3.5, 1.2 Hz, 1H), 2.53 (s, 3H). |
| 11 | 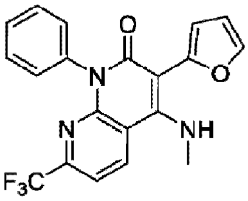 | 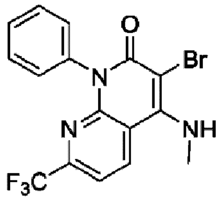 & 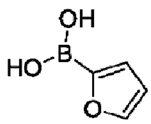 | Example 4 | MS (ES1) m/z $(M+H)^+$ = 386.1. 1H NMR (400 MHz, DMSO-d6) δ 8.77 (d, J = 8.3 Hz, 1H), 7.78 (d, J = 8.2 Hz, 1H), 7.72 (dd, J = 1.9, 0.8 Hz, 1H), 7.50 - 7.39 (m, 4H), 7.25 - 7.21 (m, 2H), 6.54 (dd, J = 3.2, 1.9 Hz, 1H), 6.43 (dd, J = 3.2, 0.8 Hz, 1H), 2.47 (d, J = 4.9 Hz, 3H). |

(continued)

| Example | Structure | Raw material & Reaction reagent | Reference Examples of the preparation method | MS(M+H)$^+$&$^1$H NMR |
|---|---|---|---|---|
| 12 | 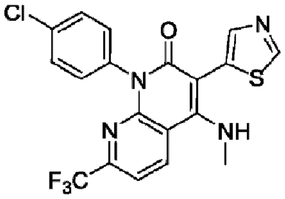 | 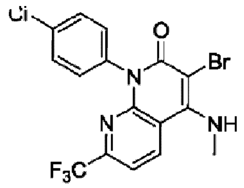 & 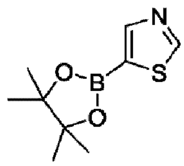 | Example 4 | MS (ESI) m/z $(M+H)^+$ = 437.0. 1H NMR (400 MHz, DMSO-d6) δ 9.13 (s, 1H), 8.83 (d, J = 8.3 Hz, 1H), 7.83 (d, J = 8.3 Hz, 1H), 7.75 (s, 1H), 7.59 - 7.53 (m, 2H), 7.46 (d, J = 5.5 Hz, 1H), 7.36 - 7.29 (m, 2H), 3.34 (s, 3H). |
| 13 | 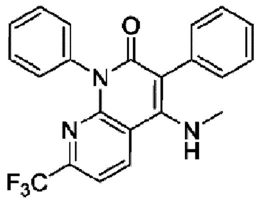 | 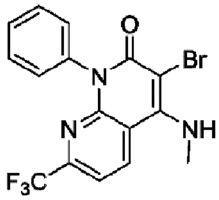 & 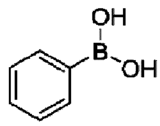 | Example 4 | MS (ESI) m/z $(M+H)^+$= 396.1. 1H NMR (400 MHz, DMSO-d6) δ 8 77 (d, J = 8.3 Hz, 1H), 7.77 (d, J = 8.3 Hz, 1H), 7.53 - 7.44 (m, 2H), 7.43 - 7.26 (m, 6H), 7.25 - 7.20 (m, 2H), 6.94 (q, J = 5.0 Hz, 1H), 2.31 (d, J = 4.9 Hz, 3H). |
| 14 | 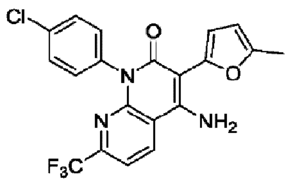 | 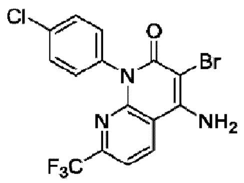 & 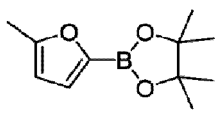 | Exam ple 4 | MS (ESI) m/z $(M+H)^+$= 419.9。 $^1$H NMR (400 MHz, DMSO-$d_6$) δ 8.90 (d, *J* = 8.2 Hz, 1H), 7.79 (d, *J* = 8.3 Hz. 1H), 7.59-7.54 (m, 2H), 7.34-7.32 (m, 3H), 7.31 (d, *J* = 2.1 Hz, 1H), 6.90 (d, *J* = 3.2 Hz, 1H), 6.22 (dd, *J* = 3.2, 1.2 Hz, 1H), 2.40 (s, 3H). |

(continued)

| Example | Structure | Raw material & Reaction reagent | Reference Examples of the preparation method | MS(M+H)+&1H NMR |
|---|---|---|---|---|
| 16 | 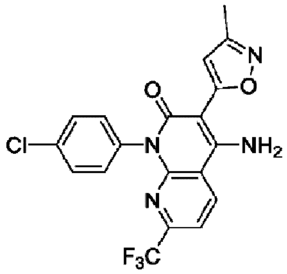 | 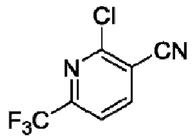 & 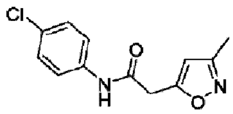 | Example 3 | MS (ES1) m/z $(M+H)^+$= 420.9. $^1H$ NMR (400 MHz, DMSO-$d_6$) $\delta$ 9.00 (d, *J* = 8.2 Hz, 1H), 7.82-7.80 (m, 3H), 7.61-7.53 (m, 2H), 7.37-7.29 (m, 2H), 6.82 (s, 1H), 2.29 (s, 3H). |
| 17 | 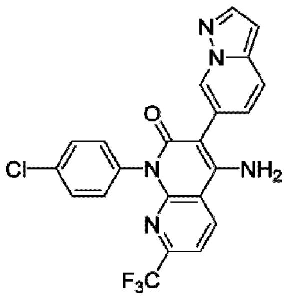 | 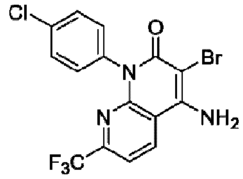 & 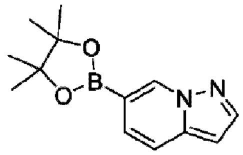 | Example 4 | MS (ESI) m/z $(M+H)^+$= 456.1. $^1H$ NMR (400 MHz, DMSO-$d_6$) $\delta$ 8.87 (d, *J* = 8.2 Hz, 1H), 8.54 (d, *J* = 1.3 Hz, 1H), 8.01 (d, *J* = 2.3 Hz, 1H), 7.79 (d, *J* = 8.2 Hz, 1H), 7.73 (d, *J* = 9.1 Hz, 1H), 7.59-7.51 (m, 2H), 7.36-7.29 (m, 2H), 7.11 (dd, *J* = 9.1, 1.5 Hz, 1H), 6.91 (s, 2H), 6.63 (d, *J* = 2.2 Hz, 1H). |
| 18 | 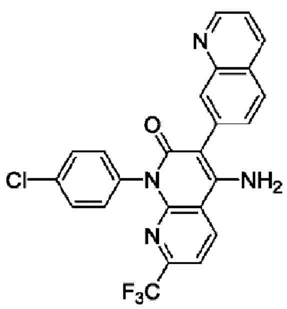 | 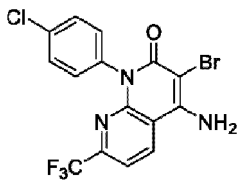 & 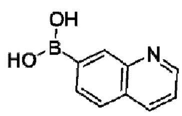 | Example 4 | MS (ESI) m/z $(M+H)^+$= 467.1. $^1H$ NMR (400 MHz, DMSO-$d_6$) $\delta$ 8.95-8.85 (m, 2H), 8.40 (dd, *J* = 8.4, 1.6 Hz, 1H), 8.03 (d, *J* = 8.4 Hz, 1H), 7.99 (d, *J* = 1.5 Hz, 1H), 7.78 (d, *J* = 8.1 Hz, 1H), 7.59-7.49 (m, 4H), 7.39-7.31 (m, 2H), 6.72 (s, 2H). |
| 19 | 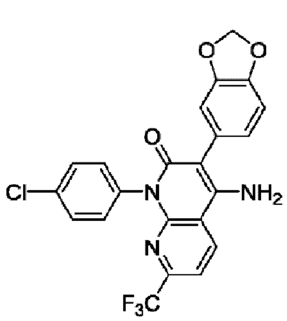 | 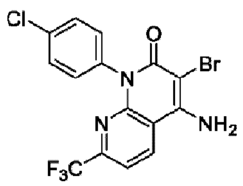 & 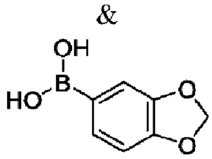 | Example 4 | MS (ESI) m/z $(M+H)^+$= 460.1. $^1H$ NMR (400 MHz, DMSO-$d_6$) $\delta$ 8.82 (d, *J* = 8.2 Hz, 1H), 7.74 (d, *J* = 8.2 Hz, 1H), 7.57-7.49 (m, 2H), 7.33-7.26 (m, 2H), 6.99 (d, *J* = 8.0 Hz, 1H), 6.84 (d, *J* = 1.6 Hz, 1H), 6.79 (dd, *J* = 7.9, 1.7 Hz, 1H), 6.49 (s, 2H), 6.05 (s, 2H). |

(continued)

| Example | Structure | Raw material & Reaction reagent | Reference Examples of the preparation method | MS(M+H)+&1H NMR |
|---|---|---|---|---|
| 20 | 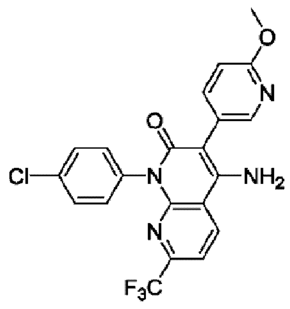 | 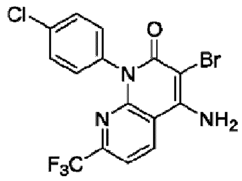 & 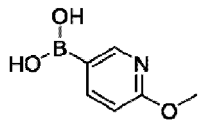 | Example 4 | MS (ESI) m/z $(M+H)^+$= 447.1. $^1$H NMR (400 MHz, DMSO-$d_6$) δ 8.84 (d, *J* = 8.2 Hz, 1H), 8.10 (d, *J* = 2.4 Hz, 1H), 7.76 (d, *J* = 8.2 Hz, 1H), 7.63 (dd, *J* = 8.5, 2.4 Hz, 1H), 7.56-7.51 (m, 2H), 7.33-7.28 (m, 2H), 6.90 (d, *J* = 8.5 Hz, 1H), 6.70 (s, 2H), 3.89 (s, 3H). |
| 21 | 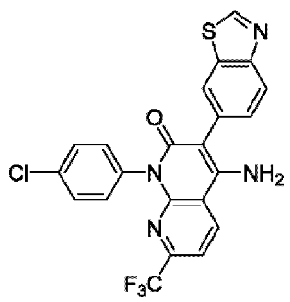 | 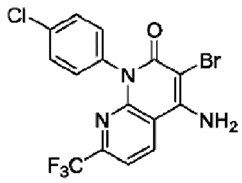 & 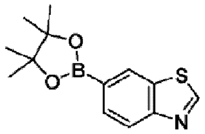 | Example 4 | MS (ESI) m/z $(M+H)^+$= 473.0. $^1$H NMR (400 MHz, DMSO-d6) δ 9.41 (s, 1H), 8.88 (d, *J* = 8.2 Hz, 1H), 8.15 (d, *J* = 8.4 Hz, 1H), 8.12 (d, *J* = 1.5 Hz, 1H), 7.78 (d, *J* = 8.2 Hz, 1H), 7.55 (d, *J* = 8.5 Hz, 2H), 7.49 (dd, *J* = 8.4, 1.6 Hz, 1H), 7.33 (d, *J* = 8.5 Hz, 2H), 6.64 (s, 2H). |
| 22 | 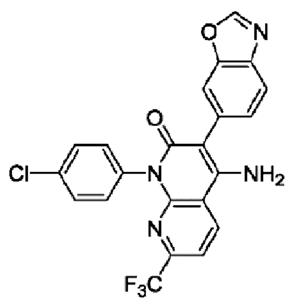 | 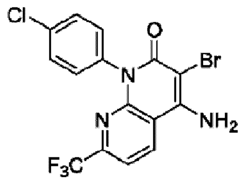 & 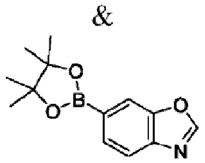 | Example 4 | MS (ESI) m/z $(M+H)^+$= 457.1. $^1$H NMR (400 MHz, DMSO-$d_6$) δ 8.86 (d, *J* = 8.2 Hz, 1H), 8.77 (s, 1H), 7.86 (d, *J* = 8.2 Hz, 1H), 7.77 (d, *J* = 8.2 Hz, 1H), 7.71 (d, *J* = 1.4 Hz. 1H), 7.59-7.51 (m, 2H), 7.38-7.29 (m, 3H), 6.63 (s, 2H). |
| 23 | 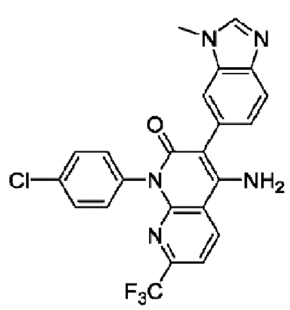 | 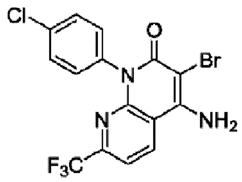 & 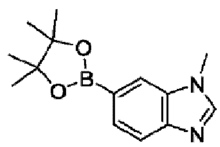 | Example 4 | MS (ESI) m/z $(M+H)^+$ = 470.1. $^1$H NMR (400 MHz, DMSO-$d_6$) δ 8.86 (d, *J* = 8.2 Hz, 1H), 8.21 (s. 1H), 7.77 (d, *J* = 8.1 Hz, 1H), 7.71 (d, *J* = 8.3 Hz, 1H), 7.61-7.50 (m, 3H), 7.33 (d, *J* = 8.4 Hz. 2H), 7.20-7.12 (m, 1H), 6.50 (s, 2H), 3.85 (s, 3H). |

(continued)

| Example | Structure | Raw material & Reaction reagent | Reference Examples of the preparation method | MS$(M+H)^+$&$^1$H NMR |
|---|---|---|---|---|
| 24 | 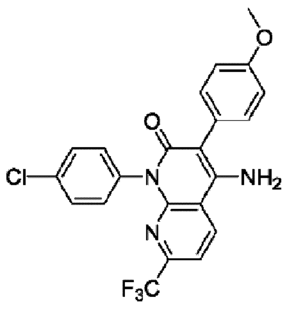 | 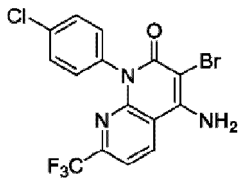 & 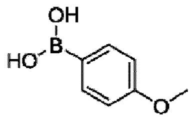 | Example 4 | MS (ESI) m/z $(M+H)^+$ = 446.1. $^1$H NMR (400 MHz, DMSO-$d_6$) $\delta$ 8.83 (d, $J$ = 8.2 Hz, 1H), 7.75 (d, $J$ = 8.1 Hz, 1H), 7.59-7.51 (m, 2H), 7.36-7.22 (m, 4H), 7.08-6.98 (m, 2H), 6.42 (s, 2H), 3.80 (s, 3H). |
| 25 | 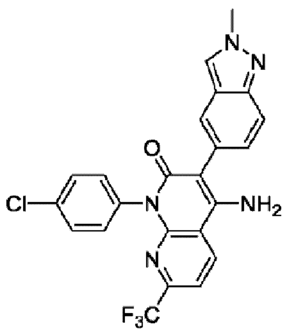 | 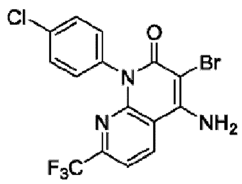 & 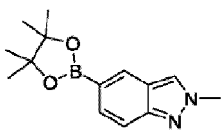 | Example 4 | MS (ESI) m/z $(M+H)^+$= 470.1. $^1$H NMR (400 MHz, DMSO-$d_6$) $\delta$ 8.84 (d, $J$ = 8.2 Hz, 1H), 8.34 (s, 1H), 7.75 (d, $J$ = 8.2 Hz, 1H), 7.65 (s, 1H), 7.62 (d, $J$ = 8.9 Hz, 1H), 7.58-7.50 (m, 2H), 7.37-7.28 (m, 2H), 7.14 (dd, $J$ = 8.9, 1.5 Hz, 1H), 6.47 (s, 2H), 4.19 (s, 3H). |
| 26 | 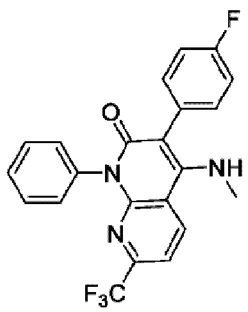 | 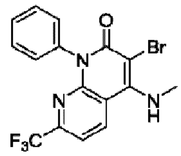 & 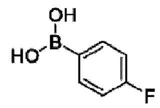 | Example 4 | MS (ES1) m/z $(M+H)^+$= 414.1. $^1$H NMR (400 MHz, DMSO-$d_6$) $\delta$ 8.77 (d, $J$ = 8.3 Hz, 1H), 7.77 (d, $J$ = 8.2 Hz, 1H), 7.47 (dd, $J$ = 8.4, 6.8 Hz, 2H), 7.43-7.29 (m, 3H), 7.25-7.04 (m, 4H), 6.97 (q, $J$ = 5.1 Hz, 1H), 2.34 (d, $J$ = 4.9 Hz, 3H). |
| 27 | 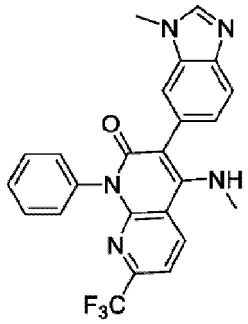 | 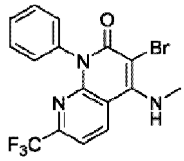 & 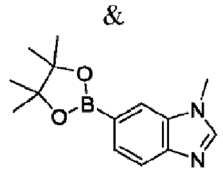 | 27 Example 4 | MS (ES1) m/z $(M+H)^+$= 450.1. $^1$H NMR (400 MHz, DMSO-d6) $\delta$ 8.77 (d, $J$ = 8.3 Hz, 1H), 7.77 (d, $J$ = 8.2 Hz, 1H), 7.47 (dd, $J$ = 8.4, 6.8 Hz, 2H), 7.43-7.29 (m, 3H), 7.25-7.04 (m, 4H), 6.97 (d, $J$ = 5.6 Hz, 1H), 3.83 (s, 3H), 2.34 (d, $J$ = 4.9 Hz, 3H). |

(continued)

| Example | Structure | Raw material & Reaction reagent | Reference Examples of the preparation method | MS(M+H)+&1H NMR |
|---|---|---|---|---|
| 28 | 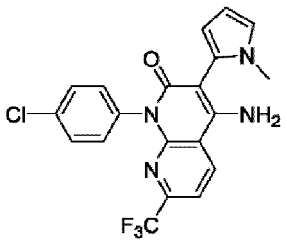 | 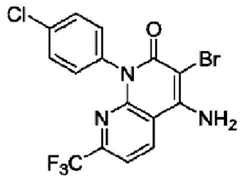 & 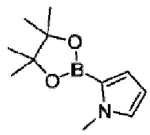 | Example 4 | MS (ESI) m/z $(M+H)^+$= 419.1. $^1H$ NMR (400 MHz, DMSO-$d_6$) $\delta$ 8.63 (d, *J* = 8.0 Hz, 1H), 7.76 (d, *J* = 8.1 Hz, 1H), 7.55 (d, *J* = 8.5 Hz, 2H), 7.36 (d, *J* = 8.4 Hz, 2H), 6.88 (t, *J* = 2.2 Hz, 1H), 6.45 (s, 2H), 6.13-6.01 (m, 2H), 3.45 (s, 3H). |
| 29 | 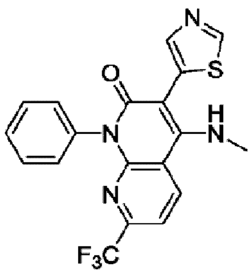 | 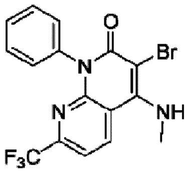 & 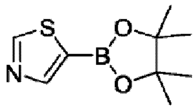 | Example 4 | MS (ESI) m/z $(M+H)^+$= 403.1. $^1H$ NMR (400 MHz, DMSO-d6) δ 9.13 (s, 1H), 8.83 (d, J = 8.3 Hz, 1H), 7.81 (d, J = 8.2 Hz, 1H), 7.76 (s, 1H), 7.49 (t, J = 7.6 Hz, 2H), 7.42 (t, J = 7.2 Hz, 2H), 7.28 - 7.23 (m, 2H), 3.35 (s, 3H). |
| 31 | 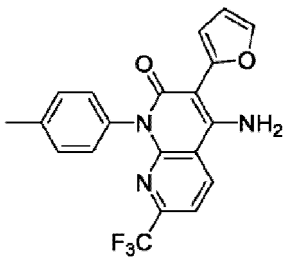 | 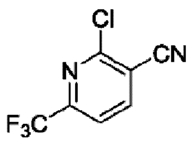 & 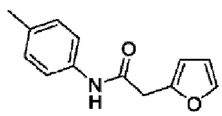 | Example 3 | MS (ESI) m/z $(M+H)^+$ = 386.0。 $^1H$ NMR (400 MHz, DMSO-$d_6$) δ 8.91 (d, *J* = 8.2 Hz, 1H), 7.81 - 7.73 (m, 2H), 7.41 (s, 2H), 7.30 (d, *J* = 7.9 Hz, 2H), 7.12 (d, *J* = 8.0 Hz. 2H), 7.04 (d, *J* = 3.4 Hz, 1H), 6.62 (dd, *J* = 3.4, 1.8 Hz, 1H), 2.41 (s, 3H). |
| 32 | 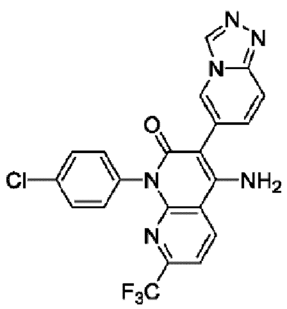 | 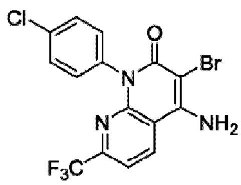 & 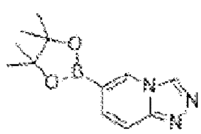 | Example 4 | MS (ESI) m/z $(M+H)^+$ = 457.1. $^1H$ NMR (400 MHz, DMSO-$d_6$) δ 9.25 (s, 1H), 8.87 (d, *J* = 8.2 Hz, 1H), 8.53 (s, 1H), 7.83-7.92 (m, 2H), 7.56 (d, *J* = 8.7 Hz, 2H), 7.33 (d, *J* = 8.6 Hz, 2H), 7.26 (dd, *J* = 9.5, 1.4 Hz, 1H), 7.01 (s, 2H). |

(continued)

| Example | Structure | Raw material & Reaction reagent | Reference Examples of the preparation method | MS(M+H)$^+$&$^1$H NMR |
|---|---|---|---|---|
| 33 | 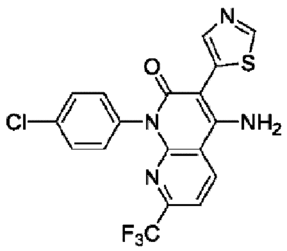 | 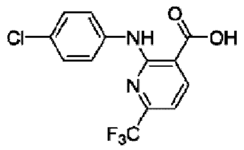 & 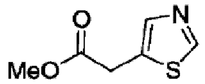 | Example 15 | MS (ESI) m/z (M+H)$^+$ = 423.0 $^1$H NMR (400 MHz, DMSO-d$_6$) $\delta$ 9.16 (s, 1H), 8.92 (d, $J$ = 8.2 Hz, 1H), 8.09 (s, 1H), 7.80 (d, $J$ = 8.2 Hz, 1H), 7.61-7.51 (m, 2H), 7.39-7.29 (m, 2H), 7.23 (s, 2H). |
| 34 | 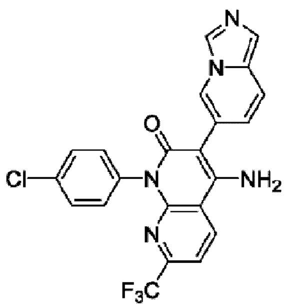 | 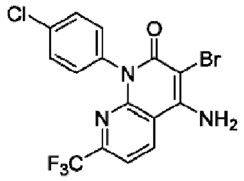 & 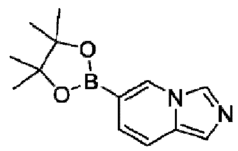 | Example 4 | MS (ESI) m/z (M+H)$^+$= 456.1. $^1$H NMR (400 MHz, DMSO-d$_6$) $\delta$ 8.86 (d, $J$ = 8.2 Hz, 1H), 8.37 (s, 1H), 8.29 (d, $J$ = 1.1 Hz, 1H), 7.78 (d, $J$ = 8.2 Hz, 1H), 7.59-7.53 (m, 3H), 7.35 (s, 1H), 7.34-7.30 (m, 2H), 6.91 (s, 2H), 6.67 (dd, $J$ = 9.3, 1.3 Hz, 1H). |
| 35 | 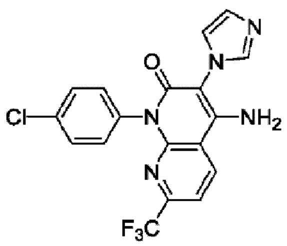 | 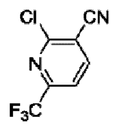 & 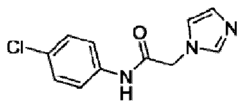 | Example 3 | MS (ESI) m/z (M+H)$^+$ = 406.1. $^1$H NMR (400 MHz, DMSO-$d_6$) $\delta$ 8.94 (d, $J$ = 8.2 Hz, 1H), 7.84 (d, $J$ = 8.2 Hz, 1H), 7.62 (s, 1H), 7.58-7.54 (m, 1H), 7.40-7.27 (m, 2H), 7.12 (d, $J$ = 11.3 Hz, 3H) |
| 36 | 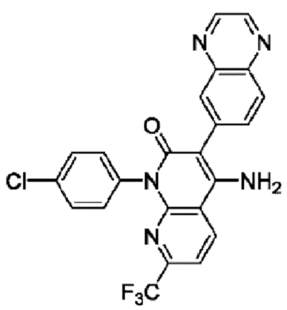 | 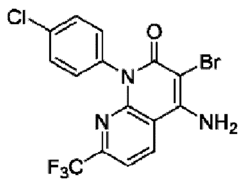 & 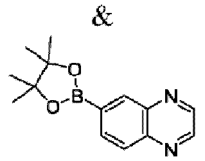 | Example 4 | MS (ESI) m/z (M+H)$^+$= 468.1. $^1$H NMR (400 MHz, DMSO-$d_6$) $\delta$ 8.97 (s, 2H), 8.90 (d, $J$ = 8.1 Hz, 1H), 8.15 (d, $J$ = 8.6 Hz, 1H), 8.06 (d, $J$ = 1.9 Hz, 1H), 7.86-7.77 (m, 2H), 7.60-7.51 (m, 2H), 7.42-7.31 (m, 2H), 6.84 (s, 2H). |

(continued)

| Example | Structure | Raw material & Reaction reagent | Reference Examples of the preparation method | $MS(M+H)^+$&$^1H$ NMR |
|---|---|---|---|---|
| 37 | 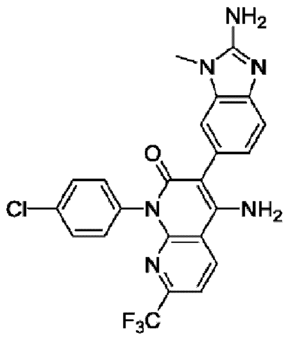 | 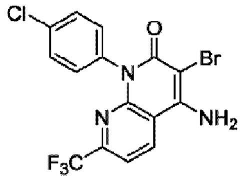 & 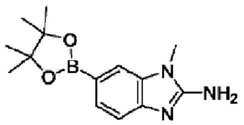 | Preparation Example 5 Example 4 | MS (ESI) m/z $(M+H)^+$= 485.1. $^1H$ NMR (400 MHz, DMSO-$d_6$) $\delta$ 8.82 (d, $J$ = 8.2 Hz, 1H), 7.75 (d, $J$ = 8.2 Hz, 1H), 7.60-7.52 (m, 2H), 7.38-7.28 (m, 2H), 7.19 (d, $J$ = 8.0 Hz, 1H), 7.07 (d, $J$ = 1.6 Hz, 1H), 6.91 (dd, $J$ = 8.1, 1.6 Hz, 1H), 6.41 (s, 2H), 6.35 (s, 2H), 3.50 (s, 3H). |
| 38 | 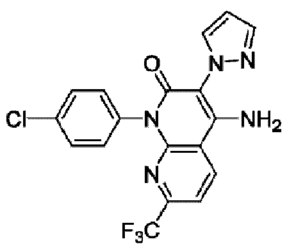 | 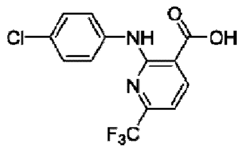 & 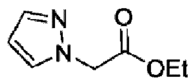 | Example 15 | MS (ESI) m/z $(M+H)^+$= 406.1. $^1H$ NMR (400 MHz, DMSO-$d_6$) $\delta$ 8.94 (d, $J$ = 8.1 Hz, 1H), 7.90 (d, $J$ = 2.4 Hz, 1H), 7.84 (d, $J$ = 8.2 Hz, 1H), 7.76 (d. $J$ = 1.8 Hz, 1H), 7.66-7.52 (m, 2H), 7.44-7.31 (m, 2H), 7.18 (s, 2H), 6.47 (t, $J$ = 2.2 Hz, 1H). |
| 39 | 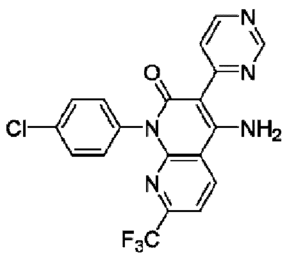 | 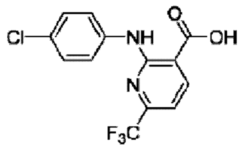 & 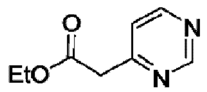 | Preparation Example 6 Example 2 | MS (ES1) m/z $(M+H)^+$= 418.1. $^1H$ NMR (400 MHz, DMSO-$d_6$) $\delta$ 9.23 (d, $J$ = 1.3 Hz, 1H), 8.99 (d, $J$ = 8.2 Hz, 1H), 8.72 (d, $J$ = 5.7 Hz, 1H), 8.45 (dd, $J$ = 5.7, 1.4 Hz, 1H), 7.84 (d, $J$ = 8.2 Hz, 1H), 7.64-7.53 (m, 2H), 7.41-7.31 (m, 2H). |
| 40 | 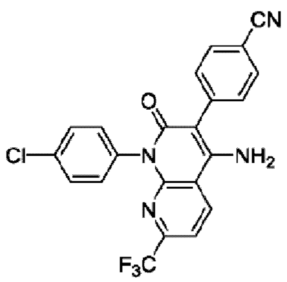 | 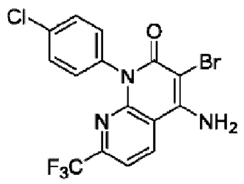 & 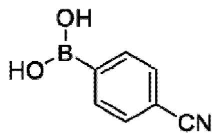 | Example 4 | MS (ESI) m/z $(M+H)^+$ = 441.1. $^1H$ NMR (400 MHz, DMSO-$d_6$) $\delta$ 8.87 (d, $J$ = 8.2 Hz, 1H), 7.96-7.87 (m, 2H), 7.78 (d, $J$ = 8.2 Hz, 1H), 7.64-7.49 (m, 4H), 7.38-7.26 (m, 2H), 6.76 (s, 2H). |

(continued)

| Example | Structure | Raw material & Reaction reagent | Reference Examples of the preparation method | MS(M+H)$^+$&$^1$H NMR |
|---|---|---|---|---|
| 41 | 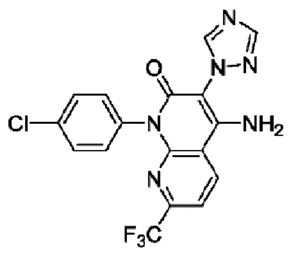 | 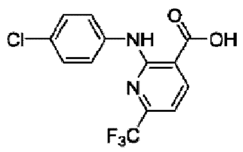 & 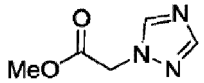 | Example 15 | MS (ESI) m/z (M+H)$^+$= 407.1. $^1$H NMR (400 MHz, DMSO-$d_6$) δ 8.96 (d, *J* = 8.2 Hz, 1H), 8.63 (s, 1H), 8.23 (s, 1H), 7.86 (d, *J* = 8.2 Hz, 1H), 7.57 (d, *J* = 8.6 Hz, 2H), 7.34 (d, *J* = 8.6 Hz, 4H). |
| 42 | 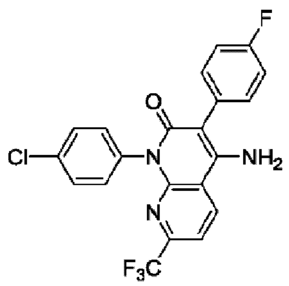 | 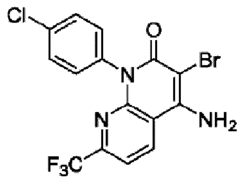 & 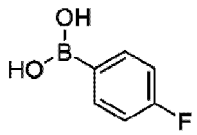 | Example 4 | MS (ESI) m/z (M+H)$^+$= 434.1. $^1$H NMR (400 MHz, DMSO-$d_6$) δ 8.85 (d, *J* = 8.1 Hz, 1H), 7.76 (d, *J* = 8.2 Hz, 1H), 7.54 (d, *J* = 8.6 Hz, 2H), 7.37 (dd, *J* = 8.6, 5.8 Hz, 2H), 7.33-7.21 (m, 4H), 6.54 (s, 2H). |
| 43 | 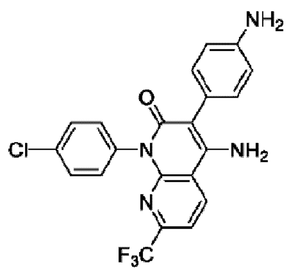 | 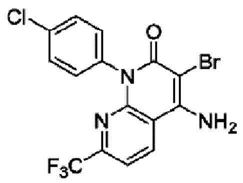 & 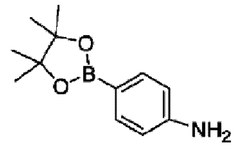 | Example 4 | MS (ESI) m/z (M+H)$^+$= 431.1. $^1$H NMR (400 MHz, DMSO-$d_6$) δ 8.78 (d, *J* = 8.2 Hz, 1H), 7.73 (d, *J* = 8.2 Hz, 1H), 7.54 (d, *J* = 8.6 Hz, 2H), 7.30 (d, *J* = 8.6 Hz, 2H), 7.01 (d, *J* = 8.4 Hz, 2H), 6.64 (d, *J* = 8.4 Hz, 2H), 6.28 (s, 2H), 5.17 (s, 2H). |
| 44 | 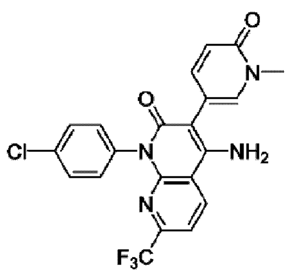 | 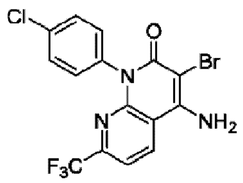 & 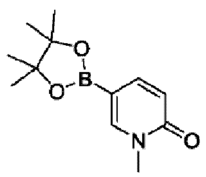 | Example 4 | MS (ESI) m/z (M+H)$^+$= 447.1. $^1$H NMR (400 MHz, DMSO-$d_6$) δ 8.83 (d, *J* = 8.2 Hz, 1H), 7.77 (d, *J* = 8.2 Hz, 1H), 7.64 (d, *J* = 2.3 Hz, 1H), 7.54 (d, *J* = 8.6 Hz, 2H), 7.35-7.25 (m, 3H), 6.86 (s, 2H), 6.44 (d, *J* = 9.3 Hz, 1H), 3.45 (s, 3H). |

(continued)

| Example | Structure | Raw material & Reaction reagent | Reference Examples of the preparation method | MS(M+H)$^+$&$^1$H NMR |
|---|---|---|---|---|
| 45 | 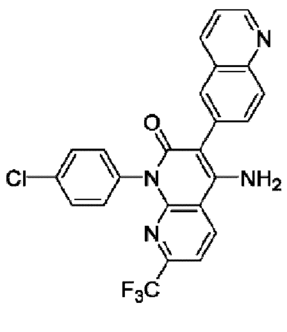 | 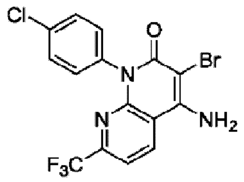 & 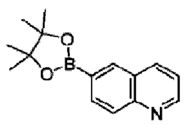 | Example 4 | MS (ESI) m/z (M+H)$^+$= 467.1. $^1$H NMR (400 MHz, DMSO-$d_6$) $\delta$ 8.96-8.85 (m, 2H), 8.39 (d, *J* = 8.1 Hz, 1H), 8.07 (d, *J* = 8.7 Hz, 1H), 7.97 (s, 1H), 7.79 (d. J = 8.2 Hz, 1H), 7.70 (d, *J* = 8.7 Hz, 1H), 7.55 (dd, *J* = 8.3. 4.2 Hz, 3H), 7.34 (d, *J* = 8.5 Hz, 2H), 6.71 (s, 2H). |
| 46 | 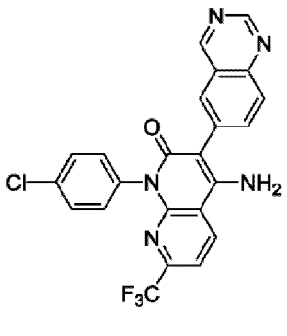 | 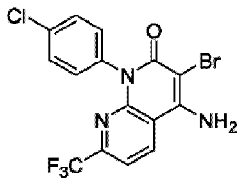 & 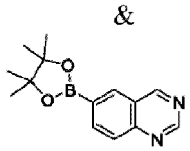 | Example 4 | MS (ESI) m/z (M+H)$^+$ = 468.1. $^1$H NMR (400 MHz, DMSO-$d_6$) $\delta$ 9.63 (s, 1H), 9.31 (s, 1H), 8.90 (d, *J* = 8.2 Hz, 1H), 8.16 (d, *J* = 1.3 Hz, 1H), 8.06 (d, *J* = 8.7 Hz, 1H), 7.98 (dd, *J* = 8.7. 1.8 Hz, 1H), 7.80 (d, *J* = 8.2 Hz, 1H), 7.55 (d, *J* = 8.6 Hz, 2H), 7.35 (d, *J* = 8.6 Hz, 2H), 6.83 (s, 2H). |
| 47 | 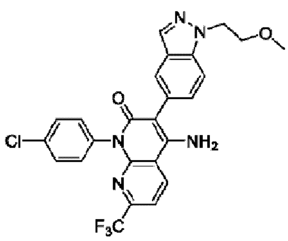 | 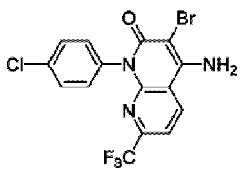 & 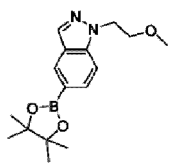 | Preparation Example 5<br>Example 4 | MS (ESI) m/z (M+H)$^+$= 514.1. $^1$H NMR (400 MHz, DMSO) $\delta$ 8.86 (d, *J* = 8.2 Hz, 1H), 8.09 (s, 1H), 7.81 - 7.67 (m, 3H), 7.55 (d, *J* = 8.6 Hz, 2H), 7.31 (dd, *J* = 14.5, 5.0 Hz, 3H), 6.53 (s, 2H), 4.59 (t, *J* = 5.2 Hz, 2H), 3.79 (t, *J* = 5.3 Hz, 2H), 3.23 (s, 3H). |

(continued)

| Example | Structure | Raw material & Reaction reagent | Reference Examples of the preparation method | MS(M+H)+&1H NMR |
|---|---|---|---|---|
| 48 | 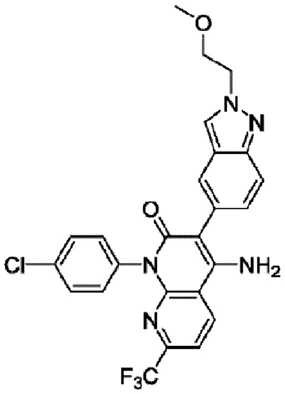 | 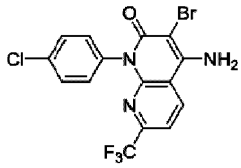<br>&<br>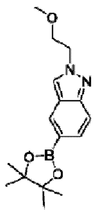 | Preparation Example 5<br><br>Example 4 | MS (ESI) m/z $(M+H)^+$= 514.1. $^1H$ NMR (400 MHz, DMSO) δ 8.85 (d, *J* = 8.1 Hz, 1H), 8.37 (s, 1H), 7.76 (d, *J* = 8.2 Hz, 1H), 7.69 - 7.61 (m, 2H), 7.55 (d, *J* = 8.5 Hz, 2H), 7.33 (d, *J* = 8.5 Hz, 2H), 7.15 (d, *J* = 8.9 Hz, 1H), 6.52 (s, 2H), 4.60 (t, *J* = 5.1 Hz, 2H), 3.84 (t. *J* = 5.1 Hz, 2H), 3.25 (s. 3H). |
| 49 | 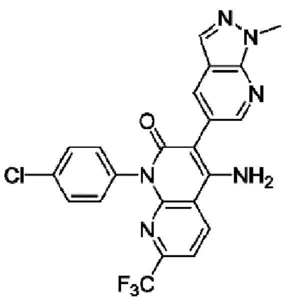 | 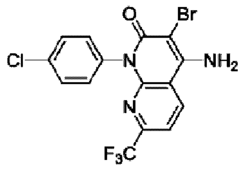<br>&<br>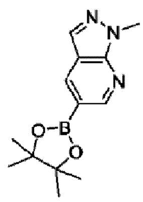 | Preparation Example 5<br><br>Example 4 | MS (ESI) m/z $(M+H)^+$= 471.1. $^1H$ NMR (400 MHz, DMSO) δ 8.87 (d, *J* = 8.2 Hz, 1H), 8.45 (d, *J* = 1.9 Hz, 1H), 8.17 (s, 2H), 7.79 (d, *J* = 8.2 Hz, 1H), 7.55 (d, *J* = 8.7 Hz, 2H), 7.34 (d, *J* = 8.6 Hz, 2H), 6.77 (s, 2H), 4.11 (s, 3H). |
| 50 | 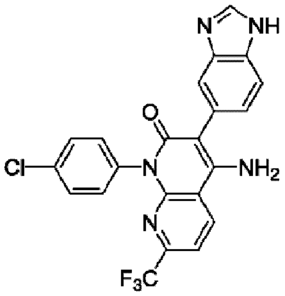 | 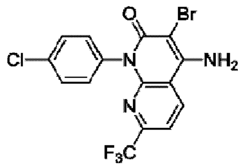<br>&<br>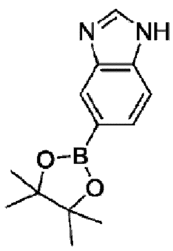 | Exam ple 4 | MS (ESI) m/z $(M+H)^+$= 456.1. $^1H$ NMR (400 MHz, DMSO-$d_6$) δ 12.47 (s, 1H), 8.85 (d, *J* = 8.2 Hz, 1H), 8.24 (s, 1H), 7.76 (d, *J* = 8.3 Hz, 1H), 7.71 (d. *J* = 8.3 Hz, 1H), 7.62 - 7.52 (m, 3H), 7.34 (d, *J* = 8.2 Hz, 2H), 7.18 - 7.12 (m, 1H), 6.44 (s, 2H). |

(continued)

| Example | Structure | Raw material & Reaction reagent | Reference Examples of the preparation method | MS(M+H)+&1H NMR |
|---|---|---|---|---|
| 51 | 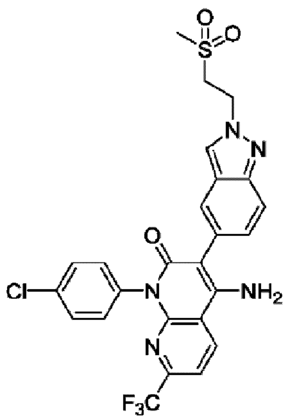 | 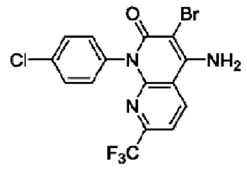 & 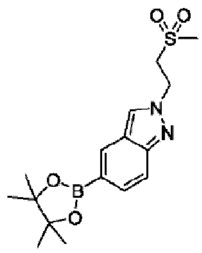 | Preparation Example 5<br>Example 4 | MS (ESI) m/z $(M+H)^+$= 562.1. $^1H$ NMR (400 MHz, DMSO) δ 8.86 (d, *J* = 8.2 Hz, 1H), 8.49 (s, 1H), 7.77 (d, *J* = 8.2 Hz, 1H), 7.72 - 7.62 (m, 2H), 7.55 (d, *J* = 8.6 Hz, 2H), 7.33 (d, *J* = 8.6 Hz, 2H), 7.18 (d, *J* = 9.0 Hz, 1H), 6.53 (s, 2H), 4.90 (t, *J* = 6.9 Hz, 2H), 3.89 (t, *J* = 6.9 Hz, 2H), 2.97 (s, 3H). |
| 52 | 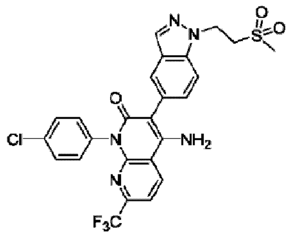 | 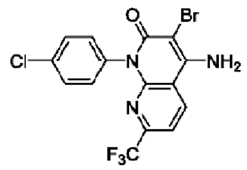 & 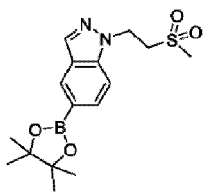 | Preparation Example 5<br>Example 4 | MS (ESI) m/z $(M+H)^+$= 562.1. $^1H$ NMR (400 MHz, DMSO) δ 8.86 (d, *J* = 8.2 Hz, 1H), 8.17 (s, 1H), 7.77 (dd, *J* = 15.7, 7.7 Hz, 3H), 7.55 (d, *J* = 8.6 Hz, 2H), 7.35 (dd, *J* = 14.0, 9.2 Hz, 3H), 6.52 (s, 2H), 4.85 (t, *J* = 6.9 Hz, 2H), 3.78 (t, *J* = 6.9 Hz, 2H), 2.96 (s, 3H). |
| 53 | 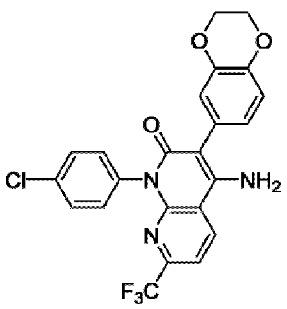 | 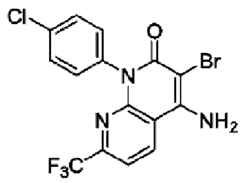 & 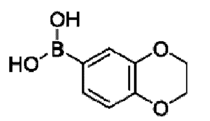 | Example 4 | MS (ESI) m/z $(M+H)^+$= 474.1. $^1H$ NMR (400 MHz, DMSO-$d_6$) δ 8.81 (d, *J* = 8.2 Hz, 1H), 7.74 (d, *J* = 8.2 Hz, 1H), 7.54 (d, *J* = 8.6 Hz, 2H), 7.30 (d, *J* = 8.6 Hz, 2H), 6.92 (d, *J* = 8.1 Hz, 1H), 6.81-6.74 (m, 2H), 6.46 (s, 2H), 4.27 (s, 4H). |

(continued)

| Example | Structure | Raw material & Reaction reagent | Reference Examples of the preparation method | MS(M+H)⁺&¹H NMR |
|---|---|---|---|---|
| 54 | 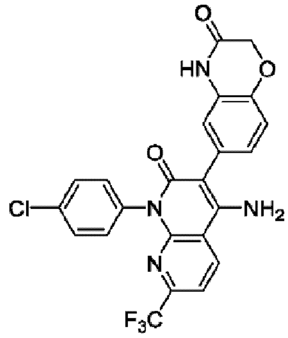 | 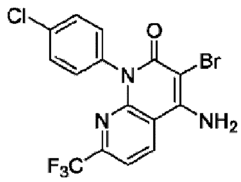 & 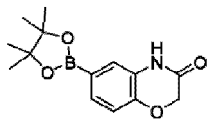 | Exam ple 4 | MS (ESI) m/z $(M+H)^+$= 487.1. $^1H$ NMR (400 MHz, DMSO) $\delta$ 10.76 (s, 1H), 8.82 (d, *J* = 8.1 Hz, 1H), 7.75 (d, *J* = 8.2 Hz, 1H), 7.54 (d, *J* = 8.6 Hz, 2H), 7.31 (d, *J* = 8.6 Hz, 2H), 7.02 (d, *J* = 8.0 Hz, 1H), 6.93-6.83 (m, 2H), 6.52 (s, 2H), 4.60 (s, 2H). |
| 55 | 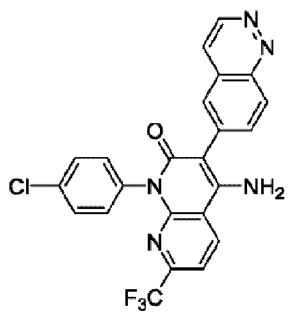 | 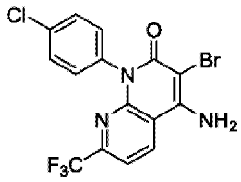 & 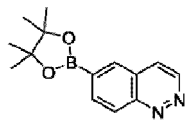 | Example 4 | MS (ESI) m/z $(M+H)^+$= 468.1. $^1H$ NMR (400 MHz, DMSO-$d_6$) $\delta$ 9.38 (d, *J* = 5.8 Hz, 1H), 8.91 (d, *J* = 8.2 Hz, 1H), 8.48 (d, *J* = 8.8 Hz, 1H), 8.24 (d, *J* = 5.8 Hz, 1H), 8.07 (s, 1H), 7.90 (dd, *J* = 8.8, 1.6 Hz, 1H), 7.81 (d, *J* = 8.2 Hz, 1H), 7.56 (d, J = 8.6 Hz, 2H), 7.35 (d, *J* = 8.6 Hz, 2H), 6.90 (s, 2H). |
| 56 | 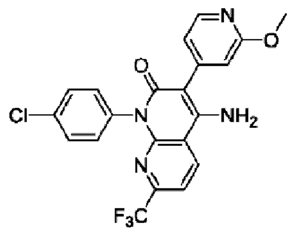 | 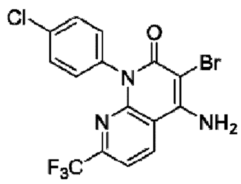 & 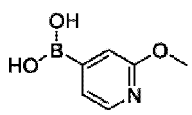 | Example 4 | MS (ESI) m/z $(M+H)^+$= 447.1. $^1H$ NMR (400 MHz, DMSO-$d_6$) $\delta$ 8.87 (d, *J* = 8.2 Hz, 1H), 8.22 (d, *J* = 5.3 Hz, 1H), 7.77 (d, *J* = 8.2 Hz, 1H), 7.54 (d, *J* = 8.6 Hz, 2H), 7.31 (d, *J* = 8.6 Hz, 2H), 6.94 (dd, *J* = 5.2, 1.1 Hz. 1H), 6.79 (s, 2H), 6.77 (s, 1H), 3.88 (s, 3H). |
| 57 | 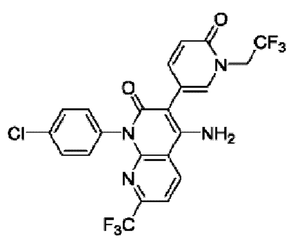 | 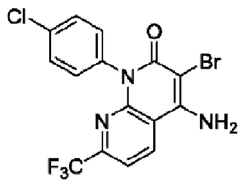 & 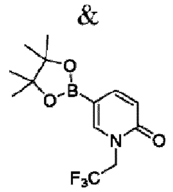 | Preparation Example 5<br>Example 4 | MS (ESI) m/z $(M+H)^+$= 515.1. $^1H$ NMR (400 MHz, DMSO-$d_6$) $\delta$ 8.84 (d, *J* = 8.3 Hz, 1H), 7.79 (d, *J* = 8.2 Hz, 1H), 7.65 (s, 1H), 7.55 (d, *J* = 8.6 Hz, 2H), 7.41 (dd, *J* = 9.5. 2.4 Hz, 1H), 7.31 (d, *J* = 8.6 Hz, 2H), 6.92 (s, 2H), 6.55 (d, *J* = 9.4 Hz, 1H), 4.88 (q, *J* = 8.4 Hz, 2H). |

(continued)

| Example | Structure | Raw material & Reaction reagent | Reference Examples of the preparation method | MS(M+H)$^+$&$^1$H NMR |
|---|---|---|---|---|
| 58 | 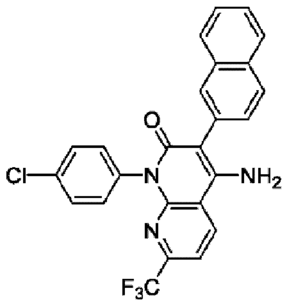 | 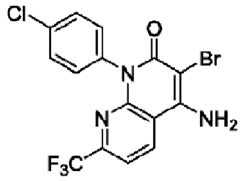 & 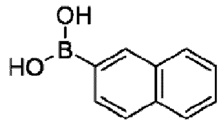 | Example 4 | MS (ESI) m/z (M+H)$^+$ = 466. 1. $^1$H NMR (400 MHz, DMSO-$d_6$) $\delta$ 8.89 (d, $J$ = 8.0 Hz, IH), 7.99-7.91 (m, 4H), 7.79 (d, $J$ = 8.2 Hz, 1H), 7.57-7.53 (m, 4H), 7.47 (d, $J$ = 8.4 Hz, 1H), 7.35 (d, $J$ = 8.6 Hz, 2H), 6.64 (s, 2H). |
| 59 | 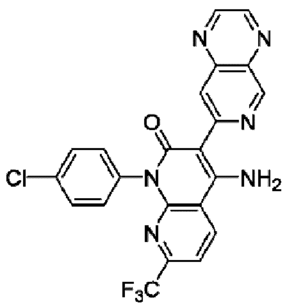 | 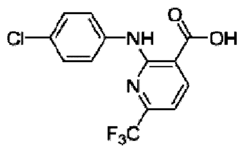 & 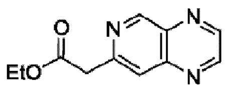 | Preparation Example 6<br>Example 2 | MS (ESI) m/z (M+H)$^+$= 469.1. $^1$H NMR (400 MHz, DMSO-$d_6$) $\delta$ 9.60 (s, 1H). 9.16 (d, $J$ = 1.6 Hz, 1H), 9.06 (d, $J$ = 1.6 Hz, 1H), 8.96 (d, $J$ = 8.2 Hz, IH), 8.67 (s, 1H), 8.36 (s, 2H), 7.83 (d, $J$ = 8.2 Hz, 1H), 7.59 (d, $J$ = 8.6 Hz, 2H), 7.39 (d, $J$ = 8.6 Hz, 2H). |
| 60 | 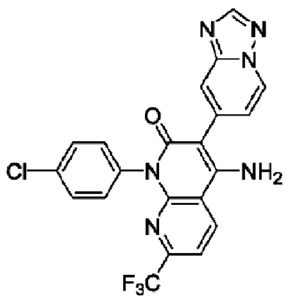 | 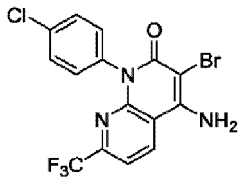 & 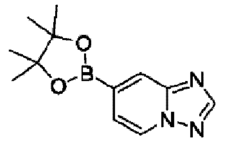 | Example 4 | MS (ESI) m/z (M+H)$^+$= 457.1. $^1$H NMR (400 MHz, DMSO-$d_6$) $\delta$ 8.97 (d, $J$ = 7.0 Hz, 1H), 8.88 (d, $J$ = 8.2 Hz, 1H), 8.51 (s, 1H), 7.81 (d, $J$ = 8.2 Hz, 1H). 7.77 (s, 1H), 7.56 (d, $J$ = 8.6 Hz, 2H), 7.34 (d, $J$ = 8.6 Hz, 2H), 7.11 (dd, $J$ = 7.0, 1.5 Hz, 1H), 6.95 (s, 2H). |
| 61 | 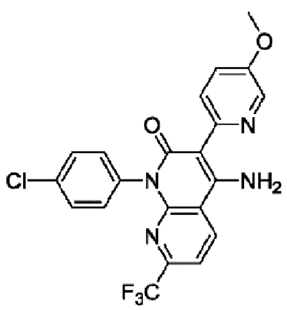 | 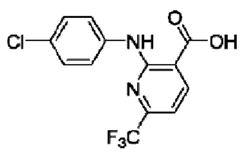 & 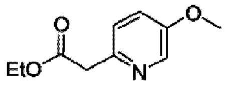 | Example Preparation Example 6<br>Example 15 | MS (ESI) m/z (M+H)$^+$ = 447. 1. $^1$H NMR (400 MHz, DMSO-$d_6$) $\delta$ 8.87 (d, $J$ = 8.2 Hz, 1H), 8.22 (d, $J$ = 5.2 Hz, 1H), 7.77 (d, $J$ = 8.2 Hz. 1H), 7.54 (d, $J$ = 8.6 Hz, 2H), 7.31 (d, $J$ = 8.6 Hz, 2H), 6.94 (d, $J$ = 5.3 Hz, 1H), 6.78 (d, $J$ = 9.8 Hz, 3H), 3.88 (s, 3H). |

(continued)

| Example | Structure | Raw material & Reaction reagent | Reference Examples of the preparation method | MS(M+H)$^+$&$^1$H NMR |
|---|---|---|---|---|
| 63 | 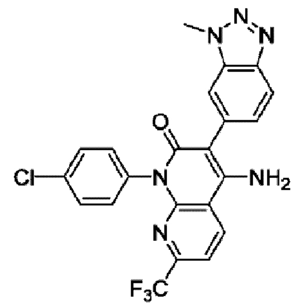 | 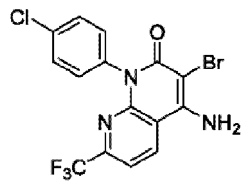 & 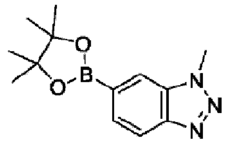 | Example 4 | MS (ESI) m/z (M+H)$^+$= 471.1. $^1$H NMR (400 MHz, DMSO-$d_6$) $\delta$ 8.88 (d, $J$ = 8.2 Hz, 1H), 8.06 (d, $J$ = 8.4 Hz, 1H), 7.86-7.75 (m, 2H), 7.55 (d, $J$ = 8.7 Hz, 2H), 7.34-7.31 (m, 3H), 6.70 (s, 2H), 4.31 (s. 3H). |
| 64 | 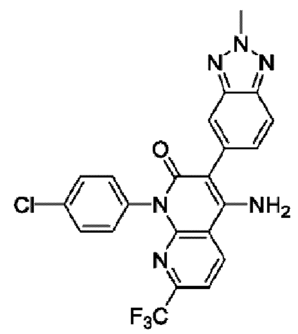 | 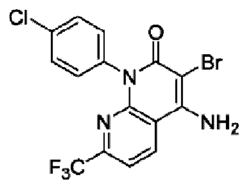 & 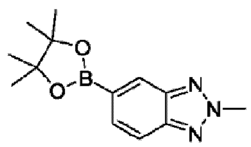 | Example 4 | MS (ESI) m/z (M+H)$^+$= 471.1. $^1$H NMR (400 MHz, DMSO-$d_6$) $\delta$ 8.87 (d, $J$ = 8.1 Hz, 1H), 7.93 (d, $J$ = 8.9 Hz, 1H), 7.83 (s, 1H), 7.78 (d, $J$ = 8.2 Hz, 1H), 7.55 (d, $J$ = 8.6 Hz, 2H), 7.34 (d, $J$ = 8.7 Hz, 3H), 6.66 (s, 2H), 4.51 (s. 3H). |
| 65 | 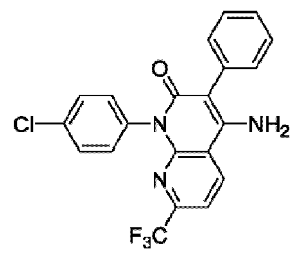 | 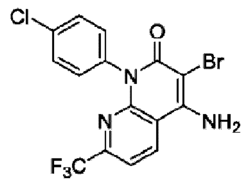 & 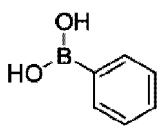 | Example 4 | MS (ESI) m/z (M+H)$^+$= 416.1. $^1$H NMR (400 MHz, DMSO-$d_6$) $\delta$ 8.86 (d, $J$ = 8.1 Hz, 1H), 7.76 (d, $J$ = 8.2 Hz, 1H), 7.55 (d, $J$ = 8.6 Hz, 2H), 7.47 (t, $J$ = 7.6 Hz, 2H), 7.40-7.26 (m, 5H), 6.47 (s, 2H). |

(continued)

| Example | Structure | Raw material & Reaction reagent | Reference Examples of the preparation method | MS(M+H)$^+$&$^1$H NMR |
|---|---|---|---|---|
| 66 | 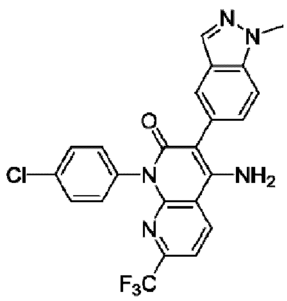 | 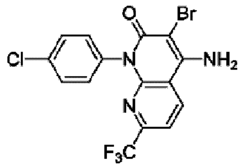 & 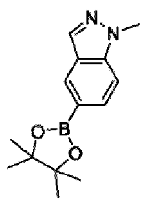 | Example 4 | MS (ESI) m/z (M+H)$^+$= 470.1. $^1$H NMR (400 MHz, DMSO) $\delta$ 8.85 (d, $J$ = 8.2 Hz, 1H), 8.06 (s, 1H), 7.76 (d, $J$ = 8.2 Hz, 1H), 7.70 (d, $J$ = 9.3 Hz, 2H), 7.55 (d, $J$ = 8.6 Hz, 2H), 7.34 (d, $J$ = 8.6 Hz, 3H), 6.49 (s, 2H), 4.08 (s, 3H). |
| 67 | 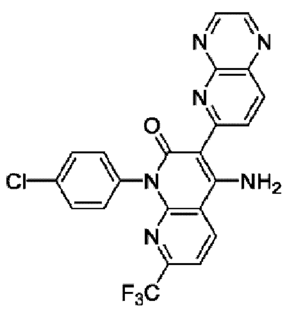 | 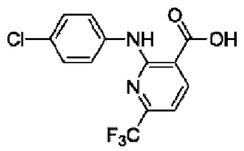 & 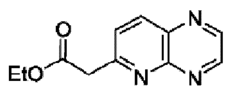 | Preparation Example 6<br>Example 2 | MS (ESI) m/z (M+H)$^+$= 469.1. $^1$H NMR (400 MHz, DMSO-$d_6$) $\delta$ 9.11 (d, $J$ = 1.9 Hz, 1H), 9.04-8.96 (m, 2H), 8.77 (d, $J$ = 9.1 Hz, 1H), 8.47 (d, $J$ = 9.1 Hz, 1H), 7.87 (d, $J$ = 8.2 Hz, 1H), 7.63-7.56 (m, 2H), 7.44-7.37 (m, 2H). |
| 68 | 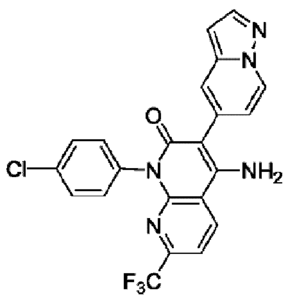 | 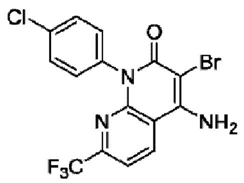 & 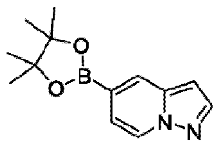 | Exam ple 4 | MS (ESI) m/z (M+H)$^+$ = 456.1. $^1$H NMR (400 MHz, DMSO-$d_6$) $\delta$ 8.87 (d, $J$ = 8.2 Hz, 1H). 8.69 (d, $J$ = 7.2 Hz, 1H), 8.00 (d, $J$ = 2.2 Hz, 1H), 7.78 (d, $J$ = 8.2 Hz, 1H), 7.65 (s, 1H), 7.59-7.51 (m, 2H), 7.38-7.29 (m, 2H), 6.83 (s, 2H), 6.78 (dd, $J$ = 7.2, 1.8 Hz, 1H), 6.62 (d, $J$ = 1.5 Hz, 1H). |
| 69 | 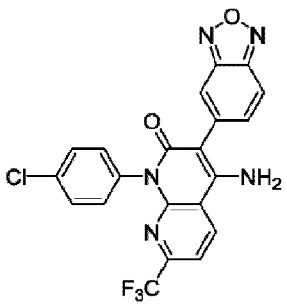 | 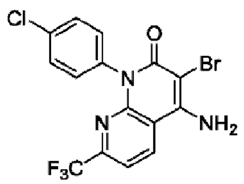 & 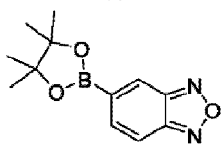 | Example 4 | MS (ESI) m/z (M+H)$^+$ = 458. 1. $^1$H NMR (400 MHz, DMSO-$d_6$) $\delta$ 8.89 (d, $J$ = 8.2 Hz, 1H), 8.04 (dd, $J$ = 9.3, 0.9 Hz, 1H), 7.98 (s, 1H), 7.82 (d, $J$ = 8.2 Hz, 1H), 7.59 7.52 (m, 2H), 7.49 (dd, $J$ = 9.3, 1.2 Hz, 1H), 7.38-7.30 (m, 2H), 7.04 (s, 2H). |

(continued)

| Example | Structure | Raw material & Reaction reagent | Reference Examples of the preparation method | MS(M+H)$^+$&$^1$H NMR |
|---|---|---|---|---|
| 70 | 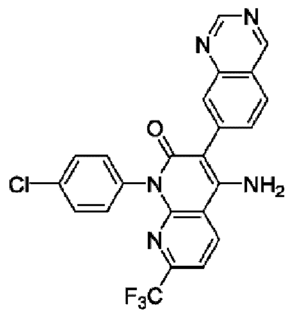 | 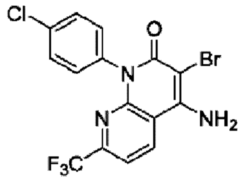 & 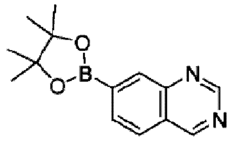 | Example 4 | MS (ES1) m/z (M+H)$^+$= 468.1. $^1$H NMR (400 MHz, DMSO) $\delta$ 9.63 (s, 1H), 9.31 (s, 1H), 8.90 (d, $J$ = 8.1 Hz, 1H), 8.20 (d, $J$ = 8.4 Hz, 1H), 7.98 (s, 1H), 7.80 (d, $J$ = 8.2 Hz, 1H), 7.73 (dd, $J$ = 8.4, 1.5 Hz, 1H), 7.59-7.52 (m, 2H), 7.39-7.31 (m, 2H). 6.86 (s, 2H). |
| 71 | 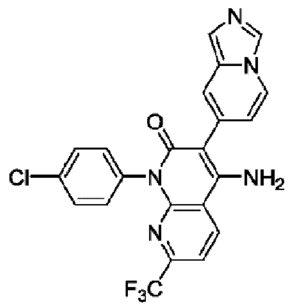 | 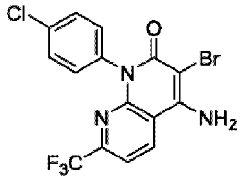 & 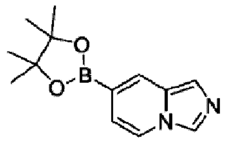 | Preparation Example 5<br>Example 4 | MS (ESI) m/z (M+H)$^+$= 456.1. $^1$H NMR (400 MHz, DMSO-$d_6$) $\delta$ 8.86 (d, $J$ = 8.2 Hz, 1H), 8.39 (s, 1H), 8.36 (d, $J$ = 7.2 Hz, 1H), 7.77 (d, $J$ = 8.2 Hz, 1H), 7.55 (d, $J$ = 8.6 Hz, 2H), 7.51 (s, 1H), 7.37 (s, 1H), 7.32 (d, $J$ = 8.6 Hz, 2H), 6.80 (s, 2H), 6.56 (d, $J$ = 6.4 Hz, 1H). |
| 72 | 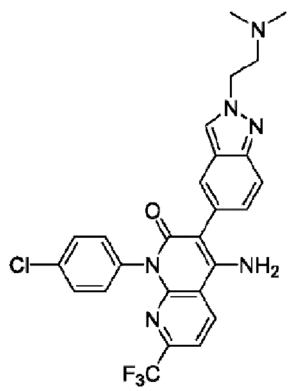 | 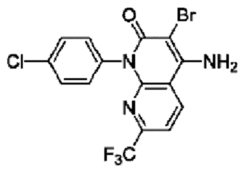 & 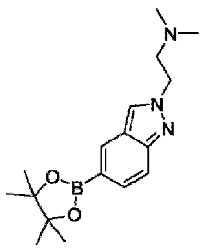 | Preparation Example 5<br>Example 4 | MS (ESI) m/z (M+H)$^+$ = 527.1。 $^1$H NMR (400 MHz, DMSO-$d_6$) $\delta$ 8.86 (d, $J$ = 8.2 Hz, 1H), 8.51 - 8.45 (m, 1H), 7.76 (d, $J$ = 8.2 Hz, 1H), 7.70 (t, $J$ = 1.2 Hz, 1H), 7.66 (dd, $J$ = 8.9, 1.0 Hz, 1H), 7.59 - 7.50 (m, 2H), 7.36 - 7.28 (m, 2H), 7.18 (dd, $J$ = 8.9, 1.6 Hz, 1H), 6.51 (s, 2H), 4.82 (t, $J$ = 6.6 Hz, 2H), 3.48 (s, 2H), 2.65 (s, 6H). |

(continued)

| Example | Structure | Raw material & Reaction reagent | Reference Examples of the preparation method | MS(M+H)$^+$&$^1$H NMR |
|---|---|---|---|---|
| 73 | 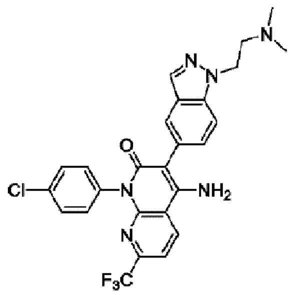 | 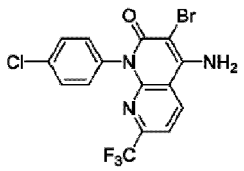 & 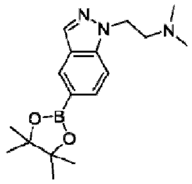 | Preparation Example 5<br>Example 4 | MS (ESI) m/z (M+H)$^+$ = 527.1。 $^1$H NMR (400 MHz, Dh4SO-$d_6$) $\delta$ 8.86 (d, *J* = 8.2 Hz, 1H), 8.18 (s, 1H), 7.83 (d, *J* = 8.8 Hz, 1H), 7.79 - 7.74 (m, 2H), 7.58 - 7.51 (m. 2H), 7.38 (dd, *J* = 8.7, 1.5 Hz, 1H), 7.35 - 7.29 (m, 2H), 6.50 (s, 2H), 4.81 (s, 2H), 3.50 (s, 2H), 2.76 (s, 6H). |
| 74 | 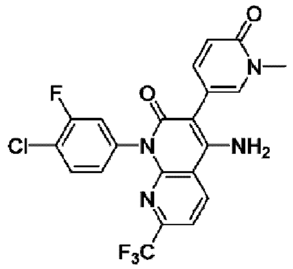 | 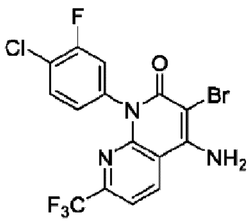 & 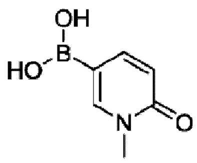 | Example 4 | MS (ESI) m/z (M+H)$^+$= 465.1. $^1$H NMR (400 MHz, DMSO-d$_6$) $\delta$ 8.84 (d, *J* = 8.1 Hz, 1H), 7.80 (d, *J* = 8.2 Hz, 1H), 7.71 (t, *J* = 8.4 Hz, 1H), 7.64 (d, *J* = 2.5 Hz, 1H), 7.48 (dd, *J* = 10.1, 2.2 Hz, 1H), 7.31 (dd, *J* = 9.3, 2.5 Hz, 1H), 7.22-7.15 (m, 1H), 6.90 (s, 2H), 6.44 (d, *J* = 9.3 Hz, 1H), 3.45 (s, 3H). |
| 75 | 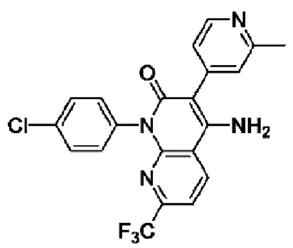 | 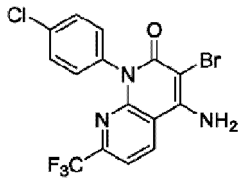 & 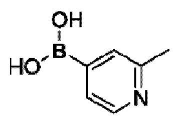 | Example 4 | MS (ESI) m/z (M+H)$^+$ = 431.1. $^1$H NMR (400 MHz, DMSO) $\delta$ 8.87 (d, *J* = 8.2 Hz, 1H), 8.49 (d, *J* = 5.1 Hz, 1H), 7.77 (d, *J* = 8.2 Hz, 1H), 7.59-7.49 (m, 2H), 7.35-7.26 (m, 2H), 7.22 (s, 1H), 7.17-7.12 (m, 1H), 6.75 (s, 2H), 2.51 (s, 3H). |

(continued)

| Example | Structure | Raw material & Reaction reagent | Reference Examples of the preparation method | MS(M+H)+&1H NMR |
|---|---|---|---|---|
| 76 | 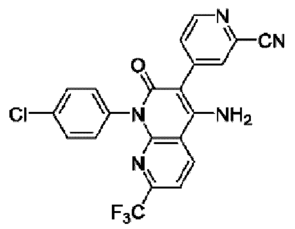 | 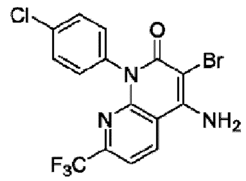 & 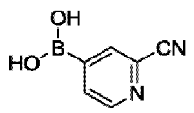 | Exam ple 4 | MS (ESI) m/z $(M+H)^+$ = 442.1. $^1H$ NMR (400 MHz, DMSO) $\delta$ 8.90 (d, $J$ = 8.2 Hz, 1H), 8.79 (dd, $J$ = 5.1, 0.7 Hz, 1H), 8.05-7.98 (m, 1H), 7.82 (d, $J$ = 8.2 Hz, 1H), 7.74 (dd, $J$ = 5.1, 1.6 Hz, 1H), 7.59-7.51 (m, 2H), 7.37-7.27 (m, 2H), 7.09 (s, 2H). |
| 77 | 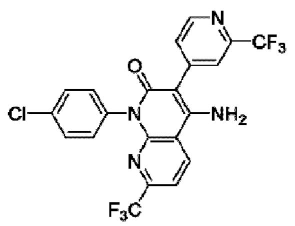 | 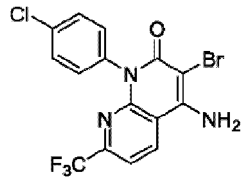 & 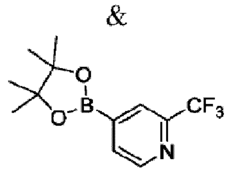 | Example 4 | MS (ESI) m/z $(M+H)^+$ = 485.1. $^1H$ NMR (400 MHz, DMSO-$d_6$) $\delta$ 8.90 (d, $J$ = 8.2 Hz, 1H), 8.82 (d, $J$ = 5.0 Hz, 1H), 7.83 (s, 1H), 7.81 (d, $J$ = 8.2 Hz, 1H), 7.72 (d, $J$ = 5.0 Hz, 1H), 7.58-7.52 (m, 2H), 7.35-7.29 (m, 2H), 7.05 (s, 2H). |
| 78 | 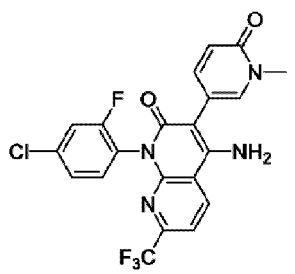 | 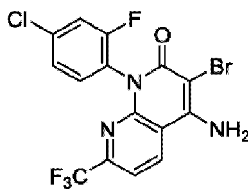 & 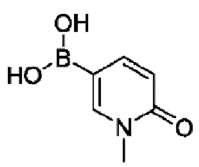 | Example 4 | MS (ESI) m/z $(M+H)^+$= 465.1. $^1H$ NMR (400 MHz, DMSO-$d_6$) $\delta$ 8.88 (d, $J$ = 8.2 Hz, 1H), 7.84 (d, $J$ = 8.2 Hz, 1H), 7.68 (d, $J$ = 2.5 Hz, 1H), 7.66 (dd, $J$ = 9.8, 2.0 Hz, 1H), 7.51-7.40 (m, 2H), 7.32 (dd, $J$ = 9.3, 2.5 Hz, 1H), 6.98 (s, 2H), 6.45 (d, $J$ = 9.3 Hz, 1H), 3.46 (s, 3H). |
| 80 | 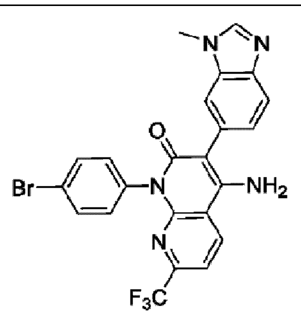 | 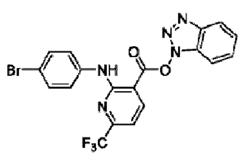 & 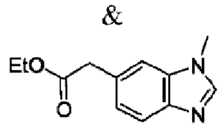 | Preparation Example 6<br>Example 79 | MS (ESI) m/z $(M+H)^+$= 514.0, 516.0. $^1H$ NMR (400 MHz, DMSO-$d_6$) $\delta$ 8.84 (d, $J$ = 8.1 Hz, 1H), 8.20 (s. 1H), 7.76 (d, $J$ = 8.2 Hz, 1H), 7.73-7.64 (m, 3H), 7.51 (d, $J$ = 0.9 Hz, 1H), 7.30-7.22 (m, 2H), 7.15 (dd, $J$ = 8.3, 1.5 Hz, 1H), 6.48 (s, 2H), 3.84 (s, 3H). |

(continued)

| Example | Structure | Raw material & Reaction reagent | Reference Examples of the preparation method | MS(M+H)$^+$&$^1$H NMR |
|---|---|---|---|---|
| 81 | 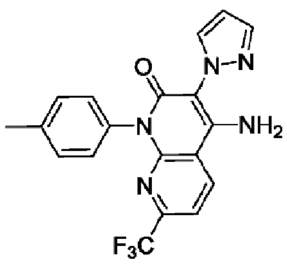 | 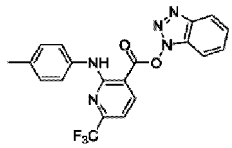 & 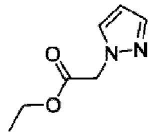 | Preparation Example 12<br>Example 15 | MS (ESI) m/z $(M+H)^+$ = 386.2。 $^1$H NMR (400 MHz, DMSO-$d_6$) δ 8.92 (d, *J* = 8.2 Hz, 1H), 7.91 (d, *J* = 2.3 Hz, 1H), 7.81 (d, *J* = 8.2 Hz, 1H), 7.76 (d, *J* = 1.5 Hz, 1H), 7.29 (d, *J* = 8.1 Hz, 2H), 7.19 - 7.04 (m, 4H), 6.47 (t, *J* = 2.0 Hz, 1H), 2.39 (s, 3H). |
| 82 | 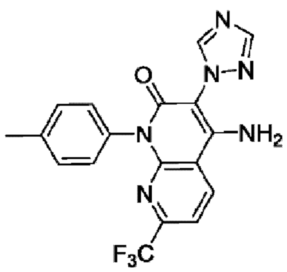 | 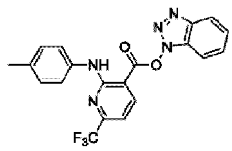 & 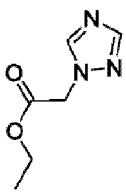 | Example 15 | MS (ES1) m/z $(M+H)^+$ = 387.1。 $^1$H NMR (400 MHz, DMSO-$d_6$) δ 8.94 (d, *J* = 8.1 Hz, 1H), 8.64 (s. 1H), 8.23 (s, 1H), 7.83 (d, *J* = 8.2 Hz, 1H), 7.29 (d, *J* = 8.0 Hz, 4H), 7.14 (d, *J* = 8.0 Hz, 2H), 2.38 (s, 3H). |
| 83 | 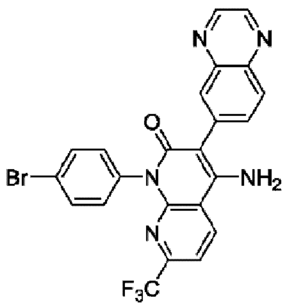 | 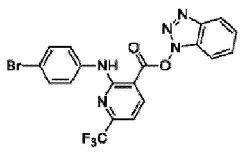 & 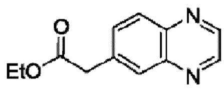 | Preparation Example 6 & Example 79 | MS (ESI) m/z $(M+H)^+$= 512.0, 514.0. $^1$H NMR (400 MHz, DMSO-$d_6$) δ 8.97 (s, 2H), 8.90 (d, *J* = 8.1 Hz, 1H), 8.15 (d, *J* = 8.7 Hz, 1H), 8.06 (d, *J* = 1.6 Hz, 1H), 7.86-7.76 (m, 2H), 7.72-7.64 (m, 2H), 7.34-.24 (m, 2H), 6.83 (s, 2H). |
| 84 | 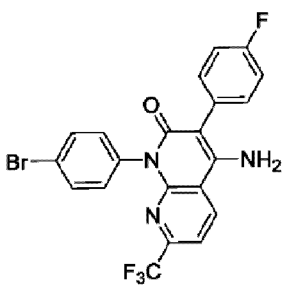 | 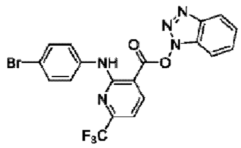 & 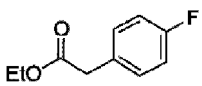 | Example 79 | MS (ESI) m/z $(M+H)^+$ = 478.0, 480.0. $^1$H NMR (400 MHz, DMSO-$d_6$) δ 8.84 (d, *J* = 8.1 Hz, 1H), 7.76 (d, *J* = 8.1 Hz, 1H), 7.67 (d, *J* = 8.5 Hz, 2H), 7.36 (dd, *J* = 8.5, 5.8 Hz, 2H), 7.31-7.20 (m, 4H), 6.45 (s, 2H). |

(continued)

| Example | Structure | Raw material & Reaction reagent | Reference Examples of the preparation method | MS(M+H)$^+$&$^1$H NMR |
|---|---|---|---|---|
| 85 | 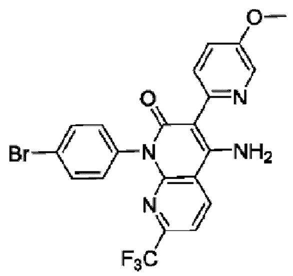 | 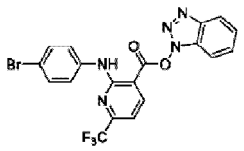 & 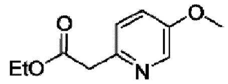 | Preparation Example 6 & Example 79 | MS (ESI) m/z (M+H)$^+$= 491.0, 493.0. $^1$H NMR (400 MHz, DMSO-$d_6$) $\delta$ 8.88 (d, $J$ = 8.2 Hz, 1H), 8.37 (d, $J$ = 3.0 Hz, 1H), 8.11 (d, $J$ = 9.1 Hz, 1H), 7.79 (d, $J$ = 8.2 Hz, 1H), 7.69 (d, $J$ = 8.5 Hz, 2H), 7.46 (dd, $J$ = 9.1, 3.1 Hz, 1H), 7.28 (d, $J$ = 8.5 Hz, 2H), 7.10 (s, 2H), 3.88 (s, 3H). |
| 86 | 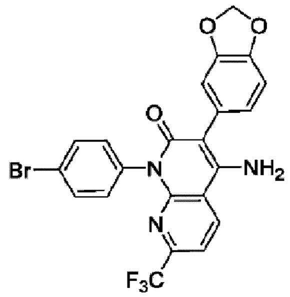 | 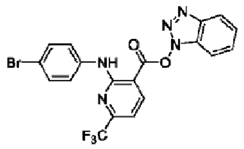 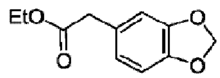 | Preparation Example 6 & Example 79 | MS (ESI) m/z (M+H)$^+$ = 504.0, 506.0. $^1$H NMR (400 MHz, DMSO-$d_6$) $\delta$ 8.82 (d, $J$ = 8.2 Hz, 1H), 7.74 (d, $J$ = 8.2 Hz, 1H), 7.67 (d, $J$ = 8.5 Hz, 2H). 7.24 (d, $J$ = 8.5 Hz, 2H), 6.99 (d, $J$ = 7.9 Hz, 1H), 6.84 (s, 1H), 6.80 (d, $J$ = 8.0 Hz, 1H), 6.48 (s, 2H), 6.05 (s, 2H). |
| 87 | 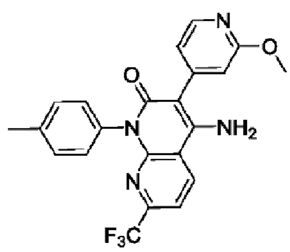 | 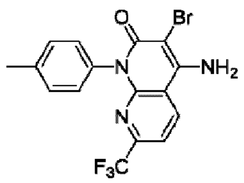 & 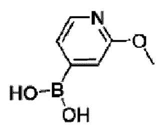 | Example 4 | MS (ESI) m/z (M+H)$^+$ = 427.2. $^1$H NMR (400 MHz, DMSO-$d_6$) δ 8.85 (d, $J$ = 8.2 Hz, 1H), 8.21 (d, $J$ = 5.2 Hz, 1H), 7.73 (d, $J$ = 8.2 Hz, 1H), 7.27 (d, $J$ = 8.1 Hz, 2H), 7.11 (d, $J$ = 8.1 Hz, 2H), 6.94 (d, $J$ = 5.2 Hz, 1H), 6.77 (s, 1H), 6.71 (s, 2H), 3.88 (s, 3H), 2.38 (s, 3H). |
| 88 | 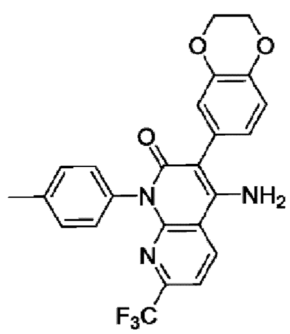 | 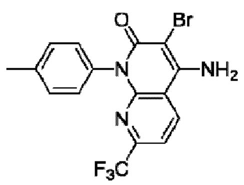 & 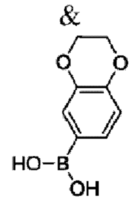 | Example 4 | MS (ESI) m/z (M+H)$^+$ = 454.2。 $^1$H NMR (400 MHz, DMSO-$d_6$) δ 8 79 (d, $J$ = 8.2 Hz, 1H), 7.70 (d, $J$ = 8.2 Hz, 1H), 7.26 (d, $J$ = 8.1 Hz, 2H), 7.10 (d, $J$ = 8.1 Hz, 2H), 6.91 (d, $J$ = 8.0 Hz, 1H), 6.78 (d, $J$ = 9.3 Hz, 2H), 6.38 (s, 2H), 4.27 (s, 4H), 2.38 (s, 3H). |

(continued)

| Example | Structure | Raw material & Reaction reagent | Reference Examples of the preparation method | MS(M+H)$^+$&$^1$H NMR |
|---|---|---|---|---|
| 89 | 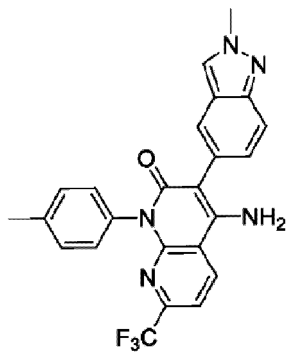 | 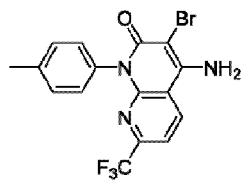 & 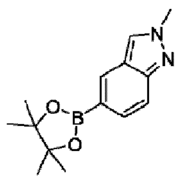 | Preparation Example 5 Example 4 | MS (ESI) m/z (M+H)$^+$ = 450.2。 $^1$H NMR (400 MHz, DMSO-$d_6$) δ 8.82 (d, *J* = 8.2 Hz, 1H), 8.33 (s, 1H), 7.72 (d, *J* = 8.1 Hz, 1H), 7.67 - 7.59 (m, 2H), 7.27 (d, 7 = 8.0 Hz, 2H), 7.13 (td, *J* = 6.4, 3.0 Hz, 3H), 6.40 (s, 2H), 4.18 (s. 3H), 2.38 (s, 3H). |
| 90 | 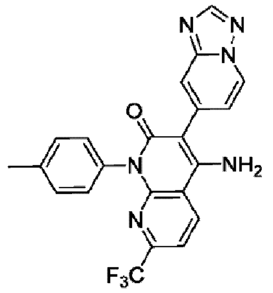 | 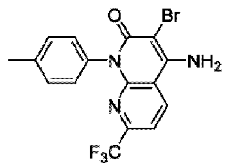 & 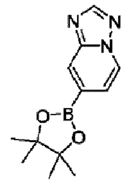 | Example 4 | MS (ESI) m/z (M+H)$^+$ = 437.2。 $^1$H NMR (400 MHz, DMSO-$d_6$) δ 8.96 (d, *J* = 7.0 Hz, 1H), 8.86 (d, *J* = 8.2 Hz, 1H), 8.50 (s, 1H), 7.82 - 7.74 (m. 2H), 7.28 (d, *J* = 8.2 Hz, 2H), 7.19 - 7.07 (m, 3H), 6.88 (s, 2H), 2.38 (s, 3H). |
| 91 | 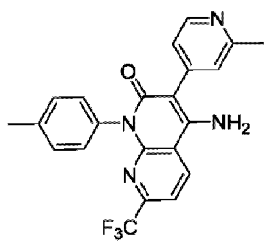 | 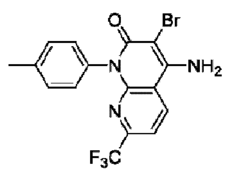 & 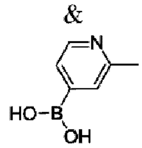 | Example 4 | MS (ESI) m/z (M+H)$^+$ = 411.2。 $^1$H NMR (400 MHz, DMSO-$d_6$) δ 8.85 (d, *J* = 8.2 Hz, 1H), 8.49 (d, *J* = 5.1 Hz, 1H), 7.74 (d, *J* = 8.2 Hz. 1H), 7.27 (d, *J* = 8.2 Hz, 2H), 7.22 (s, 1H), 7.15 (d, *J* = 4.8 Hz. 1H), 7.11 (d, *J* = 8.2 Hz, 2H), 6.68 (s, 2H), 2.50 (s, 3H). 2.38 (s, 3H). |
| 92 | 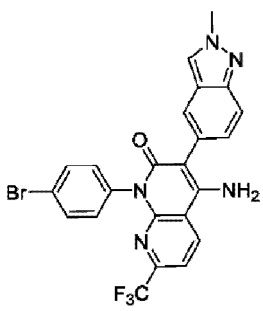 | 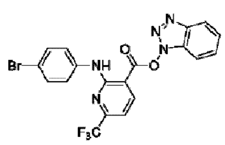 & 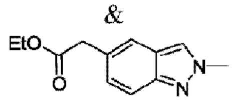 | Preparation Example 6 & Example 79 | MS (ESI) m/z (M+H)$^+$= 514.0, 516.0. $^1$H NMR (400 MHz, DMSO-$d_6$) δ 8.84 (d, *J* = 8.2 Hz, 1H), 8.34 (s, 1H), 7.75 (d, *J* = 8.2 Hz, 1H), 7.70-7.59 (m, 4H), 7.26 (d, *J* = 8.5 Hz, 2H), 7.14 (dd, *J* = 8.9, 1.1 Hz, 1H), 6.48 (s, 2H), 4.18 (s. 3H). |

(continued)

| Example | Structure | Raw material & Reaction reagent | Reference Examples of the preparation method | MS(M+H)$^+$&$^1$H NMR |
|---|---|---|---|---|
| 93 | 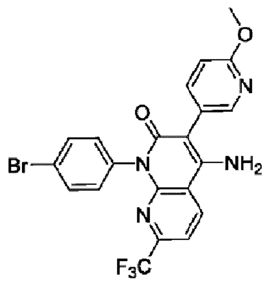 | 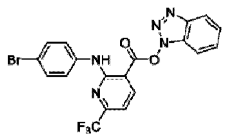 & 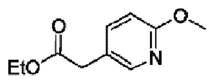 | Preparation Example 6 & Example 79 | MS (ESI) m/z (M+H)$^+$ = 491.0, 493.0. $^1$H NMR (400 MHz, DMSO-$d_6$) $\delta$ 8.84 (d, $J$ = 8.2 Hz, 1H), 8.10 (d, $J$ = 2.1 Hz, 1H), 7.76 (d, $J$ = 8.2 Hz, 1H), 7.67 (d, $J$ = 8.6 Hz, 2H), 7.63 (dd, $J$ = 8.5, 2.3 Hz, 1H), 7.25 (d, $J$ = 8.6 Hz, 2H), 6.89 (d, $J$ = 8.5 Hz, 1H), 6.70 (s, 3.89 (s, 3H). |

**Biological test**

The biological tests demonstrated that the compound of the present invention had excellent MAT2a inhibitory activity, was significantly superior to the existing compounds against the same target in the MTAP-deficient cell anti-proliferation test, and had the advantages of low potential liver toxicity and high solubility.

**Experiment Example 1:** Enzymatic Activity Test

The Colorimetric assay method was used to detect the $IC_{50}$ value of the tested compounds on MAT2a.

The specific steps were as follows: The compounds having the test starting concentration of 1 μM or 10 μM were diluted into 10 concentration points in a 3-fold gradient. 250 nL of the solutions of the compounds to be tested with 10 different concentrations were taken and added into a 384 well plate for later use. 20 μg/mL of MAT2a enzyme solution was prepared with Assay buffer (50 mM Tris, 50 mM KCl, 10 mM $MgCl_2$,0.05% polyoxyethylene lauryl ether, pH 8.0). 15 μL of the MAT2a enzyme solution at 20 μg/mL was add into the wells of the compounds to be tested at different concentrations; and 15 μL of Assay buffer was added into the negative control well. An incubation was carried out for 15 minutes after shaking for mixing well. A mixed substrate solution (comprising 400 μM ATP and 600 μM L-Methionine) was prepared with Assay buffer. 10μL of the mixed substrate solution was added to the positive control well, the compound to be tested well, and the negative control well, respectively, and the reaction began, for a reaction time of 150 min. Then, 50 μL of the reaction stop solution (BIOMOL Green™ Reagent Enzo lifesciences, Cargo No. BML-AK111-1000) was added to stop the reaction, followed by being centrifuged at 1000 rpm for 60 s and then being incubated for 15 min. OD620 was read and data were processed.

Calculation formula:

$$\text{Inhibition\%} = (OD620_{\text{positive control well}} - OD620_{\text{compound well to be tested}})/(OD620_{\text{positive control well}} - OD620_{\text{negative control well}}) \times 100$$

By using the log value of the concentration as the X-axis and the inhibition% as the Y-axis, the $IC_{50}$ value of each compound against enzyme activity was obtained through employing the log(inhibitor) vs. response-Variable slope fitting dose-effect curve of the analysis software GraphPad Prism 5. The experimental results were shown in the table below:

Table 1: $IC_{50}$ values of the compounds of the present invention against MAT2a

| Example | $IC_{50}$ (nM) | Example | $IC_{50}$ (nM) | Example | $IC_{50}$ (nM) |
|---|---|---|---|---|---|
| 1 | NA | 2 | 26.1 | 3 | 1 2. 7 |
| 4 | 20.2 | 5 | NA | 6 | 40.5 |

(continued)

| Example | $IC_{50}$ (nM) | Example | $IC_{50}$ (nM) | Example | $IC_{50}$ (nM) |
|---|---|---|---|---|---|
| 7 | 37.9 | 8 | 8.7 | 9 | 21.4 |
| 10 | 20.9 | 11 | 21.4 | 12 | 25.8 |
| 13 | 32 | 14 | 22.9 | 15 | 23.2 |
| 16 | 41.9 | 17 | NA | 18 | 51.4 |
| 19 | 12.3 | 20 | 11.5 | 21 | 16.2 |
| 22 | 15 | 23 | 16.5 | 24 | 15.6 |
| 25 | 13.3 | 26 | 17.8 | 27 | 17.5 |
| 28 | 25.3 | 29 | 25.2 | 30 | 13.4 |
| 31 | 15.4 | 32 | 14.9 | 33 | 20.2 |
| 34 | NA | 35 | 14.9 | 36 | 13.6 |
| 37 | 20.2 | 38 | 18.1 | 39 | 19.8 |
| 40 | 26.3 | 41 | 18.4 | 42 | 15.0 |
| 43 | 22 | 44 | 13.4 | 45 | 14.8 |
| 46 | 15.1 | 47 | 13.6 | 48 | 16.1 |
| 49 | 15.8 | 50 | 17.2 | 51 | 16.2 |
| 52 | 16.2 | 53 | 14.4 | 54 | 16.2 |
| 55 | 16.3 | 56 | 14.5 | 57 | 17.1 |
| 58 | 17.4 | 59 | 25.4 | 60 | 17.0 |
| 61 | 17.0 | 62 | 16.1 | 63 | 17.8 |
| 64 | 15.4 | 65 | 15.2 | 66 | 16 |
| 67 | 18.1 | 68 | 16.0 | 69 | 16.6 |
| 70 | 30.5 | 71 | 18.5 | 72 | 16.5 |
| 73 | 18.8 | 74 | 16.4 | 75 | 13.8 |
| 76 | 16.3 | 77 | 13.9 | 78 | 14.0 |
| 79 | 18.0 | 80 | 15.5 | 81 | 17.0 |
| 82 | 16.0 | 83 | 17.2 | 84 | 28.2 |
| 85 | 15.2 | 86 | 15.9 | 87 | 15.4 |
| 88 | 16.0 | 89 | 16.5 | 90 | 16.9 |
| 91 | 18.0 | 92 | 17.4 | 93 | 16.3 |
| NA represents not applicable | | | | | |

Conclusion: The above test demonstrated that the compound of the present invention had excellent MAT2a enzyme inhibitory activity.

**Experimental Example 2: Activity test of HCT116 MTAP gene homozygous deletion cells (source: Horizon Corporation)**

On day 1, cell seeding: after digestion of the cells with trypsin, the cells were resuspended to the desired density with the complete medium (RPMI-1640 supplemented with 10% FBS; the brand of FBS is EXCELL, Cat. No. FND500; the brand of RPMI-1640 is ATCC, Cat. No. 30-2001), mixed evenly, and added into the 96-well plate at 100 μL/well with a cell density of 1000-3000 cells per well. The plate was put back to the incubator for the adherent growth of cells. On

day 2, the addition of the compounds to be tested: before the addition of the compounds, the cells were starved with serum-free medium for 4 h, then the complete medium containing the corresponding concentration of compounds was added thereto, and the cells were incubated at 37 °C, 5% $CO_2$ for 120 h. On day 7, the cell culture plates were taken out from incubator and equilibrated to room temperature. 50 μL of CellTiter-Glo (Promega Company, Cat. No. G7571) reagent was added per well, and the cells were fully lysed by shaking at room temperature for 2 min, and then incubated for another 60 min to detect the fluorescence intensity. Calculation formula:

% Inhibition = 100 − (signal of the well with the compound to be tested − signal of the well with only medium and free of cells) / (signal of the well with cells but without addition of the compound − signal of the well with only medium and free of cells) × 100.

The fitting dose-effect curve of the analysis software GraphPad Prism 5 was used to give $IC_{50}$ values of the individual compounds against cellular activity. The experimental results showed that the compounds of the present invention had prominent inhibitory activity against cancer cell, and the compounds of the present invention generally had $IC_{50}$ values below 1000 nM.

Table 2: Inhibitory activity of HCT116 MTAP gene homozygous deletion (HCT116 MTAP-/-) cells

| Example | HCT116 MTAP-/- Cell IC50(nM) | Example | HCT116 MTAP-/- Cell IC50(nM) |
|---|---|---|---|
| 3 | 12.71 | 42 | 8.38 |
| 6 | 493. 5 | 44 | 18.65 |
| 7 | 799. 6 | 45 | 6.37 |
| 13 | 637.9 | 46 | 20.25 |
| 15 | 344.9 | 47 | 15.18 |
| 19 | 13.12 | 48 | 13.33 |
| 20 | 24.48 | 53 | 21.18 |
| 21 | 22.92 | 55 | 23.29 |
| | | | |
| 22 | 20.85 | 56 | 17.84 |
| 23 | 23.04 | 63 | 21.51 |
| 24 | 11.48 | 64 | 10.72 |
| 25 | 13.01 | 67 | 16.3 |
| 26 | 617.3 | 68 | 13.66 |
| 27 | 231.1 | 79 | 10.92 |
| 29 | 426.8 | 80 | 13.09 |
| 30 | 22.31 | 83 | 9.12 |
| 31 | 13.38 | 87 | 9.77 |
| 36 | 7.43 | | |

It had been demonstrated by tests that the compounds of the present invention had excellent inhibitory effects on MAT2a enzyme activity and excellent inhibitory effects on cancer cell growth, especially on cancer cells with MTAP gene deletion, and would have excellent therapeutic effects in MAT2a-related cancers or tumor diseases.

**Experimental Example 3: Inhibitory activity test of KP-4 cells (Source: Nanjing Cobioer Bioscience Co., Ltd.)**

On day 1, cell seeding: after digestion of the cells with trypsin, the cells were resuspended to $1_*10^4$/mL with

RPMI1640 complete medium (HyClone Company, Cat. No. SH30809.01) supplemented with 10% FBS (Gibco, 10099141C), mixed evenly, and added into the 96-well plate at 100 μL/well. The plate was put back to the incubator for the adherent growth of cells. On day 2, the addition of the compounds to be tested: 100 μL of the complete medium containing the compounds (the concentration of the compound in the medium was formulated as follows: 3-fold gradient dilution from 60 μM, 10 concentration gradients in total) was added, incubated for 144 hours at 37°C, 5% $CO_2$. On day 8, the cell culture plates were taken out from incubator and equilibrated to room temperature, excess medium was pipetted from the wells so that 50 μL of supernatant was retained, and 50 μL of CellCounting-Lite 2.0 (Nanjing Vazyme Biotech Co., Ltd., Cat. No. DD1101) reagent was added per well, and the cells were fully lysed by shaking at room temperature for 10 min, and incubated for 5 min to detect the fluorescence intensity.

% Inhibition = 100 − (signal of the well with the compound to be tested − signal of the well with only medium and free of cells) / (signal of the well with cells but without addition of the compound − signal of the well with only medium and free of cells) × 100.

The log value of concentration was used as the X-axis and % Inhibition was used as the Y-axis, so as to fit the dose-effect curve and calculate the IC50 value using nonlinear regression (dose response - variable slope) in the analysis software GraphPad Prism 8. The results were shown in Table 3 below.

Table 3: KP-4 cell inhibitory activity

| Example | $IC_{50}$/μM | | Example | $IC_{50}$/μM |
|---|---|---|---|---|
| 3 | 0.17 | | 41 | 1.08 |
| 15 | 9.71 | | 42 | 0.48 |
| 19 | 0.89 | | 43 | 0.48 |
| 20 | 0.65 | | 44 | 0.49 |
| 21 | 0.27 | | 45 | 0.19 |
| 22 | 0.73 | | 46 | 0.18 |
| 23 | 0.61 | | 47 | 0.55 |
| 24 | 0.10 | | 48 | 0.17 |
| 25 | 1.22 | | 49 | 0.97 |
| 26 | 10.17 | | 52 | 0.76 |
| 27 | 1.62 | | 53 | 0.24 |
| 30 | 0.55 | | 54 | 0.96 |
| 36 | 0.36 | | 55 | 0.5 |
| 38 | 0.91 | | 56 | 1.11 |
| 39 | 0.41 | | 67 | 0.75 |
| | | | 68 | 0.41 |

The inhibitory activity test of KP-4 cell showed that the compounds of the present invention, preferably the compounds in the Examples had strong inhibitory activity against KP-4 cells, typically had an inhibitory activity <20 μM, e.g., an inhibitory activity of 0.001-10 μM, especially 0.01-10 μM, which had significant advantages over existing compounds ($IC_{50}$ thereof were generally higher than 30 μM).

**Experimental Example 4: Inhibitory activity test of DOHH-2 cells (Source: Creative Bioarray Company)**

On day 1, cell seeding: cells were resuspended to the desired density with DMEM complete medium (Gibico, Cat. No. 10569010) supplemented with 10% FBS (Gibco, 10099141C), mixed evenly, and added into the 384-well plate at 30 μL/well with a cell density of 800/well. The compounds to be tested were added as follows: 30 nL of DMSO solution containing the compound (the concentration of the compound in which was formulated as: 3-fold gradient dilution from

10 mM, 10 concentration points in total) was added, incubated for 120 h at 37 °C, 5% $CO_2$. On day 6, the cells treated with compounds were removed and equilibrated to room temperature, 30 μL of CellTiter-Glo (Promega company, Cat. No. G7573) reagent was added per well, and the cells were fully lysed by shaking at room temperature, and then incubated in dark at 37°C, 5% $CO_2$ for 30 min, to detect the fluorescence intensity.

% Inhibition = 100 – (signal of the well with the compound to be tested – signal of the well with only medium and free of cells) / (signal of the well with cells but without the addition of the compound – signal of the well with only medium and free of cells) × 100.

The log value of concentration was used as the X-axis and % Inhibition was used as the Y-axis, so as to fit the dose-effect curve and calculate the IC50 value using nonlinear regression (dose response - variable slope) in the analysis software GraphPad Prism 8. The test results were shown in Table 4 below.

Table 4: DOHH-2 cell inhibitory activity

| Example | $IC_{50}$/nM |
|---|---|
| 20 | 48 |
| 21 | 507 |
| 25 | 507 |
| 31 | 430 |
| 36 | 7 |
| 44 | 495 |
| 46 | 251 |
| 47 | 559 |
| 48 | 518 |
| 68 | 200 |
| 69 | 663 |

The inhibitory activity test of DOHH-2 cell showed that the compounds of the present invention, preferably the compounds in the Examples had strong inhibitory activity against DOHH-2 cells, typically had an inhibitory activity <1 μM, such as 0.1-100 nM, preferably 0.1-50 nM, which was obviously superior to that of the existing compounds, and had a great prospect for development.

**Experimental Example 5: *In vivo* pharmacokinetic study in SD rats**

1 .Test animal

Species: SD rat. Source: Charles River Laboratory Animal Technology Co., Ltd. Number: 3 rats per dosage group.

Preparation of test sample:

1.1 An appropriate amount of the test sample was accurately weighed, into which 5% DMSO, 10% polyethylene glycol-15 hydroxystearate, 85% normal saline (all in volume percent) were added sequentially, and were fully mixed via vortex or ultrasound, to obtain the dosing solution with a test sample concentration of 0.2 mg/mL for intravenous administration.

1.2 An appropriate amount of the test sample was accurately weighed, into which 5% DMSO, 10% polyethylene glycol-15 hydroxystearate, 85% normal saline (all in volume percent) were added sequentially, and were fully mixed via vortex or ultrasound, to obtain the dosing solution with a test sample concentration of 0.5mg/mL for oral gavage administration.

2. Experimental design

| Group | Test sample | Number male | Dosage | Concentration | Administration volume | Administration mode | Collected sample |
|---|---|---|---|---|---|---|---|
| | | | (mg/kg) | (mg/mL) | (mL/kg) | | |
| 1 | Example 36 | 3 | 1 | 0.2 | 5 | IV | Plasma |
| 2 | | 3 | 5 | 0.5 | 10 | PO | Plasma |

3. Administration mode

Weighing was performed before administration, and the administration amount was calculated according to the body weight. The drug was administered orally by gavage or intravenously.

4. Time points of blood collection

Before and 0.083h, 0.25h, 0.5h, 0.75h, 1h, 2h, 4h, 8h, 24h after administration.

5. Sample collection and disposal

On the day of the test, 100 μL of blood was collected via the jugular sinus at each set time point, and the whole blood samples were placed in anticoagulation tubes containing EDTA-$K_2$. Whole blood samples were centrifuged at 1500g for 10 min to separate the plasma, and the upper plasma samples were collected into sample tubes. Biological samples were stored at -40°C to -20°C for analysis.

6. Bioanalysis and data processing

According to the requirements of SOP-BA-002 (Liquid Mass Spectrometry for biological sample analysis) of Suzhou 3D BioOptima New Drug Development Co., Ltd., an LC-MS/MS analytical method was established to determine the concentrations of compounds in rat plasma, and used to determine the concentrations of compounds in the biological samples obtained in this experiment.

Pharmacokinetic parameters were calculated using the non-compartment model of Pharsight Phoenix 8.0.

**Table 5:** Data of *in vivo* pharmacokinetic studies of test compounds administered intravenously and orally to SD rats

| Compound | | Example 36 |
|---|---|---|
| IV @ 1 mg/kg | Half life $T_{1/2}$ (h) | 5.80 |
| | Maximal concentration Cmax (ng/mL) | 2710 |
| | Area under curve $AUC_{(0-t)}$ (h*ng/mL) | 17600 |
| | Apparent clearance rate Cl (m L/m in/kg) | 0.745 |
| PO @ 5 mg/kg | Half life $T_{1/2}$ (h) | 9.14 |
| | Maximal concentration Cmax (ng/mL) | 4140 |
| | Area under curve $AUC_{(0-t)}$ (h*ng/mL) | 67100 |
| | Bioavailability F% | 76.3 |

**Experiment Example 6: *In vivo* pharmacokinetic study in ICR mice**

1 .Test animal

Species: ICR mice, SPF grade. Source: Shanghai Xipuer-Bikai Laboratory Animal Co., Ltd. Number: 3 mice per dosage form.

2. Preparation of test sample

2.1 An appropriate amount of the test sample was accurately weighed, into which 5% DMSO, 10% polyethylene glycol-15 hydroxystearate, 85% normal saline (all in volume percent) were added sequentially, and were fully mixed via vortex or ultrasound, to obtain the dosing solution with a test sample concentration of 0.4 mg/mL for intravenous administration.

2.2 An appropriate amount of the test sample was accurately weighed, into which 5% DMSO, 10% polyethylene glycol-15 hydroxystearate, 85% normal saline (all in volume percent) were added sequentially, and were fully mixed via vortex or ultrasound, to obtain the dosing solution with a test sample concentration of 1 mg/mL for oral gavage administration.

3. Experimental design

| Group | Test sample | Number male | Dosage | Concentration | Administration volume | Administration mode | Collected sample |
|---|---|---|---|---|---|---|---|
| | | | (mg/kg) | (mg/mL) | (mL/kg) | | |
| 1 | Example 36 | 3 | 2 | 0.4 | 5 | IV | Plasma |
| 2 | | 3 | 10 | 1 | 10 | PO | Plasma |

4. Administration mode

Weighing was performed before administration, and the administration amount was calculated according to the body weight. The drug was administered orally by gavage or intravenously.

5. Time points of blood collection

Before and 0.083h, 0.25h, 0.5h, 1h, 2h, 4h, 6h, 8h, 24h after administration.

6. Sample collection and disposal

Blood was collected via submandibular vein or other suitable means, and approximately 0.03mL per sample was collected via anticoagulation with heparin sodium. Blood samples were placed on ice after collection and centrifuged to separate plasma within 1 h (centrifugation conditions: centrifugal force of 6800g, 6 min, 2-8°C). The collected plasma samples were stored in a -80°C refrigerator before analysis, and the remaining plasma samples continued to be stored in a -80°C refrigerator for temporary storage after analysis.

7. Bioanalysis and data processing

When plot the plasma concentration-time curve according to the detected plasma concentration of the test substance, BLQ (Beneath Limit of Quantification) was recorded as 0. When calculating pharmacokinetic parameters, the concentration before administration was calculated as 0; BLQ (including "No peak") before $C_{max}$ was calculated as 0; BLQ (including "No peak") after $C_{max}$ was not involved in the calculation. Pharmacokinetic parameters, such as $AUC_{(0-t)}$, $T_{1/2}$, $C_{max}$, etc., were calculated by WinNonlin using the plasma concentration data at different time points.

**Table 6:** Data of *in vivo* pharmacokinetic studies of test compounds administered intravenously and orally to ICR mice

| Compound | | Example 36 |
|---|---|---|
| IV @ 2 mg/kg | Half life $T_{1/2}$ (h) | 5.53 |
| | Maximal concentration Cmax (ng/mL) | 3542.41 |
| | Area under curve $AUC_{(0-t)}$ (h*ng/m L) | 31742.76 |
| | Apparent clearance rate Cl (m L/m in/kg) | 1.03 |
| PO @ 10 mg/kg | Half life $t_{1/2}$ (h) | 9.32 |
| | Maximal concentration Cmax (ng/mL) | 18050.50 |
| | Area under curve $AUC_{(0-t)}$ (h*ng/mL) | 211271.44 |
| | Bioavailability F% | 133.11 |

## Claims

1. A compound represented by the structure of Formula I, the pharmaceutically acceptable salt, hydrate, isomer, prodrug and mixture thereof:

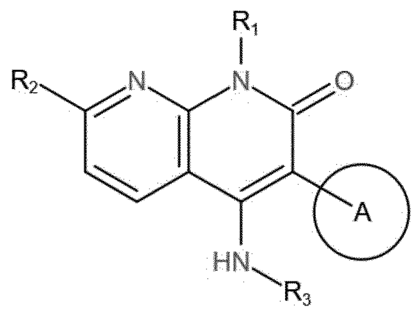

Formula I

wherein, $R_1$ is selected from 5-10 membered aryl or aromatic heterocyclic group; $R_2$ is selected from $-CF_3$ or cyclopropyl;
$R_3$ is selected from hydrogen, alkyl, aryl, aromatic heterocyclic group, cycloalkyl, aliphatic heterocyclic group, bridged cyclic group and spirocyclic group;
A is aryl or aromatic heterocyclic group,
with the proviso that the compound excludes compounds of the following formulae:

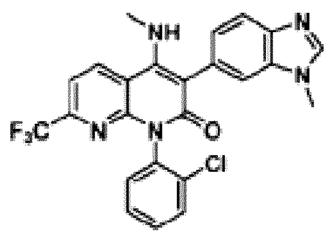

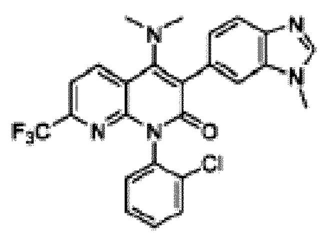

,

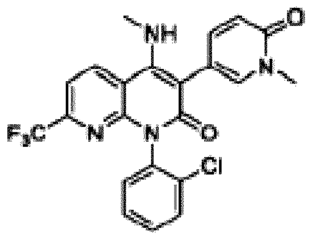

, and

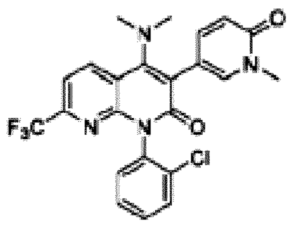

2. The compound, the pharmaceutically acceptable salt, hydrate, isomer, prodrug and mixture thereof according to claim 1, **characterized in that** $R_1$ is selected from imidazolyl, thiazolyl, pyrazolyl, phenyl, pyridinyl, pyrimidinyl, pyridazinyl, and pyrazinyl.

3. The compound, the pharmaceutically acceptable salt, hydrate, isomer, prodrug and mixture thereof according to claim 1 or 2, **characterized in that** $R_1$ can be further substituted with 0-2 $R_a$ groups; each of $R_a$ groups can be independently selected from alkyl, halogen, haloalkyl, alkoxy, haloalkoxy, hydroxy, amino, amine, carboxy, amide, cycloalkyl, and deuterium.

4. The compound, the pharmaceutically acceptable salt, hydrate, isomer, prodrug and mixture thereof according to claim 3, **characterized in that** $R_1$ can be further substituted with 0-2 $R_a$ groups; each of $R_a$ groups can be independently selected from $C_1$-$C_3$ alkyl, fluoro, chloro, bromo, iodo, $C_1$-$C_3$ haloalkyl, $C_1$-$C_3$ alkoxy, $C_1$-$C_3$ haloalkoxy, hydroxy, amino, amine, carboxy, acyl, $C_3$-$C_6$ cycloalkyl, and deuterium.

5. The compound, the pharmaceutically acceptable salt, hydrate, isomer, prodrug and mixture thereof according to claim 1, **characterized in that** $R_1$ is phenyl, and the phenyl can be further substituted with 0-2 $R_a$ groups; each of $R_a$ groups can be independently selected from $C_1$-$C_3$ alkyl, fluoro, chloro, bromo, and iodo; preferably, $R_1$ is selected from phenyl, 4-chlorophenyl, 4-bromophenyl, and 4-methylphenyl, and the phenyl can be further substituted with fluorine.

6. The compound, the pharmaceutically acceptable salt, hydrate, isomer, prodrug and mixture thereof according to any of claims 1-5, **characterized in that** $R_3$ is selected from hydrogen, $C_1$-$C_3$ alkyl, 6-10 membered aryl, 5-10 membered aromatic heterocyclic group, $C_3$-$C_6$ cycloalkyl, 3-6 membered aliphatic heterocyclic group, 4-10 membered bridged cyclic group, and spirocyclic group.

7. The compound, the pharmaceutically acceptable salt, hydrate, isomer, prodrug and mixture thereof according to claim 6, **characterized in that** $R_3$ is selected from hydrogen, methyl, ethyl, propyl, isopropyl, cyclopropyl, cyclobutyl, cyclopentyl, oxetanyl, tetrahydrofuranyl, tetrahydropyranyl, thiocyclohexyl, piperidinyl, pyrrolidinyl, phenyl, pyridinyl, pyrimidinyl, imidazolyl, pyrazolyl, thiazolyl, oxazolyl, isoxazolyl, 1,2,4-oxadiazolyl, 4-10 membered bridged cyclic group, and spirocyclic group.

8. The compound, the pharmaceutically acceptable salt, hydrate, isomer, prodrug and mixture thereof according to any of claims 1-7, **characterized in that** when $R_3$ is not hydrogen, $R_3$ can be optionally substituted with one or more groups selected from halogen, alkyl, alkoxy, cyano, hydroxy, amino, deuterium, sulfone, sulfonyl, haloalkyl, cycloalkyl, and aliphatic heterocyclic group.

9. The compound, the pharmaceutically acceptable salt, hydrate, isomer, prodrug and mixture thereof according to claim 8, **characterized in that** when $R_3$ is not hydrogen, $R_3$ can be optionally substituted with one or more groups selected from halogen, $C_1$-$C_3$ alkyl, $C_1$-$C_3$ alkoxy, cyano, hydroxyl, amino, deuterium, sulfone, sulfonyl, $C_1$-$C_3$ haloalkyl, $C_3$-$C_6$ cycloalkyl, 3-6 membered aliphatic heterocyclic group.

10. The compound, the pharmaceutically acceptable salt, hydrate, isomer, prodrug and mixture thereof according to claim 9, **characterized in that** when $R_3$ is not hydrogen, $R_3$ can be optionally substituted with one or more groups selected from fluoro, chloro, bromo, iodo, methyl, ethyl, propyl, isopropyl, cyclopropyl, cyclobutyl, cyclopentyl, oxetanyl, tetrahydrofuryl, tetrahydropyranyl, thiocyclohexyl, piperidinyl, pyrrolidinyl, trifluoromethyl, hydroxyl, amino, cyano, deuterium, sulfone, and sulfonyl.

11. The compound, the pharmaceutically acceptable salt, hydrate, isomer, prodrug and mixture thereof according to

any of claims 1-10, **characterized in that** ring A is selected from 6-10 membered aromatic cyclic group and 5-10 membered aromatic heterocyclic group.

**12.** The compound, the pharmaceutically acceptable salt, hydrate, isomer, prodrug and mixture thereof according to claim 11, **characterized in that** the ring A is selected from phenyl, naphthyl, imidazolyl, pyrazolyl, triazolyl, thiazolyl, furanyl, pyrrolyl, thienyl, oxazolyl, isoxazolyl, oxadiazolyl, pyridinyl, pyrimidinyl, pyridazinyl, pyrazinyl, benzoimidazolyl, benzopyrazolyl, benzothiazolyl, benzoxazolyl, benzobisoxazole, imidazopyridinyl, benzisoxazolyl, naphthyridinyl, quinolinyl, isoquinolinyl, quinoxalinyl, pyrazolopyridinyl, triazolopyridinyl, pyridonyl, quinazolinyl, cinnolinyl, pyridopyrazine, benzotriazolyl, and benzoxadiazolyl.

**13.** The compound, the pharmaceutically acceptable salt, hydrate, isomer, prodrug and mixture thereof according to claim 12, **characterized in that** the ring A can be further substituted with one or more groups selected from alkyl, cycloalkyl, aliphatic heterocyclic group, halogen, alkoxy, amino, amine, hydroxy, cyano, haloalkyl, haloalkoxy, $-(CH_2)_nOCH_3$, $-(CH_2)_nSO_2CH_3$, and $-(CH_2)_nN(CH_3)_2$, wherein n = 1, 2, or 3.

**14.** The compound, the pharmaceutically acceptable salt, hydrate, isomer, prodrug and mixture thereof according to claim 13, **characterized in that** the ring A can be further substituted with one or more groups selected from $C_1$-$C_3$ alkyl, $C_1$-$C_3$ alkoxy, halogen, $C_1$-$C_3$ haloalkyl, amino, cyano, $-(CH_2)_nOCH_3$, $-(CH_2)_nSO_2CH_3$, and $-(CH_2)_nN(CH_3)_2$, wherein n = 1, 2, or 3.

**15.** The compound, the pharmaceutically acceptable salt, hydrate, isomer, prodrug and mixture thereof according to claim 13, **characterized in that** the ring A can be further substituted with one or more groups selected from methyl, methoxy, $-CF_3$, $-CH_2CF_3$, $-NH_2$, F, cyano, $-(CH_2)_2OCH_3$, $-(CH_2)_2SO_2CH_3$, $-(CH_2)_2N(CH_3)_2$.

**16.** The compound, the pharmaceutically acceptable salt, hydrate, isomer, prodrug and mixture thereof according to claim 13, **characterized in that** substituent(s) on the ring A can further form a ring, and form a fused ring with the ring A.

**17.** The compound, the pharmaceutically acceptable salt, hydrate, isomer, prodrug and mixture thereof according to any of claims 1-16, **characterized in that** the ring A is selected from the following groups:

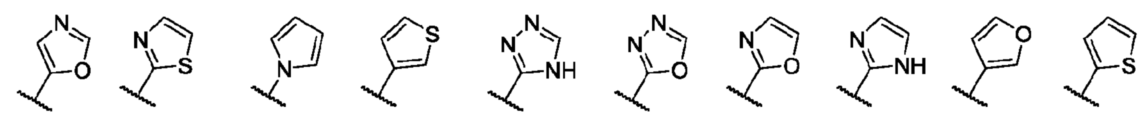

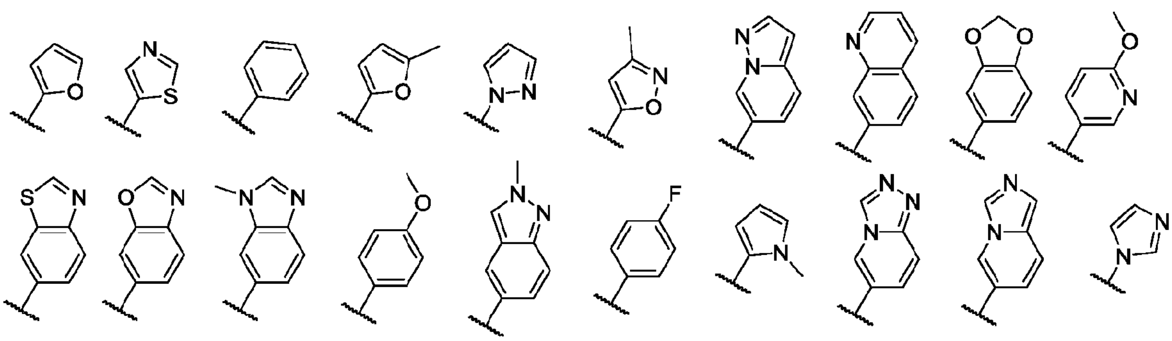

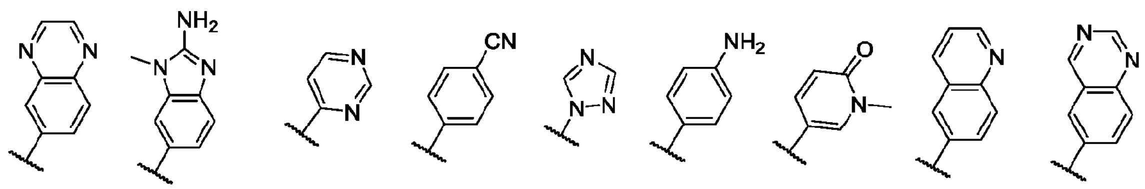

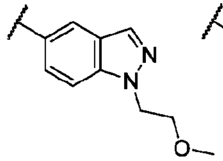
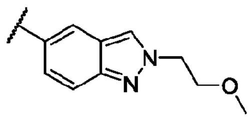
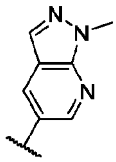
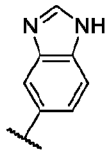
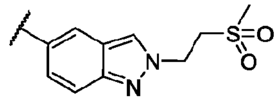

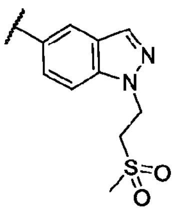
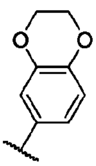
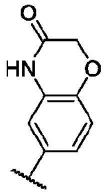
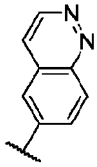
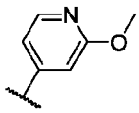
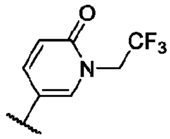
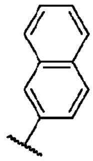

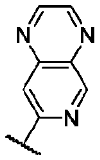
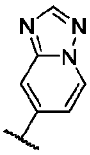
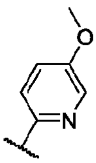
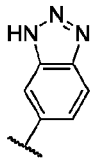
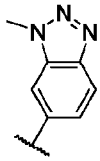
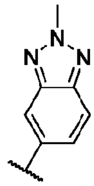
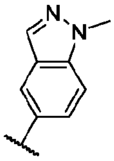
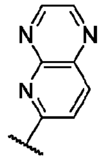

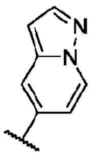
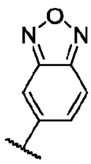
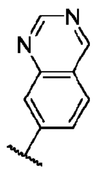
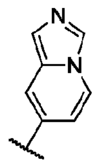
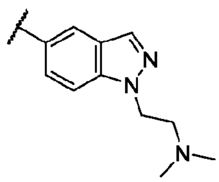
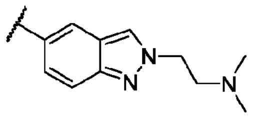
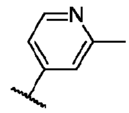

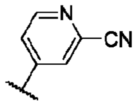
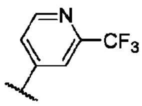
.

**18.** The compound, the pharmaceutically acceptable salt, hydrate, isomer, prodrug and mixture thereof according to any of claims 1-17, **characterized in that** the compound has the structure represented by Formula II or Formula III below:

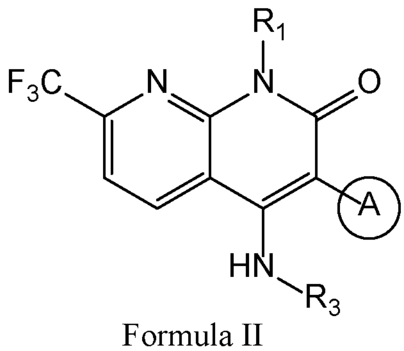
Formula II

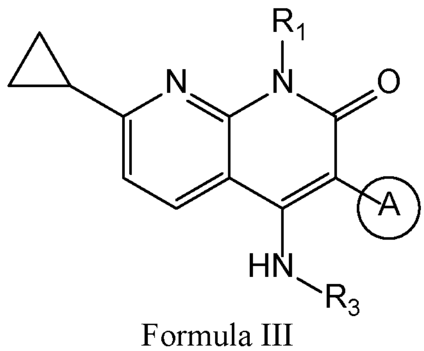
Formula III

.

**19.** The compound, the pharmaceutically acceptable salt, hydrate, isomer, prodrug and mixture thereof according to any of claims 1-18, **characterized in that** the compound of Formula I is selected from:

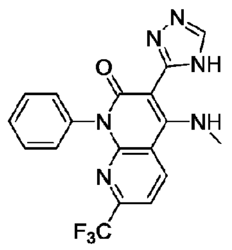
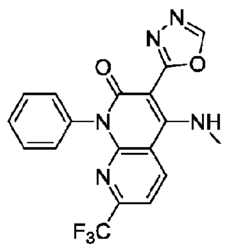
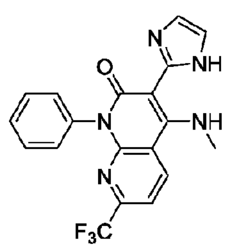
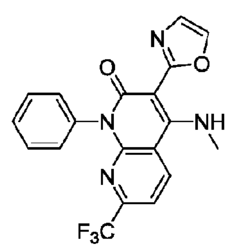

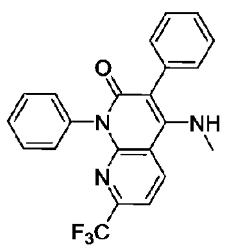
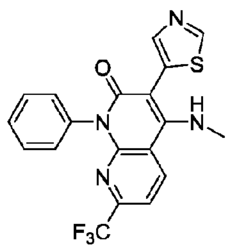
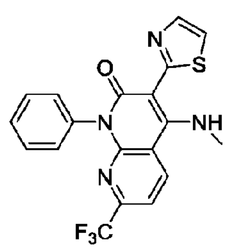
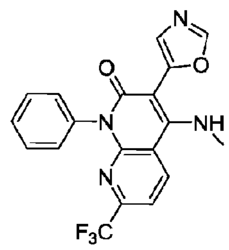

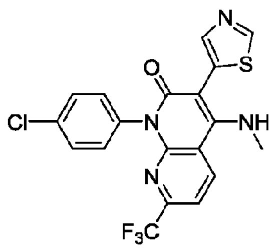
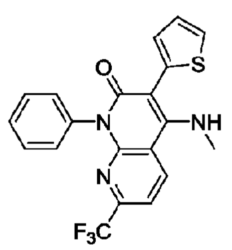
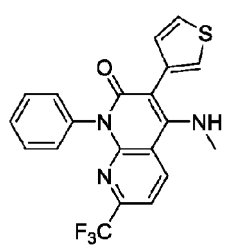
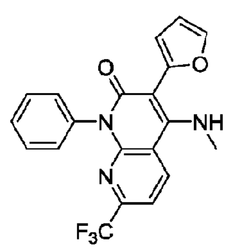

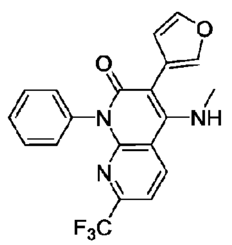
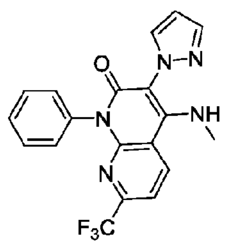
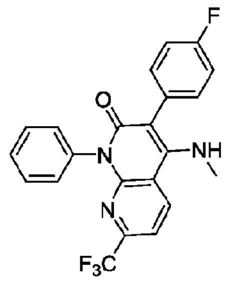
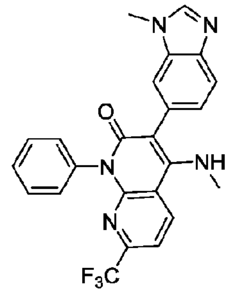

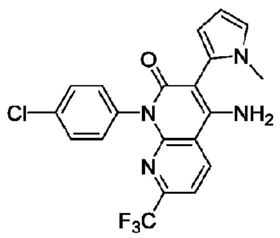
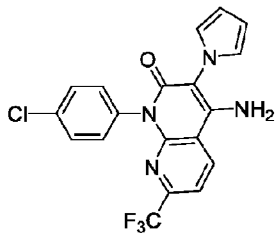
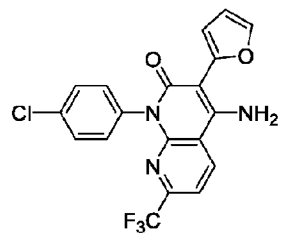

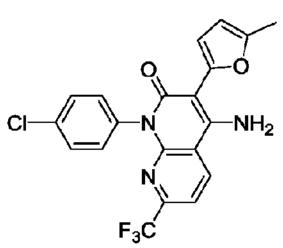
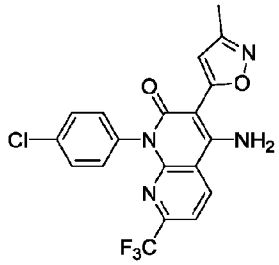
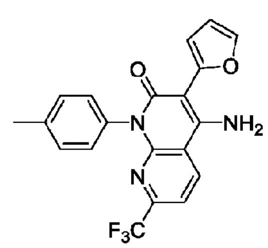

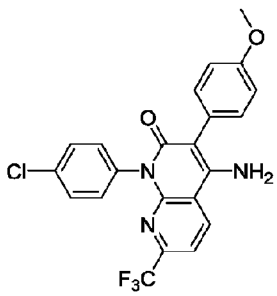
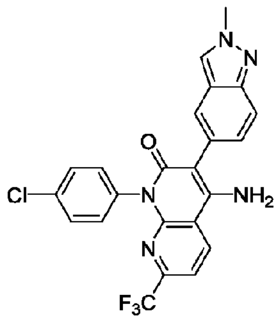
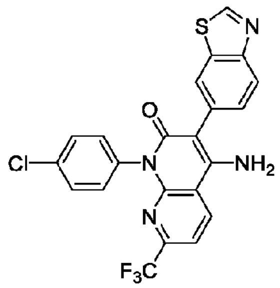

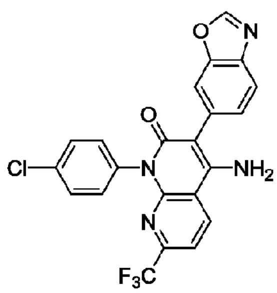
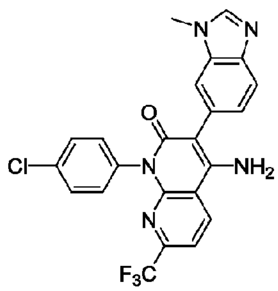
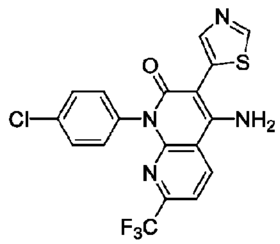

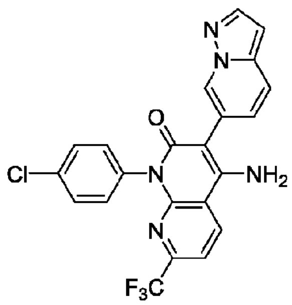
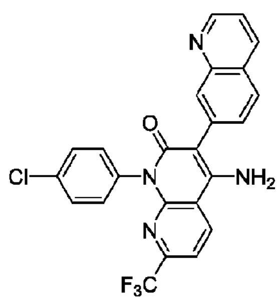
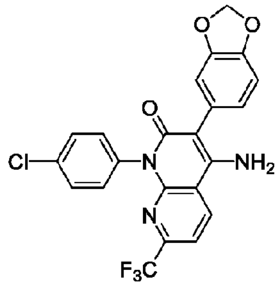

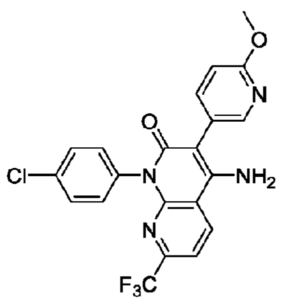
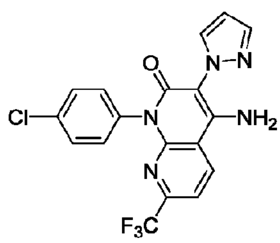
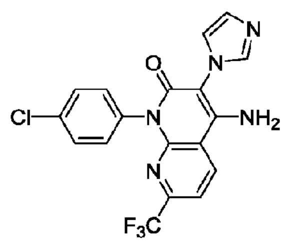

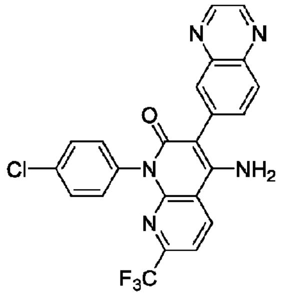
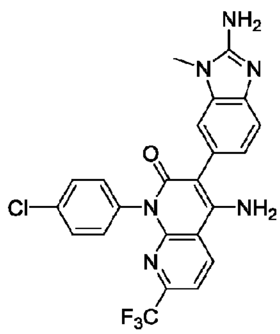
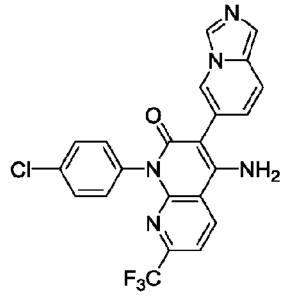

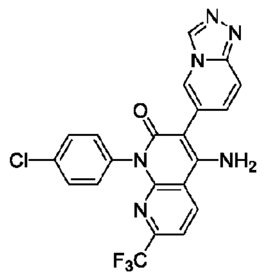
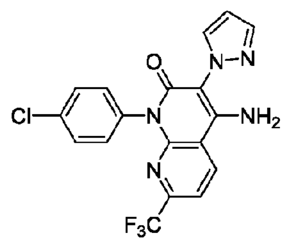
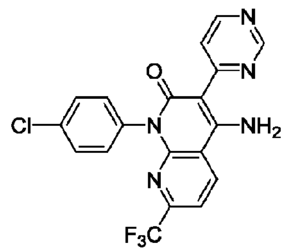

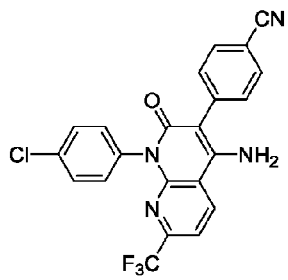
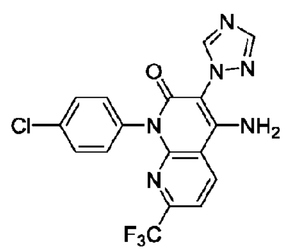
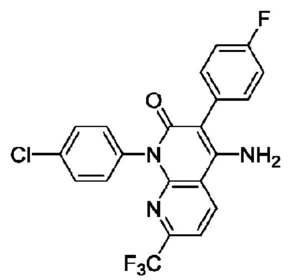

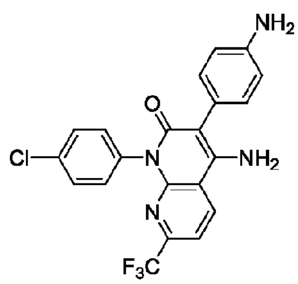
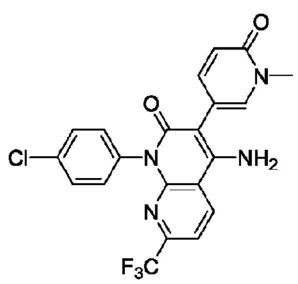
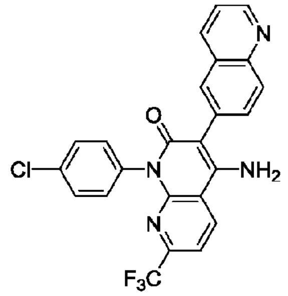

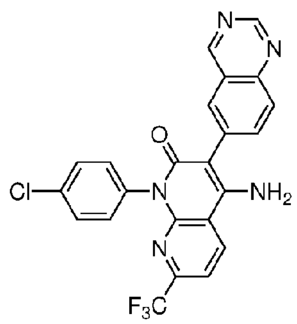
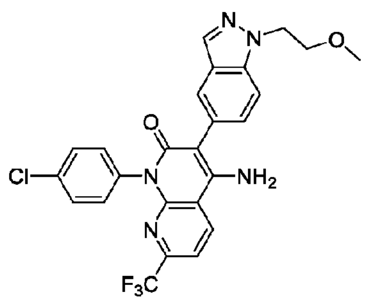
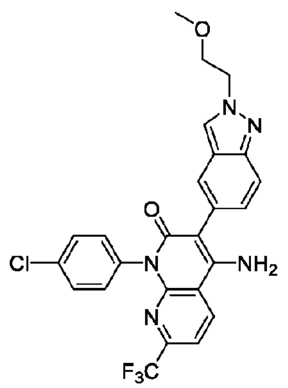

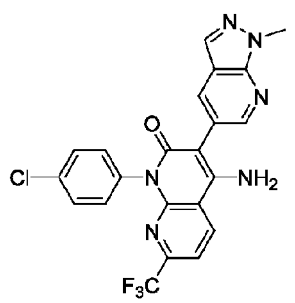
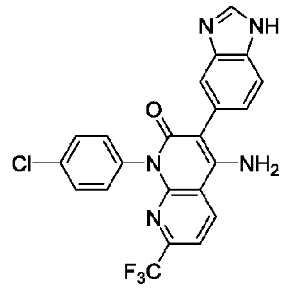
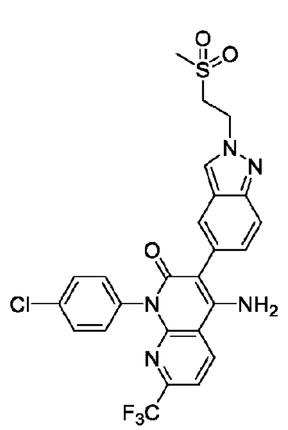

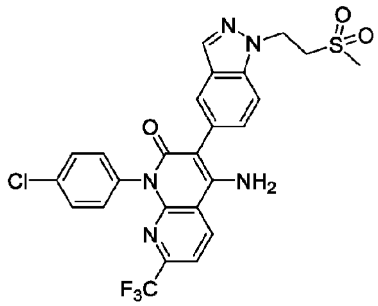
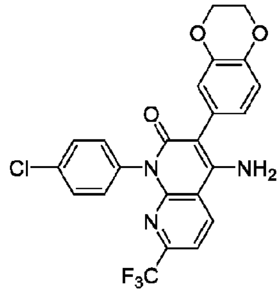
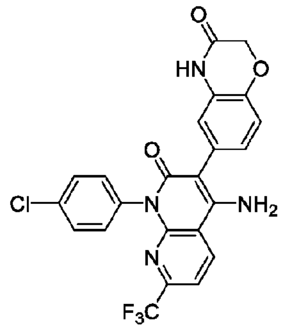

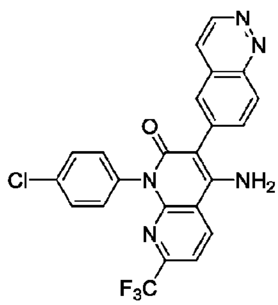
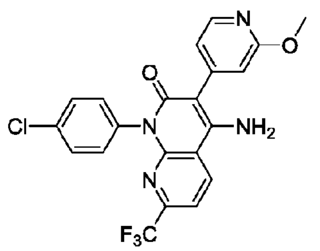
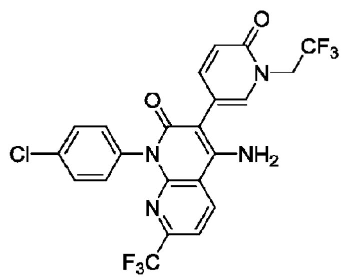

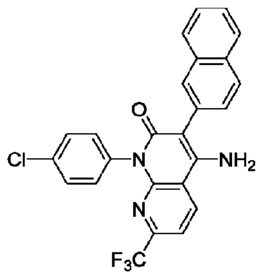
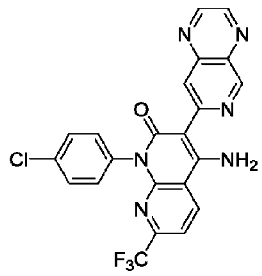
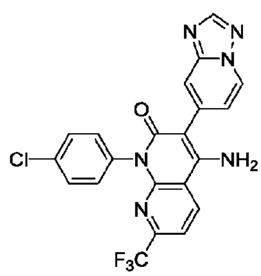

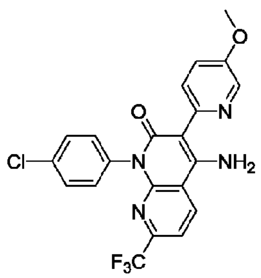
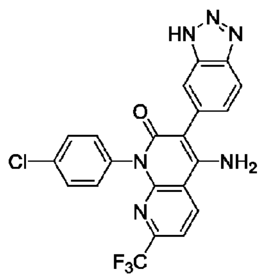
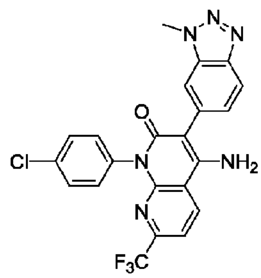

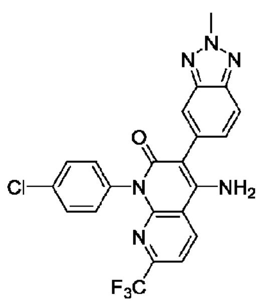
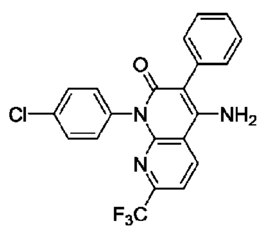
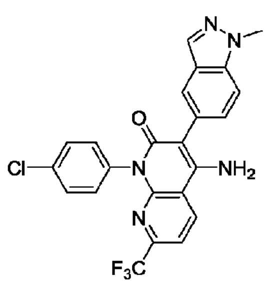

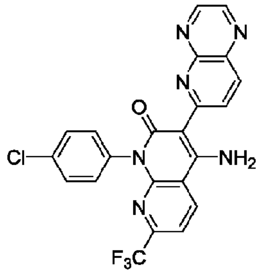

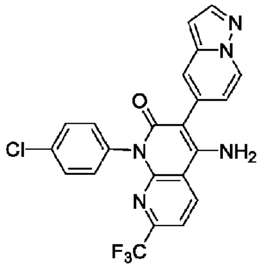

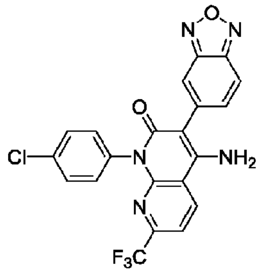

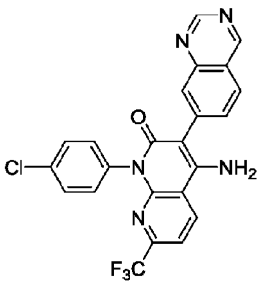

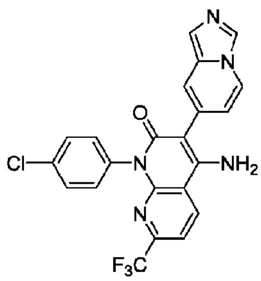

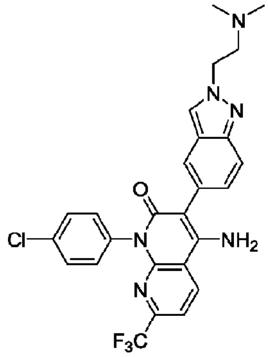

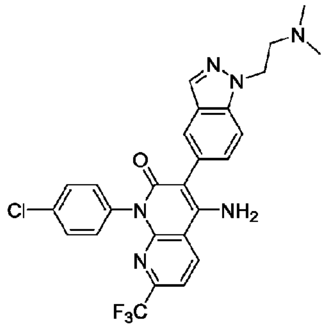

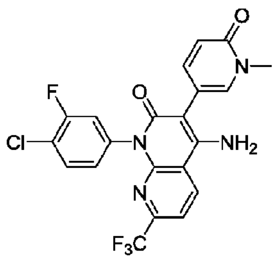

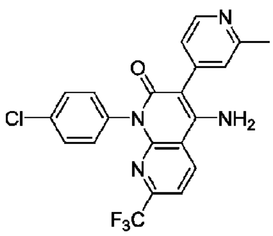

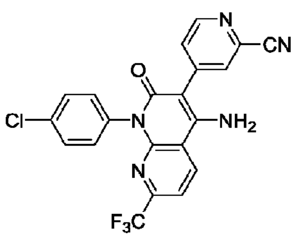

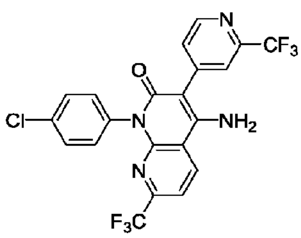

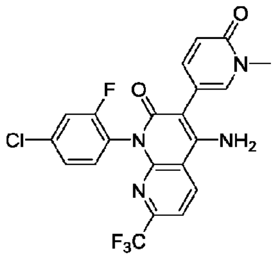

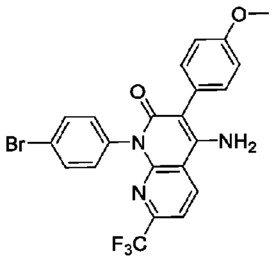

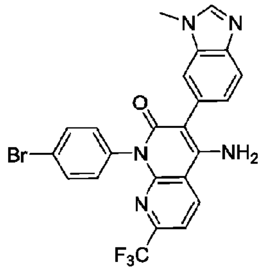

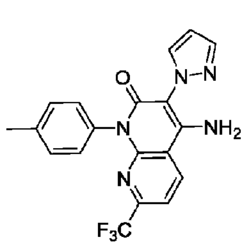

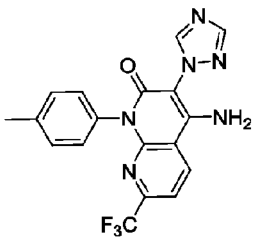
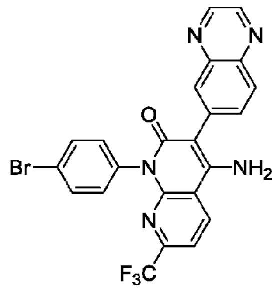
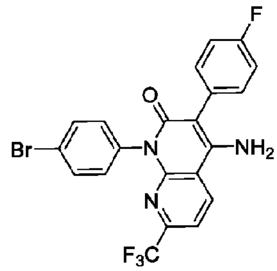
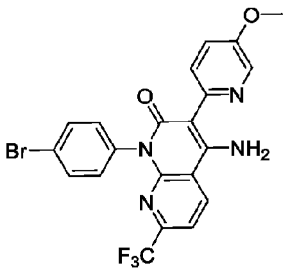
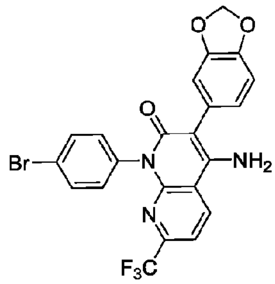
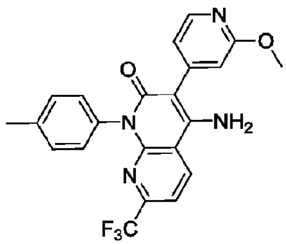
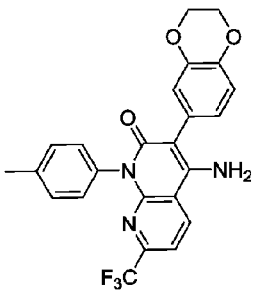
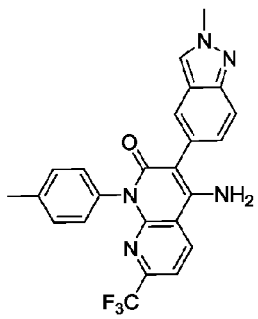
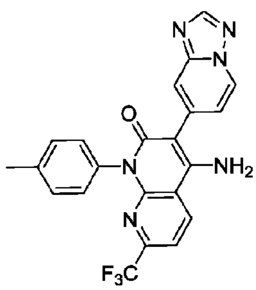
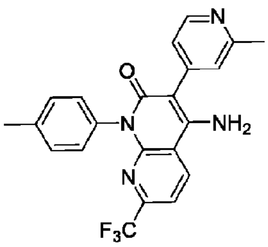
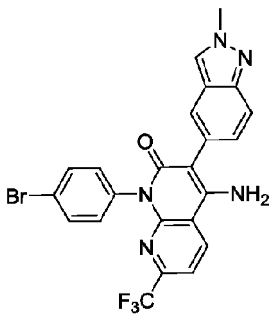
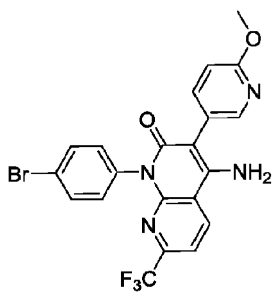
.

**20.** Use of the compound, the pharmaceutically acceptable salt, hydrate, isomer, prodrug or mixture thereof according to any of claims 1-19 in the preparation of a medicament for the treatment of a MAT2a-related disease.

**21.** A pharmaceutical composition comprising a therapeutically effective dose of the compound, or the pharmaceutically acceptable salt, hydrate, isomer, prodrug or mixture thereof according to any of claims 1-19, and a pharmaceutically acceptable carrier.

**22.** Use of the pharmaceutical composition according to claim 21 in the preparation of a medicament for the treatment of a MAT2a-related disease.

**23.** The use according to claim 20 or 22, **characterized in that** the MAT2a-related disease is cancer or tumor, further, the cancer or tumor comprises neuroblastoma, intestinal cancer such as rectal cancer, colon cancer, familial adenomatous polyposis cancer and hereditary nonpolyposis colorectal cancer, esophageal cancer, lip cancer, laryngeal cancer, hypopharyngeal cancer, tongue cancer, salivary gland cancer, gastric cancer, adenocarcinoma, medullary thyroid carcinoma, papillary thyroid carcinoma, renal carcinoma, renal parenchymal carcinoma, ovarian cancer, cervical cancer, uterine body cancer, endometrial cancer, choriocarcinoma, pancreatic cancer, prostate cancer, testicular cancer, breast cancer, urinary system cancer, melanoma, brain tumor such as glioblastoma, astrocytoma, meningioma, medulloblastoma and peripheral neuroectodermal tumor, Hodgkin's lymphoma, non-Hodgkin's lymphoma, Burkitt's lymphoma, acute lymphoblastic leukemia (ALL), chronic lymphoblastic leukemia (CLL), acute myeloid leukemia (AML), chronic myeloid leukemia (CML), adult T-cell leukemia, hepatocellular carcinoma, gallbladder cancer, bronchogenic carcinoma, small cell lung cancer, non-small cell lung cancer, multiple myeloma, basal cell tumor, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma, and plasmacytoma. In one embodiment, the cancer is lung cancer, non-small cell lung cancer (NSLC), bronchioloalveolar carcinoma, bone cancer, pancreatic cancer, skin cancer, head and neck cancer, skin or intraocular melanoma, uterine cancer, ovarian cancer, rectal cancer, anal cancer, gastric cancer, stomach cancer, colon cancer, breast cancer, uterine cancer, fallopian tube cancer, endometrial cancer, cervical cancer, vaginal cancer, vulva cancer, Hodgkin's disease, esophageal cancer, small intestine cancer, endocrine system cancer, thyroid cancer, parathyroid cancer, adrenal cancer, soft tissue sarcoma, urethral cancer, penile cancer, prostate cancer, bladder cancer, renal or ureteral carcinoma, renal cell carcinoma, renal pelvic carcinoma, mesothelioma, hepatocellular carcinoma, biliary tract cancer, chronic or acute leukemia, lymphoblastic lymphoma, central nervous system (CNS) tumor, spinal axis tumor, brain stem glioma, glioblastoma multiforme, astrocytoma, schwannoma, ependymoma, medulloblastoma, meningioma, squamous cell carcinoma, pituitary adenoma, including a refractory form of any of the above-mentioned cancers, or a combination of one or more of the above-mentioned cancers.

**INTERNATIONAL SEARCH REPORT**

International application No.
**PCT/CN2022/089900**

**A. CLASSIFICATION OF SUBJECT MATTER**

C07D 471/04(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS, CNKI, EPODOC, WPI, REGISTRY(STN), CAPLUS(STN): 甲硫氨酸腺苷转移酶, 抑制剂, 癌症, 肿瘤, methionine, adenosyltransferase, MAT, inhibitor, cancer, tumor, tumour

**C. DOCUMENTS CONSIDERED TO BE RELEVANT**

| Category* | Citation of document, with indication, where appropriate, of the relevant passages | Relevant to claim No. |
|---|---|---|
| X | WO 2020243376 A1 (AGIOS PHARMACEUTICALS, INC.) 03 December 2020 (2020-12-03)<br>abstract, and claims 1, 35-36, 39 and 41-59 | 1-23 |
| PX | WO 2021254529 A1 (JIANGSU SIMCERE PHARMACEUTICAL CO., LTD.) 23 December 2021 (2021-12-23)<br>abstract, and claims 1 and 14-15, and description, p. 12, paragraph 2, and embodiments 20-23 | 1-23 |
| A | WO 2020123395 A1 (IDEAYA BIOSCIENCES, INC.) 18 June 2020 (2020-06-18)<br>entire document | 1-23 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 July 2022** | **29 July 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/089900**

| Patent document cited in search report | | | Publication date (day/month/year) | Patent family member(s) | | | Publication date (day/month/year) |
|---|---|---|---|---|---|---|---|
| WO | 2020243376 | A1 | 03 December 2020 | EP | 3976611 | A1 | 06 April 2022 |
| | | | | SG | 11202112952 S | A | 30 December 2021 |
| | | | | BR | 112021023825 | A2 | 08 February 2022 |
| | | | | CA | 3142340 | A1 | 03 December 2020 |
| | | | | PE | 20220387 | A1 | 18 March 2022 |
| | | | | AR | 119046 | A1 | 17 November 2021 |
| | | | | IL | 288395 | D0 | 01 January 2022 |
| | | | | CO | 2021017981 | A2 | 19 April 2022 |
| | | | | TW | 202110841 | A | 16 March 2021 |
| | | | | AU | 2020284018 | A1 | 27 January 2022 |
| | | | | CR | 20210670 | A | 11 February 2022 |
| WO | 2021254529 | A1 | 23 December 2021 | | None | | |
| WO | 2020123395 | A1 | 18 June 2020 | IL | 283743 | D0 | 29 July 2021 |
| | | | | CO | 2021008895 | A2 | 30 July 2021 |
| | | | | KR | 20210103498 | A | 23 August 2021 |
| | | | | US | 11130759 | B1 | 28 September 2021 |
| | | | | CL | 2021001508 | A1 | 21 January 2022 |
| | | | | US | 11084798 | B1 | 10 August 2021 |
| | | | | TW | 202039487 | A | 01 November 2020 |
| | | | | JP | 2022512156 | A | 02 February 2022 |
| | | | | SG | 11202105469 Y | A | 29 June 2021 |
| | | | | US | 2022106276 | A1 | 07 April 2022 |
| | | | | PH | 12021551274 | A1 | 06 December 2021 |
| | | | | BR | 112021010842 | A2 | 24 August 2021 |
| | | | | EP | 3894396 | A1 | 20 October 2021 |
| | | | | US | 11046691 | B1 | 29 June 2021 |
| | | | | CA | 3121236 | A1 | 18 June 2020 |
| | | | | AU | 2019395338 | A1 | 29 July 2021 |
| | | | | CN | 113166078 | A | 23 July 2021 |

Form PCT/ISA/210 (patent family annex) (January 2015)